# EUROPEAN PATENT SPECIFICATION

(11) **EP 4 111 961 B1**
(45) Date of publication and mention of the grant of the patent: **11.02.2026**
(21) Application number: 21760200.2
(22) Date of filing: 20.02.2021
(51) Int. Cl.: A61B 5/145

(54) **TISSUE COMPONENT NONINVASIVE MEASUREMENT METHOD, APPARATUS AND SYSTEM, AND WEARABLE DEVICE**
NICHTINVASIVES MESSVERFAHREN FÜR GEWEBEKOMPONENTEN, VORRICHTUNG UND SYSTEM SOWIE WEARABLE-VORRICHTUNG
PROCÉDÉ, APPAREIL ET SYSTÈME DE MESURE NON INVASIVE DE COMPOSANT TISSULAIRE ET DISPOSITIF PORTABLE

(30) Priority: 26.02.2020 CN 202010121193
(43) Date of publication of application: 04.01.2023
(73) Proprietor: Sunrise Technologies Co., Ltd., Fengtai District Beijing 100071 (CN)
(72) Inventor: XU, Kexin, Beijing 100071 (CN)
(74) Representative: Isarpatent
(86) International application number: PCT/CN2021/077058
(87) International publication number: WO 2021/169872

(56) References cited:
- WO-A1-2019/184341
- CN-A- 1 699 973
- CN-A- 1 699 973
- CN-A- 101 292 875
- CN-A- 105 510 238
- CN-A- 105 510 238
- CN-A- 105 745 526
- CN-A- 110 411 947
- CN-B- 1 699 973
- JP-A- 2006 078 437
- JP-B2- 6 630 061
- US-A1- 2016 139 045

## Description

### TECHNICAL FIELD

The present disclosure relates to a field of spectrum detection technology, and in particular, to a non-invasive detection method, device, system and wearable apparatus for a tissue element.

### BACKGROUND

A near-infrared spectroscopy detection method has characteristics of rapidness, non-invasiveness, and multidimensional information, etc., and is generally adopted to detect a tissue element, including blood glucose, fat, and white blood cells, etc. However, due to a weak absorption of the tissue element to be detected and a small change in a concentration of the tissue element to be detected of a detected object, a valid signal for detection is weak. Moreover, it is very vulnerable to an interference of a human body background and a change in a measurement environment, and the interference may even cover up an information of the tissue element to be detected, which makes it difficult to extract a weak signal under the interference of a large background noise.

In order to solve the above-mentioned problem, a reference measurement method based on a floating reference theory is proposed. That is, for the tissue element to be detected, there is a source-detection distance at which the absorption and the scattering have a same influence on a diffusely-scattered light intensity and opposite directions, therefore the diffusely-scattered light intensity value emitted from an emission position corresponding to this source-detection distance has zero sensitivity to a change in a concentration of the tissue element to be detected. Such emission position with above characteristics may be referred to as a reference position (or a benchmark position), and the corresponding source-detection distance is a reference distance. Moreover, for the tissue element to be detected, there is also a source-detection distance corresponding to an emission position where the diffusely-scattered light intensity value having a greatest sensitivity to the change in the concentration of the tissue element is emitted. Such emission position with above characteristics may be referred to as a measurement position, and the corresponding source-detection distance is a measurement distance. The diffusely-scattered light intensity value corresponding to the reference distance reflects a response of an interference other than the change in the concentration of the tissue element to be detected in a detection process. The diffusely-scattered light intensity value corresponding to the measurement distance reflects a response of the tissue element to be detected and the response of the interference other than the tissue element to be detected. Therefore, the reference position and/or the measurement position need to be accurately determined.

In a related art, diffusely-scattered light intensity values emitted from a surface of a detected site are generally received by photosensitive surfaces at a limited number of source-detection distances from a center of an incident beam with a central incidence. The limited number of source-detection distances is determined according to an average parameter of most detected objects. On this basis, the reference distance and the measurement distance are further determined from the source-detection distances.

In a process of achieving a concept of the present disclosure, the inventor found that the related art at least has a problem that a detection accuracy is not high.

JP6630061B2 discloses a diffusion spectrum data processing method, a modeling method, a prediction method and a processing device. SOLUTION: A spectrum data processing method irradiates a medium to be measured including a specific component with probe light, acquires first spectrum data at a first radial position of the medium to be measured and second spectrum data at a second radial position, arbitrarily selects the first radial position and the second radial position, and performs difference processing on the first spectrum data and second spectrum data.

CN1699973A discloses a method for measuring concentration by using floating reference, which includes the following steps: according to a rate of change of light intensity with the concentration of an object to be measured at different positions away from a light source (or different measuring times at the same position), obtaining measuring points and reference points required by the measurement, and using the light intensity measuring value at the reference points and the measuring points to realize the measurement of component concentration in the object to be measured. The invention is characterized in that the method includes the following steps: measuring the light intensity parameters at the reference points; measuring light intensity parameters at the measuring points; performing data pretreatment to the measuring results, performing difference treatment to signals at the measuring points based on the light intensity at the reference points, establishing a correction model between the concentration and the light intensity difference signal; and realizing the measurement of the concentration of the object to me measured.

### SUMMARY

The invention is set out by the appended set of claims.

An aspect of the present disclosure provides a method of determining a distance in a non-invasive detection of a tissue element, including: acquiring, for a detected site of a detected object, a first light intensity value for each predetermined wavelength of at least one predetermined wavelength at each source-detection distance of at least two source-detection distances; and determining a first light intensity measurement value and/or a first light intensity reference value from the first light intensity values corresponding to the predetermined wavelength according to an absolute value of a light intensity variation caused by a change in a concentration of a tissue element to be detected, determining a source-detection distance corresponding to the first light intensity measurement value as a measurement distance, and determining a source-detection distance corresponding to the first light intensity reference value as a reference distance, wherein the first light intensity measurement value corresponds to a greatest absolute value of the light intensity variation caused by the change in the concentration of the tissue element to be detected, the first light intensity reference value corresponds to a smallest absolute value of the light intensity variation caused by the change in the concentration of the tissue element to be detected, and the light intensity variation caused by the change in the concentration of the tissue element to be detected is a variation between the first light intensity value and a corresponding predetermined light intensity value.

Another aspect of the present disclosure provides a method of determining a distance in a non-invasive detection of a tissue element, including: acquiring, for a detected site of a detected object, a tissue optical parameter corresponding to each predetermined wavelength of at least one predetermined wavelength and a tissue optical parameter change relationship caused by a change in a concentration of a tissue element to be detected; and determining each measurement distance and/or each reference distance according to the tissue optical parameter corresponding to each predetermined wavelength and the tissue optical parameter change relationship caused by the change in the concentration of the tissue element to be detected.

Another aspect of the present disclosure provides a non-invasive detection method for a tissue element, including: acquiring, for a detected site of a detected object, a second light intensity measurement value for each predetermined wavelength of at least one predetermined wavelength at a measurement distance, and/or a second light intensity reference value for each predetermined wavelength of at least one predetermined wavelength at a reference distance, wherein each measurement distance and each reference distance are determined according to the method of determining the distance in the non-invasive detection of the tissue element; and determining a concentration of a tissue element to be detected according to the second light intensity measurement value for each predetermined wavelength and/or the second light intensity reference value for each predetermined wavelength.

Another aspect of the present disclosure provides a device of determining a distance in a non-invasive detection of a tissue element, including: a first acquisition module configured to acquire, for a detected site of a detected object, a first light intensity value corresponding to each predetermined wavelength of at least one predetermined wavelength at each source-detection distance of at least two source-detection distances; and a first determination module configured to determine a first light intensity measurement value and/or a first light intensity reference value from the first light intensity values corresponding to the predetermined wavelength according to an absolute value of a light intensity variation caused by a change in a concentration of a tissue element to be detected, determine a source-detection distance corresponding to the first light intensity measurement value as a measurement distance, and determine a source-detection distance corresponding to the first light intensity reference value as a reference distance, wherein the first light intensity measurement value corresponds to a greatest absolute value of the light intensity variation caused by the change in the concentration of the tissue element to be detected, the first light intensity reference value corresponds to a smallest absolute value of the light intensity variation caused by the change in the concentration of the tissue element to be detected, and the light intensity variation caused by the change in the concentration of the tissue element to be detected is a variation between the first light intensity value and a corresponding predetermined light intensity value.

Another aspect of the present disclosure provides a device of determining a distance in a non-invasive detection of a tissue element, including: a second acquisition module configured to acquire, for a detected site of a detected object, a tissue optical parameter corresponding to each predetermined wavelength of at least one predetermined wavelength and a tissue optical parameter change relationship caused by a change in a concentration of a tissue element to be detected; and a second determination module configured to determine each measurement distance and/or each reference distance according to the tissue optical parameter corresponding to each predetermined wavelength and the tissue optical parameter change relationship caused by the change in the concentration of the tissue element to be detected.

Another aspect of the present disclosure provides a non-invasive detection device for a tissue element, including: a light intensity sensor configured to acquire, for a detected site of a detected object, a second light intensity measurement value for each predetermined wavelength of at least one predetermined wavelength at a measurement distance, and/or a second light intensity reference value for each predetermined wavelength of at least one predetermined wavelength at a reference distance, wherein each measurement distance and each reference distance are determined using the device of determining the distance in the non-invasive detection of the tissue element; and a processor configured to determine a concentration of a tissue element to be detected according to the second light intensity measurement value for each predetermined wavelength and/or the second light intensity reference value for each predetermined wavelength.

Another aspect of the present disclosure provides a wearable apparatus, including a body and the non-invasive detection device for the tissue element described above; the non-invasive detection device for the tissue element is arranged on the body; and the wearable apparatus is worn on the detected site.

Another aspect of the present disclosure provides a non-invasive detection system for a tissue element, including the wearable apparatus described above and a terminal; the processor is communicatively connected with the light intensity sensor and the terminal respectively; the wearable apparatus is worn on the detected site; the light intensity sensor is configured to acquire, for the detected site of the detected object, a second light intensity measurement value for each predetermined wavelength of at least one predetermined wavelength at a measurement distance, and/or a second light intensity reference value for each predetermined wavelength of at least one predetermined wavelength at a reference distance, wherein each measurement distance and each reference distance are determined using the device of determining the distance in the non-invasive detection of the tissue element; the processor is configured to process each second light intensity measurement value and/or each second light intensity reference value for each predetermined wavelength to obtain each processed second light intensity measurement value and/or each processed second light intensity reference value for each predetermined wavelength, and transmit each processed second light intensity measurement value and/or each processed second light intensity reference value for each predetermined wavelength to the terminal; and the terminal is configured to determine the concentration of the tissue element to be detected according to each processed second light intensity measurement value and/or each processed second light intensity reference value for each predetermined wavelength.

### BRIEF DESCRIPTION OF THE DRAWINGS

FIG. 1 shows a flowchart of a method of determining a distance in a non-invasive detection of a tissue element according to embodiments of the present disclosure;
FIG. 2 shows a schematic diagram of a target ring beam formed on a surface of a detected site according to embodiments of the present disclosure;
FIG. 3 shows a schematic diagram of acquiring a first light intensity value based on a photosensitive surface according to embodiments of the present disclosure;
FIG. 4 shows a schematic diagram of forming a target ring beam by a point-shaped light spot scanning according to embodiments of the present disclosure;
FIG. 5 shows a schematic diagram of forming a target ring beam by a beam projection according to embodiments of the present disclosure;
FIG. 6 shows another schematic diagram of acquiring a first light intensity value based on a photosensitive surface according to embodiments of the present disclosure;
FIG. 7 shows another schematic diagram of acquiring a first light intensity value based on a photosensitive surface according to embodiments of the present disclosure;
FIG. 8 shows still another schematic diagram of acquiring a first light intensity value based on a photosensitive surface according to embodiments of the present disclosure;
FIG. 9 shows a schematic diagram of shielding an interference light according to embodiments of the present disclosure;
FIG. 10 shows another schematic diagram of shielding an interference light according to embodiments of the present disclosure;
FIG. 11 shows a flowchart of another method of determining a distance in a non-invasive detection of a tissue element according to embodiments of the present disclosure;
FIG. 12 shows a flowchart of still another method of determining a distance in a non-invasive detection of a tissue element according to embodiments of the present disclosure;
FIG. 13 shows a flowchart of a non-invasive detection method for a tissue element according to embodiments of the present disclosure;
FIG. 14 shows a schematic diagram of a measurement ring beam and a reference ring beam formed on a surface of the detected site according to embodiments of the present disclosure;
FIG. 15 shows a schematic diagram of acquiring a second light intensity measurement value and a second light intensity reference value based on a photosensitive surface according to embodiments of the present disclosure;
FIG. 16 shows another schematic diagram of acquiring a second light intensity measurement value and a second light intensity reference value based on a photosensitive surface according to embodiments of the present disclosure;
FIG. 17 shows another schematic diagram of shielding an interference light according to embodiments of the present disclosure;
FIG. 18 shows a flowchart of another non-invasive detection method for a tissue element according to embodiments of the present disclosure;
FIG. 19 shows a flowchart of still another non-invasive detection method for a tissue element according to embodiments of the present disclosure;
FIG. 20 shows a schematic structural diagram of a device of determining a distance in a non-invasive detection of a tissue element according to embodiments of the present disclosure;
FIG. 21 shows a schematic structural diagram of a first acquisition module according to embodiments of the present disclosure;
FIG. 22 shows a schematic structural diagram of a first forming sub-module according to embodiments of the present disclosure;
FIG. 23 shows a schematic structural diagram of another device of determining a distance in a non-invasive detection of a tissue element according to embodiments of the present disclosure;
FIG. 24 shows a schematic structural diagram of another device of determining a distance in a non-invasive detection of a tissue element according to embodiments of the present disclosure;
FIG. 25 shows a schematic structural diagram of another device of determining a distance in a non-invasive detection of a tissue element according to embodiments of the present disclosure;
FIG. 26 shows a schematic structural diagram of device of determining a distance in a non-invasive detection of a tissue element according to embodiments of the present disclosure;
FIG. 27 shows a schematic structural diagram of still another device of determining a distance in a non-invasive detection of a tissue element according to embodiments of the present disclosure;
FIG. 28 shows a schematic diagram of another photosensitive surface in non-contact with a surface of a detected site according to embodiments of the present disclosure;
FIG. 29 shows a schematic diagram of still another photosensitive surface in non-contact with a surface of a detected site according to embodiments of the present disclosure;
FIG. 30 shows another schematic diagram of shielding an interference light according to embodiments of the present disclosure;
FIG. 31 shows still another schematic diagram of shielding an interference light according to embodiments of the present disclosure;
FIG. 32 shows a schematic structural diagram of a non-invasive detection device for a tissue element according to embodiments of the present disclosure;
FIG. 33 shows a schematic structural diagram of a light intensity sensor according to embodiments of the present disclosure;
FIG. 34 shows a schematic structural diagram of a ring beam generator according to embodiments of the present disclosure;
FIG. 35 shows another schematic diagram of shielding an interference light according to embodiments of the present disclosure;
FIG. 36 shows a schematic structural diagram of a wearable apparatus according to embodiments of the present disclosure; and
FIG. 37 shows a schematic structural diagram of a non-invasive detection system for a tissue element according to embodiments of the present disclosure.

### DETAILED DESCRIPTION OF EMBODIMENTS

Embodiments of the present disclosure will be further described below with reference to the accompanying drawings.

In a process of achieving a concept of the present disclosure, the inventor found that a reference distance and a measurement distance may vary with wavelengths, vary with detected objects, and vary with detected sites. If the reference distance and the measurement distance corresponding to each predetermined wavelength are determined for a detected site of a detected object, it is required to provide photosensitive surfaces at various source-detection distances from a center of an incident beam, which puts forward a high requirement for a production level of a photoelectric detector, or said which depends on the production level of the photoelectric detector. However, limited by a current production level of photoelectric detector, it is difficult to provide photosensitive surfaces at various source-detection distances from the center of the incident beam. The photosensitive surfaces may only be provided at a limited number of source-detection distances according to an average parameter for many detected objects. As a result, it is difficult to accurately determine the reference distance and the measurement distance corresponding to each predetermined wavelength for the detected site of the detected object by using the related art, and thus the detection accuracy is not high.

In order to improve the detection accuracy, the reference distance and/or the measurement distance need to be accurately determined. To solve this problem, the inventor proposes a solution of changing an arrangement of a light source and a photosensitive surface, which will be described below with reference to specific embodiments.

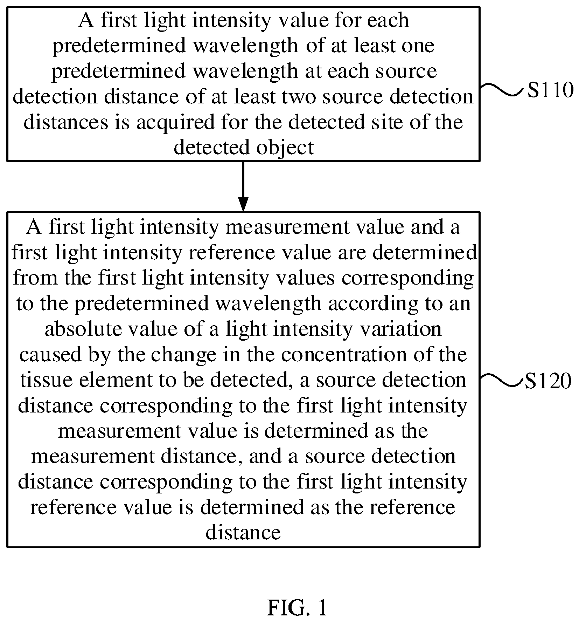

FIG. 1 shows a flowchart of a method of determining a distance in a non-invasive detection of a tissue element according to embodiments of the present disclosure. Such embodiments may be applied to improve the detection accuracy of a concentration of a tissue element to be detected.

As shown in FIG. 1, the method includes operations S110 to S120.

In operation S110, a first light intensity value corresponding to each predetermined wavelength of at least one predetermined wavelength at each source-detection distance of at least two source-detection distances is acquired for the detected site of the detected object.

According to embodiments of the present disclosure, the source-detection distance may represent a distance between a light source and an emission position. The light source here may be understood as a light beam formed on a surface of the detected site, and the emission position may represent a position where the light intensity value is emitted. The light intensity value is emitted from the surface of the detected site after the light beam passes through the detected site. It should be noted that the light intensity value described in embodiments of the present disclosure refers to a diffusely-reflected light intensity value, and the light intensity value used to determine the measurement distance and the reference distance in embodiments of the present disclosure is the first light intensity value. It should also be noted that if a photosensitive surface is arranged corresponding to the emission position, the source-detection distance may represent a distance between the light source and the photosensitive surface. The photosensitive surface here may be in contact with the detected site, or may be in non-contact with the detected site, which may be provided according to actual situations and is not specifically limited here. The photosensitive surface may be used to receive the light intensity value emitted from the surface of the detected site.

For the detected site of the detected object, at least one first light intensity value corresponding to each predetermined wavelength at each source-detection distance may be acquired. That is, when the detected site of the detected object is determined, for each predetermined wavelength, at least one first light intensity value for the predetermined wavelength at each source-detection distance is acquired. Each first light intensity value here may be a first light intensity value obtained through an in vivo test, a first light intensity value obtained through a Monte Carlo simulation, or a first light intensity value obtained through an in vitro test. Different first light intensity values for the same predetermined wavelength at the same source-detection distance correspond to different concentrations of the tissue element to be detected. That is, at least one first light intensity value for the same predetermined wavelength at the same source-detection distance is acquired, and different first light intensity values correspond to different concentrations of the tissue element to be detected.

If each first light intensity value is the first light intensity value obtained through the in vivo test or the first light intensity value obtained through the in vitro test, then acquiring the first light intensity value corresponding to each predetermined wavelength at each source-detection distance for the detected site of the detected object may be understood as follows. For the detected site of the detected object, at least two target ring beams corresponding to each predetermined wavelength are formed on the surface of the detected site, different target ring beams have different source-detection distances, each source-detection distance is an inner radius or outer radius of the target ring beam, and different target ring beams have the same geometric center. At least one first light intensity value emitted from the surface of the detected site after each target ring beam passes through the detected site is acquired based on the photosensitive surface corresponding to the geometric center. It should be noted that if the tissue element to be detected is blood glucose, the above-mentioned in vivo test may include OGTT (Oral Glucose Tolerance Test).

If each first light intensity value is the first light intensity value obtained through the Monte Carlo simulation, then the acquiring the first light intensity value corresponding to each predetermined wavelength at each source-detection distance for the detected site of the detected object may be understood as follows. A tissue optical parameter and a skin structure parameter for each predetermined wavelength in a three-layer skin tissue model are acquired for the detected site of the detected object. Based on the Monte Carlo simulation, the first light intensity value corresponding to each predetermined wavelength at each source-detection distance is determined according to each tissue optical parameter, each skin tissue structure parameter, a tissue optical parameter change relationship caused by a change in the concentration of the tissue element to be detected, at least two predetermined source-detection distances and a predetermined incident photon number. The Monte Carlo simulation may achieve a simulation of an optical propagation path of random scattering in a biological tissue, and a spatial distribution of the diffusely-scattered light intensity value and a distribution of an absorbed photon part in the tissue may be obtained. The three-layer skin tissue model may be understood as including epidermis, dermis and subcutaneous tissue. The tissue optical parameter may include an absorption coefficient, a scattering coefficient, an anisotropy factor and an average refractive index of each skin layer. The skin tissue structure parameter may be understood as a thickness of each layer of skin tissue, that is, a thickness of the epidermis, a thickness of the dermis and a thickness of the subcutaneous tissue. The tissue optical parameter change relationship caused by the change in the concentration of the tissue element to be detected may include an absorption coefficient change relationship caused by the change in the concentration of the tissue element to be detected, and a reduced scattering coefficient change relationship caused by the change in the concentration of the tissue element to be detected. The tissue element to be detected may include blood glucose, fat, and white blood cells, etc.

In operation S120, a first light intensity measurement value and/or a first light intensity reference value are/is determined from the first light intensity values corresponding to the predetermined wavelength according to an absolute value of a light intensity variation caused by the change in the concentration of the tissue element to be detected, a source-detection distance corresponding to the first light intensity measurement value is determined as the measurement distance, and a source-detection distance corresponding to the first light intensity reference value is determined as the reference distance. The first light intensity measurement value is a first light intensity value corresponding to a greatest absolute value of a light intensity variation caused by the change in the concentration of the tissue element to be detected. The first light intensity reference value is a first light intensity value corresponding to a smallest absolute value of a light intensity variation caused by the change in the concentration of the tissue element to be detected. The light intensity variation caused by the change in the concentration of the tissue element to be detected is a variation between the first light intensity value and a corresponding predetermined light intensity value.

According to embodiments of the present disclosure, the measurement distance is the source-detection distance corresponding to the emission position where the diffusely-scattered light intensity value having the greatest sensitivity to the change in the concentration of the tissue element to be detected is emitted, and the reference distance is the source-detection distance corresponding to the emission position where the diffusely-scattered light intensity value having zero sensitivity to the change in the concentration of the tissue element to be detected is emitted, where the sensitivity of the diffusely-scattered light intensity value to the change in the concentration of the tissue element to be detected is a ratio of the light intensity variation to a variation of the concentration of the tissue element to be detected. Therefore, when the variation of the concentration of the tissue element to be detected is determined, the measurement distance is the source-detection distance corresponding to the emission position where the light intensity variation with the greatest absolute value is emitted, and the reference distance is the source-detection distance corresponding to the emission position where the light intensity variation with the smallest absolute value is emitted. The above-mentioned diffusely-reflected light intensity value is the first light intensity value.

Based on the above, the first light intensity measurement value and/or the first light intensity reference value may be determined from the first light intensity values corresponding to the predetermined wavelength according to the absolute value of the light intensity variation caused by the change in the concentration of the tissue element to be detected. The source-detection distance corresponding to the first light intensity measurement value is determined as the measurement distance, and the source-detection distance corresponding to the first light intensity reference value is determined as the reference distance. The first light intensity measurement value is the first light intensity value corresponding to the greatest absolute value of the light intensity variation caused by the change in the concentration of the tissue element to be detected. The first light intensity reference value is the first light intensity value corresponding to the smallest absolute value of the light intensity variation caused by the change in the concentration of the tissue element to be detected. The light intensity variation caused by the change in the concentration of the tissue element to be detected is a variation between the first light intensity value and the corresponding predetermined light intensity value. Each predetermined light intensity value may be understood as a light intensity value emitted from the surface of the detected site when the concentration of the tissue element to be detected is a predetermined concentration. If each first light intensity value is the first light intensity value obtained through the in vivo test, then each predetermined light intensity value may be a light intensity value obtained when the detected object is in a fasting state. If each first light intensity value is the first light intensity value obtained by the Monte Carlo simulation or the first light intensity value obtained by the in vitro test, then each predetermined light intensity value may be a light intensity value emitted from the surface of the detected site when the predetermined concentration is zero.

According to embodiments of the present disclosure, the determining the first light intensity measurement value and/or the first light intensity reference value from the first light intensity values corresponding to the predetermined wavelength according to the absolute value of the light intensity variation caused by the change in the concentration of the tissue element to be detected may be understood as follows. For each predetermined wavelength, the first light intensity measurement value may be determined from the first light intensity values corresponding to the predetermined wavelength. Alternatively, the first light intensity measurement value and the first light intensity reference value may be determined from the first light intensity values corresponding to the predetermined wavelength. Alternatively, the first light intensity reference value may be determined from the first light intensity values corresponding to the predetermined wavelength. It may be understood that for at least one predetermined wavelength, there may be the following cases.

**In** a first case, only the first light intensity measurement value corresponding to each predetermined wavelength is determined. In a second case, the first light intensity measurement value and the first light intensity reference value corresponding to each wavelength are determined. In a third case, at least one first light intensity measurement value corresponding to at least one predetermined wavelength among the predetermined wavelengths is determined, and at least one first light intensity reference value corresponding to at least one other predetermined wavelength among the predetermined wavelengths is determined. In a fourth case, at least one first light intensity measurement value and at least one first light intensity reference value corresponding to at least one predetermined wavelength among the predetermined wavelengths are determined, and at least one first light intensity reference value corresponding to at least one other predetermined wavelength among the predetermined wavelengths is determined. In a fifth case, at least one first light intensity measurement value and at least one first light intensity reference value corresponding to at least one predetermined wavelength among the predetermined wavelengths are determined, and at least one first light intensity measurement value corresponding to at least one other predetermined wavelength among the predetermined wavelengths is determined.

On this basis, from the perspective of the measurement distance and the reference distance, for the at least one predetermined wavelength, there may be the following cases. In a first case, only the measurement distance corresponding to each predetermined wavelength is determined. In a second case, the measurement distance and the reference distance corresponding to each wavelength are determined. In a third case, at least one measurement distance corresponding to at least one predetermined wavelength among the predetermined wavelengths is determined, and at least one reference distance corresponding to at least one other predetermined wavelength among the predetermined wavelengths is determined. In a fourth case, at least one measurement distance and at least one reference distance corresponding to at least one predetermined wavelength among the predetermined wavelengths are determined, and at least one reference distance corresponding to at least one other predetermined wavelength among the predetermined wavelength is determined. In a fifth case, at least one measurement distance and at least one reference distance corresponding to at least one predetermined wavelength among the predetermined wavelengths are determined, and at least one measurement distance corresponding to at least one other predetermined wavelength among the predetermined wavelengths is determined. For each predetermined wavelength, the determination of the measurement distance and/or the reference distance corresponding to the predetermined wavelength may be set according to actual situations, which is not specifically limited herein.

For example, *λᵢ* represents the predetermined wavelength, *i* ∈ [1, *M*], *M* represents a number of the predetermined wavelength, *M*≥1*. ρⱼ* represents the source-detection distance, *j* ∈ [2, *N*], *N* represents a number of the source-detection distances, *N* ≥ 2 . *Tₖ* represents the concentration of the tissue element to be detected, *k* ∈ [1, *P*], *P* represents a number of the concentration of the tissue element to be detected, *P*≥1. A predetermined concentration corresponding to each predetermined light intensity value is represented by *T*₀.

When the concentration of the tissue element to be detected is *Tₖ,* one first light intensity value *ϕ*(*λᵢ*, *ρⱼ*)|*_{Tₖ}* corresponding to each predetermined wavelength *λᵢ* at each source-detection distance *ρⱼ* may be acquired for the detected site of the detected object. It may be understood that for each predetermined wavelength *λᵢ*, *N* first light intensity values *ϕ*(*λᵢ, ρⱼ*)|*_{λᵢ}, _{Tₖ}* corresponding to each concentration *Tₖ* of the tissue element to be detected may be acquired. Accordingly, for each predetermined wavelength *λᵢ*, *P* first light intensity value sets may be acquired, and each first light intensity value set includes *N* first light intensity values *ϕ*(*λᵢ*, *ρⱼ*)|*_{λᵢ}, _{Tₖ}*. For each first light intensity value set corresponding to each predetermined wavelength *λᵢ*, each first light intensity value *ϕ*(*λᵢ*, *ρⱼ*)|*_{λᵢ}, _{Tₖ}* in the first light intensity value set may be calculated with the predetermined light intensity value to determine absolute values of *N* light intensity variations caused by the change in the concentration of the tissue element to be detected. The greatest absolute value of the light intensity variation caused by the change in the concentration of the tissue element to be detected and the smallest absolute value of the light intensity variation caused by the change in the concentration of the tissue element to be detected are determined from the absolute values of the *N* light intensity variations caused by the change in the concentration of the tissue element to be detected. The change in the concentration of the tissue element to be detected may be represented by *Tₖ - T*₀*.* The first light intensity value corresponding to the greatest absolute value of the light intensity variation caused by the change in the concentration of the tissue element to be detected is determined as the first light intensity measurement value, and the first light intensity value corresponding to the smallest absolute value of the light intensity variation caused by the change in the concentration of the tissue element to be detected is determined as the first light intensity reference value. It may be understood that for each predetermined wavelength *λᵢ*, one first light intensity measurement value and one first light intensity reference value corresponding to each concentration *Tₖ* of the tissue element to be detected may be acquired. However, for each predetermined wavelength *λᵢ*, the first light intensity measurement values for different concentrations *Tₖ* of the tissue element to be detected correspond to the same source-detection distance, and the first light intensity reference values for different concentrations *Tₖ* of the tissue element to be detected also correspond to the same source-detection distance. The source-detection distance corresponding to the first light intensity measurement value may be determined as the measurement distance, and the source-detection distance corresponding to the first light intensity reference value may be determined as the reference distance.

According to the technical solution of embodiments of the present disclosure, as the first light intensity value corresponding to each predetermined wavelength at each source-detection distance may be acquired for the detected site of the detected object, the first light intensity measurement value and/or the first light intensity reference value may be accurately determined, and then the measurement distance and/or the reference distance may be accurately determined. On this basis, the accurate determination of the measurement distance and/or the reference distance provides a basis for determining the concentration of the tissue element to be detected, thereby improving the detection accuracy.

According to embodiments of the present disclosure, operation 110 may include the following operations. For the detected site of the detected object, at least two target ring beams corresponding to each predetermined wavelength are formed on the surface of the detected site. Different target ring beams have different source-detection distances, each source-detection distance is an inner or outer radius of the target ring beam, and different target ring beams have the same geometric center. The first light intensity value emitted from the surface of the detected site after each target ring beam passes through the detected site is acquired based on the photosensitive surface corresponding to the geometric center.

According to embodiments of the present disclosure, in order to accurately determine the measurement distance and the reference distance, dynamic target ring beams with variable size may be incident, the photosensitive surface is provided at a position corresponding to the center of the target ring beams, and different target ring beams may have the same geometric center. Different target ring beams have different sizes, each size includes sizes of inner radius and outer radius, and the photosensitive surface is arranged at the position corresponding to the center of each target ring beam. Therefore, each first light intensity value received by the photosensitive surface is the first light intensity value generated after a corresponding target ring beam is incident and passes through a corresponding transmission path. The photosensitive surface and the target ring beam of each size may also correspond to one source-detection distance.

According to embodiments of the present disclosure, as the measurement distance and the reference distance may vary with wavelengths, vary with detected objects and vary with detected sites, then for detected sites of each detected object, the first light intensity values corresponding to each predetermined wavelength may be acquired in the above-mentioned manner, so that the reference distance and/or the measurement distance corresponding to each predetermined wavelength may be accurately determined for the detected sites of the detected object.

According to embodiments of the present disclosure, the above-mentioned emission and reception manners of the target ring beam and the photosensitive surface may greatly reduce the requirements for the photoelectric detector, thereby reducing the manufacturing cost and being easy for implementation. Moreover, a continuous detection may be achieved. The so-called continuous detection may be understood as a continuity of the source-detection distance.

Based on the above, the implementation is as follows. For the detected site of the detected object, target ring beams of different sizes corresponding to each predetermined wavelength are formed on the surface of the detected site, and the photosensitive surface is provided at the position corresponding to the center of each target ring beam. At least one first light intensity value emitted from the surface of the detected site after each target ring beam passes through the detected site is received based on the photosensitive surface. The target ring beams may be formed on the surface of the detected site with the same geometric center. It should be noted that different target ring beams have different inner and outer radii. Both inner radius and outer radius refer to radius. The size of each target ring beam may be set according to actual situations, which is not specifically limited here. Different first light intensity values for the same target ring beam corresponding to the same predetermined wavelength correspond to different concentrations of the tissue element to be detected. That is, at least one first light intensity value for the same target ring beam corresponding to the same predetermined wavelength is acquired, and different first light intensity values correspond to different concentrations of the tissue element to be detected. In addition, different target ring beams may have the same or different ring widths, which may be set according to actual situations and is not specifically limited herein. The ring width of each target ring beam may be understood as a difference between the outer radius of the target ring beam and the inner radius of the target ring beam. Each target ring beam may be formed by a point-shaped light spot or by a beam projection. Different predetermined wavelengths may correspond to the same target ring beam or different target ring beams, which may be set according to actual situations and is not specifically limited here.

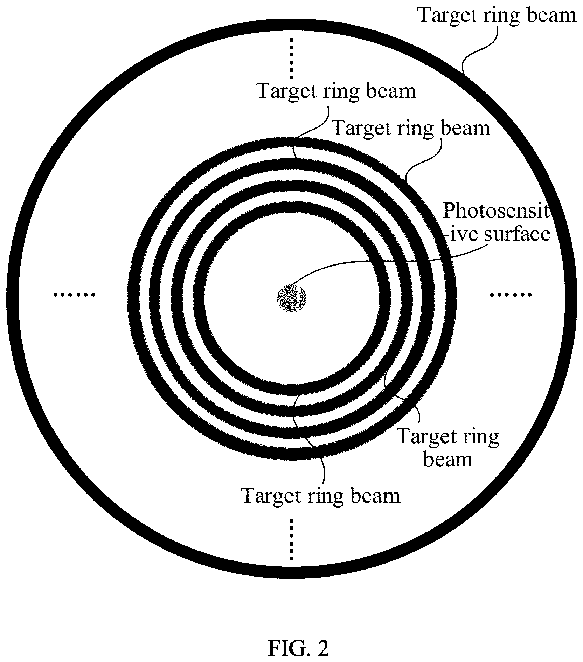

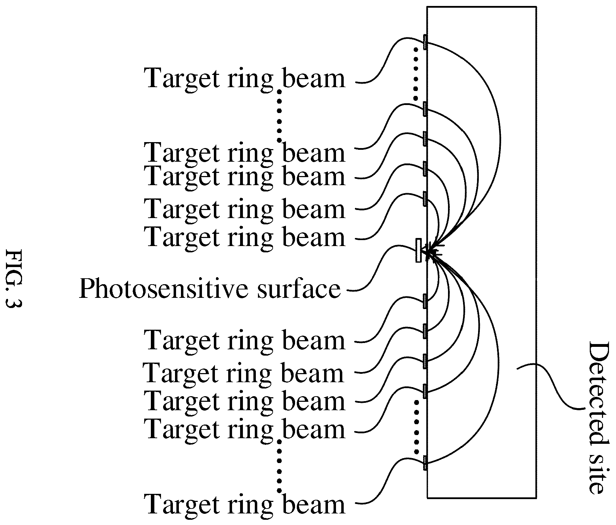

For example, FIG. 2 shows a schematic diagram of target ring beams formed on the surface of the detected site according to embodiments of the present disclosure. For the detected site of the detected object, *S* target ring beams from the inside to the outside corresponding to each predetermined wavelength are formed on the surface of the detected site, wherein *S*≥2. The ring width of each target ring beam may be 0.1 mm. An interval between two adjacent target ring beams may be 0.1 mm. Different target ring beams have the same geometric center. The photosensitive surface is arranged at a position corresponding to the geometric center, and the photosensitive surface may receive the first light intensity value emitted from the surface of the detected site after each target ring beam passes through the detected site. Based on the photosensitive surface, at least *S* first light intensity values emitted from the surface of the detected site after the *S* different target ring beams pass through the detected site may be respectively received, as shown in FIG. 3. FIG. 3 shows a schematic diagram of acquiring the first light intensity values based on the photosensitive surface according to embodiments of the present disclosure.

In addition, the first light intensity values corresponding to each predetermined wavelength may be acquired in two manners. In a first manner, the first light intensity values are acquired according to the predetermined wavelength. That is, for each predetermined wavelength, a plurality of target ring beams are formed on the surface of the detected site, and when each target ring beam is formed, the first light intensity value emitted from the surface of the detected site after the target ring beam passes through the detected site is received at the center of the target ring beam based on the photosensitive surface, so that the first light intensity value corresponding to each target ring beam for the predetermined wavelength may be acquired. In the same way, the first light intensity value corresponding to each target ring beam for each predetermined wavelength may be acquired. In a second manner, the first light intensity values are acquired according to the target ring beams. That is, the target ring beams of the same size corresponding to different predetermined wavelengths are sequentially formed on the surface of the detected site. When the target ring beam of the same size corresponding to each predetermined wavelength is formed, the first light intensity value emitted from the surface of the detected site after the target ring beam of the same size passes through the detected site is acquired at the center of the target ring beam based on the photosensitive surface. In this way, the first light intensity values corresponding to the target ring beams of the same size for the predetermined wavelengths may be acquired. In the same way, the first light intensity values respectively corresponding to the target ring beams of different sizes for the predetermined wavelengths may be acquired.

According to embodiments of the present disclosure, as the first light intensity value corresponding to each predetermined wavelength at each source-detection distance may be acquired by adjusting the size of the target ring beam, the first light intensity measurement value and the first light intensity reference value may be accurately determined, and then the measurement distance and the reference distance may be accurately determined. In addition, the emission and reception manners of the target ring beam and the photosensitive surface may greatly reduce the requirements for the photoelectric detector, thereby reducing the manufacturing cost and being easy for implementation. Moreover, a continuous detection may be achieved.

According to embodiments of the present disclosure, each target ring beam may be formed by the point-shaped light spot scanning or formed by the beam projection.

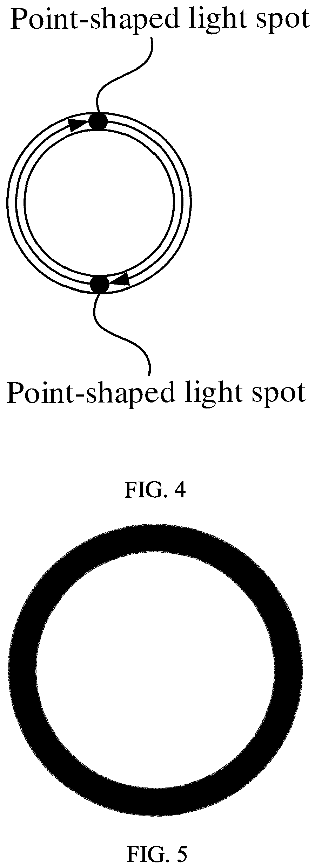

According to embodiments of the present disclosure, each target ring beam may be formed in two manners. A first manner is the point-shaped light spot scanning, and a second manner is the beam projection. For the first manner, FIG. 4 shows a schematic diagram of forming the target ring beam by the point-shaped light spot scanning according to embodiments of the present disclosure. For the second manner, FIG. 5 shows a schematic diagram of forming the target ring beam by the beam projection according to embodiments of the present disclosure.

According to embodiments of the present disclosure, the photosensitive surface is in contact or non-contact with the surface of the detected site.

According to embodiments of the present disclosure, a form of the non-invasive detection of the tissue element may include a contact detection and a non-contact detection. The contact detection may prevent interference light from being received by the photosensitive surface, thereby further improving the detection accuracy. The non-contact detection may avoid an influence of interfering factors such as temperature and pressure on the change of the light intensity value, thereby further improving the detection accuracy.

If the photosensitive surface is arranged in contact with the surface of the detected site, it may be considered that the non-invasive detection of the tissue element is the contact detection. It may be understood that the above may prevent the interference light from being received by the photosensitive surface, thereby further improving the detection accuracy.

If the photosensitive surface is arranged in non-contact with the surface of the detected site, the form of the non-invasive detection of tissue element may be determined according to whether the first light intensity value is acquired by the photosensitive surface through a light guide part, and whether the light guide part is in contact with the surface of the detected site if the first light intensity value is acquired through the light guide part. The light guide part includes a first end of the light guide part and a second end of the light guide part. A distance between the first end of the light guide part and the surface of the detected site is greater than a distance between the second end of the light guide part and the surface of the detected site. The first end of the light guide part and the second end of the light guide part are opposite end faces. The second end of the light guide part is in contact or non-contact with the surface of the detected site. The second end of the light guide part is an entrance of a light beam, that is, a light beam that is emitted after the target ring beam passes through the surface of the detected site may enter the light guide part through the second end of the light guide part and may be transmitted to the first end of the light guide part.

According to embodiments of the present disclosure, if the photosensitive surface is in non-contact with the surface of the detected site, and the photosensitive surface does not receive the first light intensity value through the light guide part, the form of the non-invasive detection of tissue element may be considered as the non-contact detection. If the first light intensity value is acquired by the photosensitive surface through the light guide part, the photosensitive surface needs to be arranged at the first end of the light guide part in order to achieve the non-contact between the photosensitive surface and the surface of the detected site. On this basis, the form of the non-invasive detection of tissue element is determined according to whether the second end of the light guide part is in contact with the surface of the detected site. That is, if the second end of the light guide part is in contact with the surface of the detected site, it may be considered that the form of the non-invasive detection of tissue element is the contact detection. If the second end of the light guide part is in non-contact with the surface of the detected site, it may be considered that the form of the non-invasive detection of tissue element is the non-contact detection.

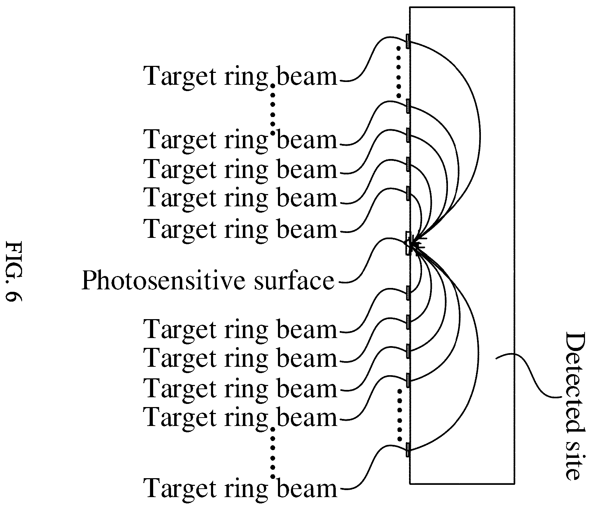

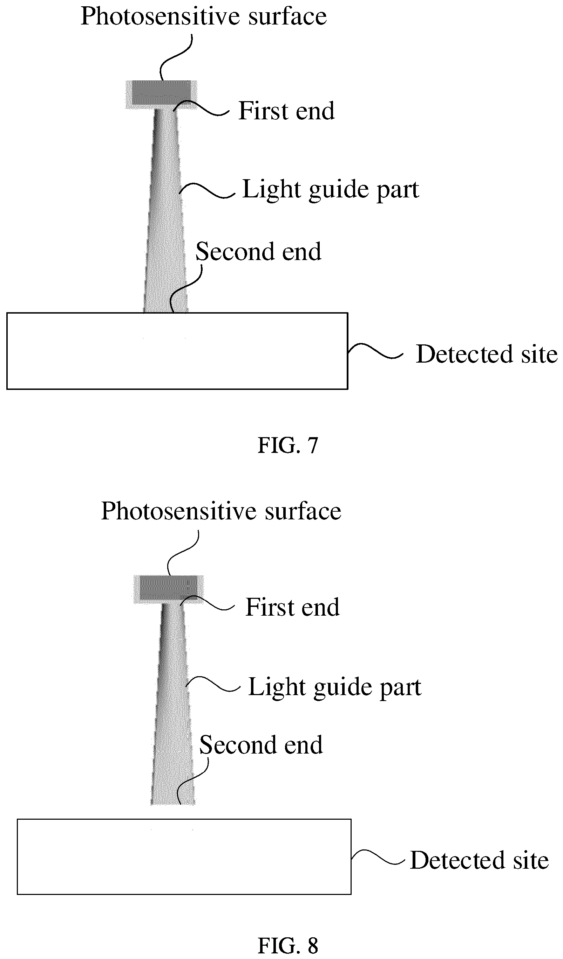

To sum up, the contact detection may include the following two manners. In a first manner, the photosensitive surface is in contact with the surface of the detected site, as shown in FIG. 6. FIG. 6 shows another schematic diagram of acquiring the first light intensity value based on the photosensitive surface according to embodiments of the present disclosure. In FIG. 6, the photosensitive surface is in contact with the surface of the detected site. In a second manner, the photosensitive surface is arranged at the first end of the light guide part, and the second end of the light guide part is in contact with the surface of the detected site, as shown in FIG. 7. FIG. 7 shows still another schematic diagram of acquiring the first light intensity value based on the photosensitive surface according to embodiments of the present disclosure. In FIG. 7, the second end of the light guide part is in contact with the surface of the detected site.

The non-contact detection may include the following two manners. In a first manner, the photosensitive surface is in non-contact with the surface of the detected site, and the photosensitive surface does not receive the first light intensity value through the light guide part, as shown in FIG. 3. In a second manner, the photosensitive surface is arranged at the first end of the light guide part, and the second end of the light guide part is in non-contact with the surface of the detected site, as shown in FIG. 8. FIG. 8 shows another schematic diagram of acquiring the first light intensity value based on the photosensitive surface according to embodiments of the present disclosure. In FIG. 8, the second end of the light guide part is in non-contact with the surface of the detected site.

According to embodiments of the present disclosure, the non-contact between the photosensitive surface and the surface of the detected site may be achieved by an arrangement that the photosensitive surface is arranged at the first end of the light guide part, the second end of the light guide part is in contact or non-contact with the surface of the detected site, and the first end of the light guide part and the second end of the light guide part are opposite end faces.

According to embodiments of the present disclosure, in order to achieve the non-contact between the photosensitive surface and the surface of the detected site, the photosensitive surface may be arranged at the first end of the light guide part. The first end of the light guide part is in non-contact with the surface of the detected site, that is, the photosensitive surface may be arranged on a first end face of the light guide part in non-contact with the surface of the detected site. The second end of the light guide part opposite to the first end of the light guide part may be in contact with the surface of the detected site, or may be in non-contact with the surface of the detected site, which may be set according to actual situations and is not specifically limited here. If the photosensitive surface is arranged at the first end of the light guide part, and the second end of the light guide part is in contact with the surface of the detected site, it may be considered that the form of the non-invasive detection of tissue element is the contact detection, as shown in FIG. 7. If the photosensitive surface is arranged at the first end of the light guide part, and the second end of the light guide part is in non-contact with the surface of the detected site, it may be considered that the form of the non-invasive detection of tissue element is the non-contact detection, as shown in FIG. 8.

According to embodiments of the present disclosure, the photosensitive surface is in non-contact with the surface of the detected site. Before acquiring the first light intensity value that is emitted from the surface of the detected site after each target ring beam passes through the detected site based on the photosensitive surface corresponding to the geometric center, the method may further include an operation of shielding interference light.

According to embodiments of the present disclosure, after the target ring beam is transmitted to the detected site, a part of the target ring beam may be directly reflected on the surface of the detected site to form a surface-reflected light, and a part of the target ring beam passes through the detected site and a diffusely-scattered light (i.e., the first light intensity value) is emitted from the surface of the detected site. The surface-reflected light does not interact with the tissue and therefore may not carry valid information. The valid information may be understood as a response caused by the change in the concentration of the tissue element to be detected in the detection process. Therefore, the surface-reflected light may be regarded as the interference light. The diffusely-scattered light interacts with the skin tissue and carries the valid information, and thus may be regarded as valid light.

If the photosensitive surface is in non-contact with the surface of the detected site, the surface-reflected light may be generated. Based on this, in order to further improve the detection accuracy, the interference light may be shielded before acquiring the first light intensity value emitted from the surface of the detected site after each target ring beam passes through the detected site based on the photosensitive surface corresponding to the geometric center, so that the first light intensity value emitted from the surface of the detected site after each target ring beam passes through the detected site is acquired based on the photosensitive surface. The interference light may be shielded in the following two manners.

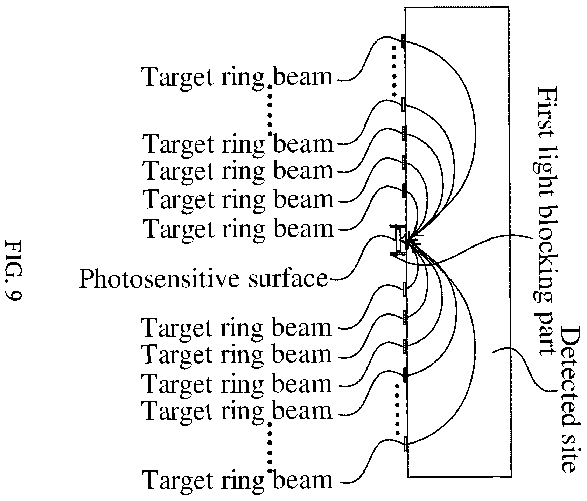

In a first manner, if the photosensitive surface is in non-contact with the surface of the detected site, and the photosensitive surface does not receive the first light intensity value through the light guide part, a first light blocking part may be provided in a gap region between the photosensitive surface and the surface of the detected site, and the first light blocking part is in contact with the surface of the detected site. The photosensitive surface is arranged on an inner side of the first light blocking part. The first light blocking part is integral with the photosensitive surface, or the first light blocking part is separate from the photosensitive surface, as shown in FIG. 9. FIG. 9 shows a schematic diagram of shielding the interference light according to embodiments of the present disclosure.

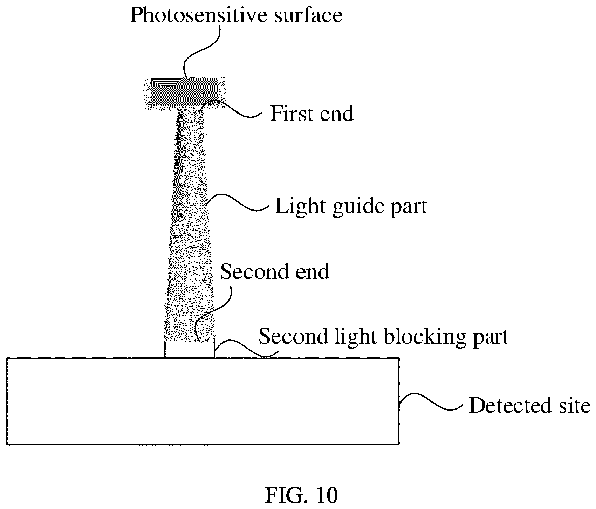

In a second manner, if the photosensitive surface is arranged at the first end of the light guide part, and the second end of the light guide part is in non-contact with the surface of the detected site, a second light blocking part may be provided in a gap region between the light guide part and the surface of the detected site, a first end of the second light blocking part is in contact with the second end of the light guide part, a second end of the second light blocking part is in contact with the surface of the detected site, and the second end of the second light blocking part and the first end of the second light blocking part are opposite end faces. A distance between the first end of the second light blocking part and the surface of the detected site is greater than a distance between the second end of the second light blocking part and the surface of the detected site, as shown in FIG. 10. FIG. 10 shows another schematic diagram of shielding the interference light according to embodiments of the present disclosure.

According to embodiments of the present disclosure, the interference light is shielded before acquiring the first light intensity value emitted from the surface of the detected site after each target ring beam passes through the detected site, so that only the diffusely-scattered light is acquired. As the diffusely-scattered light carries the valid information, the detection accuracy may be further improved.

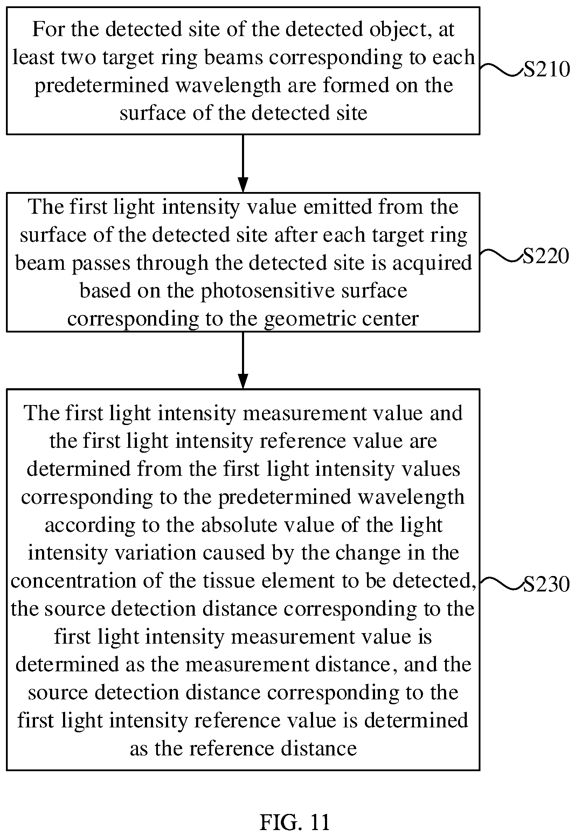

FIG. 11 shows a flowchart of another method of determining a distance in a non-invasive detection of a tissue element according to embodiments of the present disclosure. Such embodiments may be applied to improve the detection accuracy of the concentration of the tissue element to be detected.

As shown in FIG. 11, the method includes operations S210 to S220.

In operation S210, for the detected site of the detected object, at least two target ring beams corresponding to each predetermined wavelength are formed on the surface of the detected site.

According to embodiments of the present disclosure, different target ring beams have different source-detection distances, each source-detection distance is an inner radius or outer radius of the target ring beam, and different target ring beams have the same geometric center. The predetermined wavelength includes at least one predetermined wavelength. Each target ring beam is formed by the point-shaped spot scanning or the beam projection.

In operation S220, the first light intensity value emitted from the surface of the detected site after each target ring beam passes through the detected site is acquired based on the photosensitive surface corresponding to the geometric center.

According to embodiments of the present disclosure, the photosensitive surface is in contact or non-contact with the surface of the detected site. The non-contact between the photosensitive surface and the surface of the detected site may be achieved by an arrangement that the photosensitive surface is arranged at the first end of the light guide part, the second end of the light guide part is in contact or non-contact with the surface of the detected site, and the first end of the light guide part and the second end of the light guide part are opposite end faces. If the photosensitive surface is in non-contact with the surface of the detected site, the method may further include an operation of shielding interference light before operation S220.

In operation S230, the first light intensity measurement value and/or the first light intensity reference value are/is determined from the first light intensity values corresponding to the predetermined wavelength according to the absolute value of the light intensity variation caused by the change in the concentration of the tissue element to be detected, the source-detection distance corresponding to the first light intensity measurement value is determined as the measurement distance, and the source-detection distance corresponding to the first light intensity reference value is determined as the reference distance.

According to embodiments of the present disclosure, the first light intensity measurement value is the first light intensity value corresponding to the greatest absolute value of the light intensity variation caused by the change in the concentration of the tissue element to be detected, and the first light intensity reference value is the first light intensity value corresponding to the smallest absolute value of the light intensity variation caused by the change in the concentration of the tissue element to be detected. The light intensity variation caused by the change in the concentration of the tissue element to be detected is the variation between the first light intensity value and the corresponding predetermined light intensity value.

According to the technical solution of embodiments of the present disclosure, as the first light intensity value corresponding to each predetermined wavelength at each source-detection distance may be acquired by adjusting the size of the target ring beam, the first light intensity measurement value and/or the first light intensity reference value may be accurately determined, and then the measurement distance and the reference distance may be accurately determined. On this basis, the accurate determination of the measurement distance and the reference distance provides a basis for determining the concentration of the tissue element to be detected, thereby improving the detection accuracy. In addition, the emission and reception manners of the target ring beam and the photosensitive surface may greatly reduce the requirements for the photoelectric detector, thereby reducing the manufacturing cost and being easy for implementation, while achieving the continuous detection.

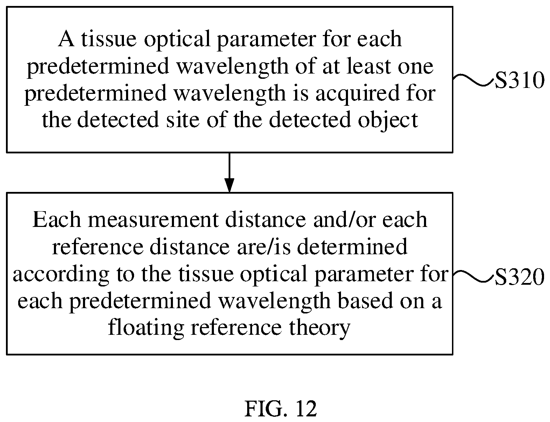

FIG. 12 shows a flowchart of still another method of determining a distance in a non-invasive detection of a tissue element according to embodiments of the present disclosure. Such embodiments may be applied to improve the detection accuracy of the concentration of the tissue element to be detected.

As shown in FIG. 12, the method includes operations S310 to S320.

In operation S310, a tissue optical parameter corresponding to each predetermined wavelength of at least one predetermined wavelength and a tissue optical parameter change relationship caused by the change in the concentration of the tissue element to be detected are acquired for the detected site of the detected object.

In operation S320, each measurement distance and/or each reference distance are/is determined according to the tissue optical parameter corresponding to each predetermined wavelength and the tissue optical parameter change relationship caused by the change in the concentration of the tissue element to be detected.

According to embodiments of the present disclosure, for a body, a body tissue may be simplified into a complex medium constituted by a scattering body and a scattering background, when an incident beam enters the tissue, absorption and scattering may occur, the absorption may directly cause an attenuation of light energy, and the scattering may affect a light energy distribution by changing a transmitting direction of photon, a light intensity value of diffusely-scattered light emitted from the surface of the detected site is a result of a combined effect of the absorption and the scattering. The absorption and the scattering may be reflected by the tissue optical parameter and the tissue optical parameter change caused by the change in the concentration of the tissue element to be detected. According to the above, the measurement distance and the reference distance are determined by the absorption and the scattering in different cases. Therefore, for the detected site of the detected object, in order to acquire the measurement distance and the reference distance corresponding to each predetermined wavelength, the tissue optical parameter corresponding to each predetermined wavelength and the tissue optical parameter change relationship caused by the change in the concentration of the tissue element to be detected may be acquired. For the tissue optical parameter corresponding to each predetermined wavelength and the tissue optical parameter change relationship caused by the change in the concentration of the tissue element to be detected, reference may be made to the above description.

After the tissue optical parameter corresponding to each predetermined wavelength is acquired, the measurement distance and/or the reference distance corresponding to each predetermined wavelength may be determined according to the tissue optical parameter corresponding to each predetermined wavelength and the tissue optical parameter change relationship caused by the change in the concentration of the tissue element to be detected. That is, for each predetermined wavelength, the measurement distance and/or the reference distance corresponding to the predetermined wavelength may be determined according to the tissue optical parameter corresponding to the predetermined wavelength and the tissue optical parameter change relationship caused by the change in the concentration of the tissue element to be detected. The measurement distance and/or the reference distance corresponding to each predetermined wavelength may be determined according to the tissue optical parameter corresponding to each predetermined wavelength and the tissue optical parameter change relationship caused by the change in the concentration of the tissue element to be detected based on the floating reference theory. It should be noted that a premise is that the detected site of the detected object is determined. In other words, the above-mentioned measurement distance and the reference distance corresponding to each predetermined wavelength correspond to the detected site of the detected object.

According to embodiments of the present disclosure, the corresponding measurement distance and/or reference distance may be determined through the tissue optical parameter and the tissue optical parameter change relationship caused by the change in the concentration of the tissue element to be detected.

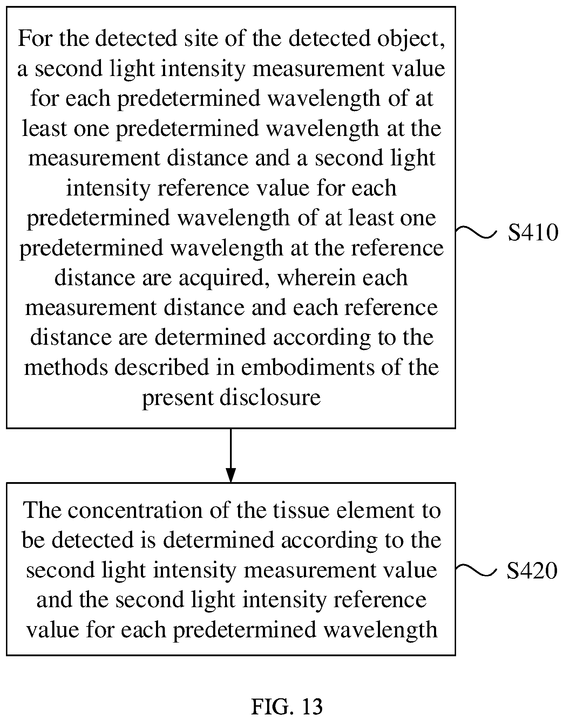

FIG. 13 shows a flowchart of a non-invasive detection method for a tissue element according to embodiments of the present disclosure. Such embodiments may be applied to improve the detection accuracy of the concentration of the tissue element to be detected.

As shown in FIG. 13, the method includes operations S410 to S420.

In operation S410, for the detected site of the detected object, a second light intensity measurement value corresponding to each predetermined wavelength of at least one predetermined wavelength at the measurement distance and/or a second light intensity reference value corresponding to each predetermined wavelength of at least one predetermined wavelength at the reference distance are/is acquired. Each measurement distance and each reference distance are determined according to the methods of determining the distance in the non-invasive detection of the tissue element according to embodiments of the present disclosure.

According to embodiments of the present disclosure, in order to determine the concentration of the tissue element to be detected, the second light intensity measurement value and/or the second light intensity reference value corresponding to each predetermined wavelength may be acquired for the detected site of the detected object. The second light intensity measurement value may be a second light intensity value corresponding to each predetermined wavelength at the measurement distance. The second light intensity reference value may be a second light intensity value corresponding to each predetermined wavelength at the reference distance. It should be noted that different predetermined wavelengths may correspond to the same measurement distance or different measurement distances, and different predetermined wavelengths may correspond to the same reference distance or different reference distances. Each measurement distance and each reference distance may be determined according to the methods described in embodiments of the present disclosure, including the following two methods.

In a first method, for the detected site of the detected object, each measurement distance and each reference distance may be determined by analyzing, for each predetermined wavelength, the acquired at least one first light intensity value corresponding to each source-detection distance. That is, for each predetermined wavelength, at least one first light intensity value corresponding to each source-detection distance is acquired, and each first light intensity value is analyzed, so as to determine a measurement distance and/or a reference distance corresponding to the predetermined wavelength. In other words, for the detected site of the detected object, at least one first light intensity value corresponding to each predetermined wavelength at each source-detection distance is acquired. The first light intensity measurement value and/or the first light intensity reference value may be determined from the first light intensity values corresponding to the predetermined wavelength according to the absolute value of the light intensity variation caused by the change in the concentration of the tissue element to be detected, the source-detection distance corresponding to the first light intensity measurement value is determined as the measurement distance, and the source-detection distance corresponding to the first light intensity reference value is determined as the reference distance. The acquiring at least one first light intensity value corresponding to each predetermined wavelength at each source-detection distance for the detected site of the detected object may be understood as follows. In a first manner, for the detected site of the detected object, at least two target ring beams corresponding to each predetermined wavelength are formed on the surface of the detected site, different target ring beams correspond to different source-detection distances, each source-detection distance is the inner radius or outer radius of the target ring beam, and different target ring beams have the same geometric center. At least one first light intensity value emitted from the surface of the detected site after each target ring beam passes through the detected site is acquired based on the photosensitive surface corresponding to the geometric center. In a second manner, for the detected site of the detected object, the tissue optical parameter and the skin structure parameter corresponding to each predetermined wavelength in the three-layer skin tissue model are acquired. Based on the Monte Carlo simulation, the first light intensity value corresponding to each predetermined wavelength at each source-detection distance is determined according to each tissue optical parameter, each skin tissue structure parameter, the tissue optical parameter change relationship caused by the change in the concentration of the tissue element to be detected, at least two predetermined source-detection distances and the predetermined incident photon number.

In a second method, for the detected site of the detected object, the tissue optical parameter corresponding to each predetermined wavelength and the tissue optical parameter change relationship caused by the change in the concentration of the tissue element to be detected are acquired. Each measurement distance and/or each reference distance may be determined according to the tissue optical parameter for each predetermined wavelength and the tissue optical parameter change relationship caused by the change in the concentration of the tissue element to be detected.

According to embodiments of the present disclosure, the acquiring the second light intensity measurement value corresponding to each predetermined wavelength at the measurement distance and/or the second light intensity reference value corresponding to each predetermined wavelength at the reference distance for the detected site of the detected object may be understood as follows. For each predetermined wavelength, the second light intensity measurement value corresponding to the predetermined wavelength at the measurement distance may be acquired; or the second light intensity reference value corresponding to the predetermined wavelength at the reference distance may be acquired; or the second light intensity measurement value corresponding to the predetermined wavelength at the measurement distance and the second light intensity reference value corresponding to the predetermined wavelength at the reference distance may be acquired. For at least one predetermined wavelength, there may be the following cases.

In a first case, only the second light intensity measurement value corresponding to each predetermined wavelength is acquired. In a second case, the second light intensity measurement value and the second light intensity reference value corresponding to each wavelength are acquired. In a third case, at least one second light intensity measurement value corresponding to at least one predetermined wavelength among the predetermined wavelengths is acquired, and at least one second light intensity reference value corresponding to at least one other predetermined wavelength among the predetermined wavelengths is acquired. In a fourth case, at least one second light intensity measurement value and at least one second light intensity reference value corresponding to at least one predetermined wavelength among the predetermined wavelengths are acquired, and at least one second light intensity reference value corresponding to at least one other predetermined wavelength among the predetermined wavelengths is acquired. In a fifth case, at least one second light intensity measurement value and at least one second light intensity reference value corresponding to at least one predetermined wavelength among the predetermined wavelengths are acquired, and at least one second light intensity measurement value corresponding to at least one other predetermined wavelength among the predetermined wavelengths is acquired. For each predetermined wavelength, the acquiring the second light intensity measurement value and/or the second light intensity reference value corresponding to the predetermined wavelength may be set according to actual situations, which is not specifically limited herein.

According to embodiments of the present disclosure, the second light intensity measurement value and/or the second light intensity reference value may be accurately determined, and the concentration of the tissue element to be detected may be determined according to the accurately determined second light intensity measurement value and/or second light intensity reference value, so that the detection accuracy may be improved.

In operation S420, the concentration of the tissue element to be detected is determined according to the second light intensity measurement value and/or the second light intensity reference value corresponding to each predetermined wavelength.

According to embodiments of the present disclosure, after each light intensity value corresponding to each predetermined wavelength is acquired, the concentration of the tissue element to be detected may be determined according to the second light intensity measurement value and/or the second light intensity reference value corresponding to each predetermined wavelength. That is, for at least one predetermined wavelength, there may be the following cases.

In a first case, only the second light intensity measurement value corresponding to each predetermined wavelength is acquired. In this case, the concentration of the tissue element to be detected may be determined according to the second light intensity measurement value corresponding to each predetermined wavelength.

In a second case, the second light intensity measurement value and the second light intensity reference value corresponding to each wavelength are acquired. In this case, the concentration of the tissue element to be detected may be determined using a difference operation. That is, for each predetermined wavelength, a difference operation is performed between the second light intensity measurement value and the second light intensity reference value corresponding to the predetermined wavelength, so as to obtain a light intensity difference value. The concentration of the tissue element to be detected is determined according to the light intensity difference value corresponding to each predetermined wavelength. The difference operation is performed because the second light intensity reference value corresponding to the reference distance reflects the response caused by the interference other than the change in the concentration of the tissue element to be detected in the detection process, while the second light intensity measurement value corresponding to the measurement distance reflects the response of the tissue element to be detected and the response of the interference other than the tissue element to be detected. Therefore, a reference-measurement may be used, that is, the second light intensity reference value corresponding to the reference distance may be used to correct the second light intensity measurement value corresponding to the measurement distance, so as to eliminate common mode interference to the greatest extent, thereby further improving the detection accuracy.

In a third case, at least one second light intensity measurement value corresponding to at least one predetermined wavelength among the predetermined wavelengths is acquired, and at least one second light intensity reference value corresponding to at least one other predetermined wavelength among the predetermined wavelengths is acquired. In this case, the concentration of the tissue element to be detected may be determined according to the at least one second light intensity measurement value and the at least one second light intensity reference value corresponding to respective predetermined wavelength(s).

In a fourth case, at least one second light intensity measurement value and at least one second light intensity reference value corresponding to at least one predetermined wavelength among the predetermined wavelengths are acquired, and at least one second light intensity reference value corresponding to at least one other predetermined wavelength among the predetermined wavelengths is acquired. In this case, the concentration of the tissue element to be detected may be determined using a difference operation. That is, for a predetermined wavelength corresponding to which the second light intensity measurement value and the second light intensity reference value are acquired, a difference operation is performed between the second light intensity measurement value and the second light intensity reference value corresponding to the predetermined wavelength, so as to obtain a light intensity difference value. The concentration of the tissue element to be detected is determined according to the light intensity difference value(s) corresponding to the at least one predetermined wavelength among the predetermined wavelengths and the at least one second light intensity reference value corresponding to the at least one other predetermined wavelength among the predetermined wavelengths. Therefore, the reference-measurement may be used, that is, the second light intensity reference value corresponding to the reference distance may be used to correct the second light intensity measurement value corresponding to the measurement distance, so as to eliminate common mode interference to the greatest extent, thereby further improving the detection accuracy.

In a fifth case, at least one second light intensity measurement value and at least one second light intensity reference value corresponding to at least one predetermined wavelength among the predetermined wavelengths are acquired, and at least one second light intensity measurement value corresponding to at least one other predetermined wavelength among the predetermined wavelengths is acquired. In this case, the concentration of the tissue element to be detected may be determined using a difference operation. That is, for a predetermined wavelength corresponding to which the second light intensity measurement value and the second light intensity reference value are acquired, a difference operation is performed between the second light intensity measurement value and the second light intensity reference value corresponding to the predetermined wavelength, so as to obtain a light intensity difference value. The concentration of the tissue element to be detected is determined according to the light intensity difference value(s) corresponding to the at least one predetermined wavelength among the predetermined wavelengths and the at least one second light intensity measurement value corresponding to the at least one other predetermined wavelength among the predetermined wavelengths. Therefore, the reference-measurement may be used, that is, the second light intensity reference value corresponding to the reference distance may be used to correct the second light intensity measurement value corresponding to the measurement distance, so as to eliminate common mode interference to the greatest extent, thereby further improving the detection accuracy.

According to the technical solution of embodiments of the present disclosure, the measurement distance and/or the reference distance corresponding to each predetermined wavelength may be accurately acquired for the detected site of the detected object. Therefore, the second light intensity measurement value and/or the second light intensity reference value may be accurately determined according to the accurately determined measurement distance and/or reference distance. Because the concentration of the tissue element to be detected is determined according to the accurately determined second light intensity measurement value and/or the second light intensity reference value, the detection accuracy may be improved.

According to embodiments of the present disclosure, operation 410 may be performed as follows. For the detected site of the detected object, a measurement ring beam and/or a reference ring beam corresponding to each predetermined wavelength are/is formed on the surface of the detected site. An inner radius or outer radius of each measurement ring beam is the corresponding measurement distance, an inner radius or outer radius of each reference ring beam is the corresponding reference distance, and each measurement ring beam and each reference ring beam have the same geometric center. The second light intensity measurement value emitted from the surface of the detected site after each measurement ring beam passes through the detected site is acquired based on the photosensitive surface corresponding to the geometric center, and/or the second light intensity reference value emitted from the surface of the detected site after each reference ring beam passes through the detected site is acquired based on the photosensitive surface corresponding to the geometric center.

According to embodiments of the present disclosure, the second light intensity measurement value and/or the second light intensity reference value may be acquired in the following manners. For the detected site of the detected object, a measurement ring beam and/or a reference ring beam corresponding to each predetermined wavelength may be formed on the surface of the detected site. That is, when the detected site of the detected object is determined, a measurement ring beam and/or a reference ring beam corresponding to each predetermined wavelength may be formed. The inner radius or outer radius of each measurement ring beam is the corresponding measurement distance, that is, each measurement ring beam may be a beam of which the source-detection distance from the emission position is the corresponding measurement distance. The inner radius or outer radius of each reference ring beam is the corresponding reference distance, that is, each reference ring beam may be a beam of which the source-detection distance from the emission position is the corresponding reference distance. As the photosensitive surface is provided corresponding to the emission position, each measurement ring beam may be a beam of which the source-detection distance from the photosensitive surface is the corresponding measurement distance, and each reference ring beam may be a beam of which the source-detection distance from the photosensitive surface is the corresponding reference distance. The measurement ring beam corresponds to the measurement distance, and the reference ring beam corresponds to the reference distance. It should be noted that each measurement ring beam and each reference ring beam may be formed by the point-shaped light spot scanning or by the beam projection.

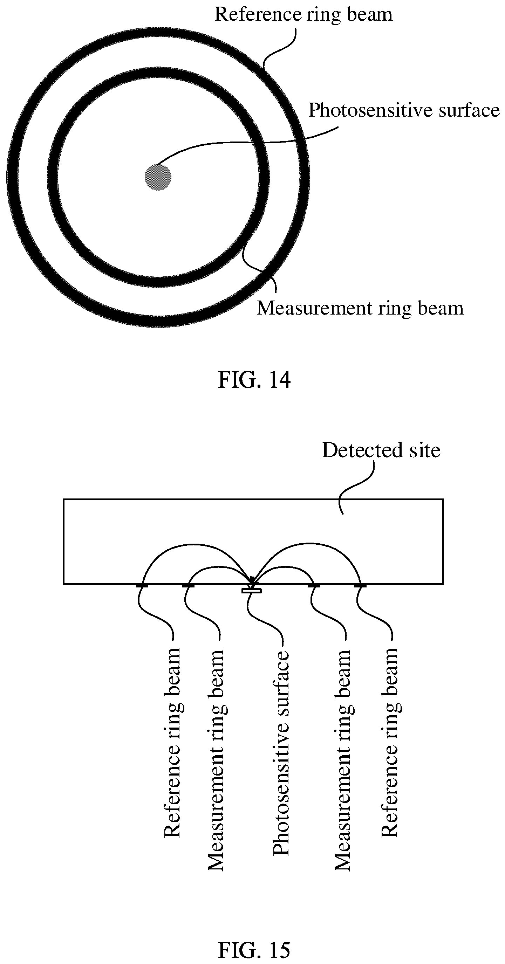

For example, as shown in FIG. 14, FIG. 14 shows a schematic diagram of the measurement ring beam and the reference ring beam formed on the surface of the detected site according to embodiments of the present disclosure. For the detected site of the detected object, a measurement ring beam and a reference ring beam corresponding to each predetermined wavelength are formed on the surface of the detected site. The measurement ring beam and the reference ring beam have the same geometric center. The photosensitive surface is provided at a position corresponding to the geometric center. The photosensitive surface may receive the second light intensity measurement value emitted from the surface of the detected site after each measurement ring beam passes through the detected site, and the second light intensity reference value emitted from the surface of the detected site after each reference ring beam passes through the detected site. As shown in FIG. 15, FIG. 15 shows a schematic diagram of acquiring the second light intensity measurement value and the second light intensity reference value based on the photosensitive surface according to embodiments of the present disclosure.

According to embodiments of the present disclosure, the measurement distance and/or the reference distance may be accurately determined, and the second light intensity measurement value and/or the second light intensity reference value may be accurately determined according to the accurately determined measurement distance and/or reference distance, in combination with the method of forming the measurement ring beam and/or the reference ring beam. Since the concentration of the tissue element to be detected is determined according to the accurately determined second light intensity measurement value and/or the second light intensity reference value, the detection accuracy may be improved.

According to embodiments of the present disclosure, each measurement ring beam is formed by the point-shaped light spot scanning or the beam projection, and each reference ring beam is formed by the point-shaped light spot scanning or the beam projection.

According to embodiments of the present disclosure, each measurement ring beam and each reference ring beam may be formed by two manners. A first manner is the point-shaped light spot scanning, and a second manner is the beam projection. For the first manner, reference may be made to FIG. 4. For the second manner, reference may be made to FIG. 5.

According to embodiments of the present disclosure, operation 420 may include the following operations. For each predetermined wavelength, a difference operation is performed between the second light intensity measurement value and the second light intensity reference value corresponding to the predetermined wavelength, so as to obtain a light intensity difference value. The concentration of the tissue element to be detected may be determined according to the light intensity difference value corresponding to each predetermined wavelength.

According to embodiments of the present disclosure, in order to further improve the detection accuracy, for each predetermined wavelength, a difference operation is performed between the second light intensity measurement value and the second light intensity reference value corresponding to the predetermined wavelength, so as to obtain the light intensity difference value corresponding to the predetermined wavelength. Based on this, the light intensity difference value corresponding to each predetermined wavelength may be obtained, and the concentration of the tissue element to be detected may be determined according to the light intensity difference value corresponding to each predetermined wavelength. The determining the concentration of the tissue element to be detected according to the light intensity difference value corresponding to each predetermined wavelength may be understood as follows. The light intensity difference value corresponding to each predetermined wavelength may be input into a pre-trained tissue element prediction model to obtain a prediction result, and the prediction result is the concentration of the tissue element to be detected. The specific calculation process may refer to patent document published on November 23, 2005 with a publication number CN1699973A, which will not be described in detail here.

According to embodiments of the present disclosure, the second light intensity reference value corresponding to the reference distance reflects the response caused by the interference other than the change in the concentration of the tissue element to be detected in the detection process, while the second light intensity measurement value corresponding to the measurement distance reflects the response of the tissue element to be detected and the response of the interference other than the tissue element to be detected. Therefore, the reference-measurement may be used, that is, the second light intensity reference value corresponding to the reference distance may be used to correct the second light intensity measurement value corresponding to the measurement distance, so as to eliminate common mode interference to the greatest extent, thereby further improving the detection accuracy.

As shown in FIG. 7, FIG. 8, FIG. 15 and FIG. 16, according to embodiments of the present disclosure, the photosensitive surface is in contact or non-contact with the surface of the detected site.

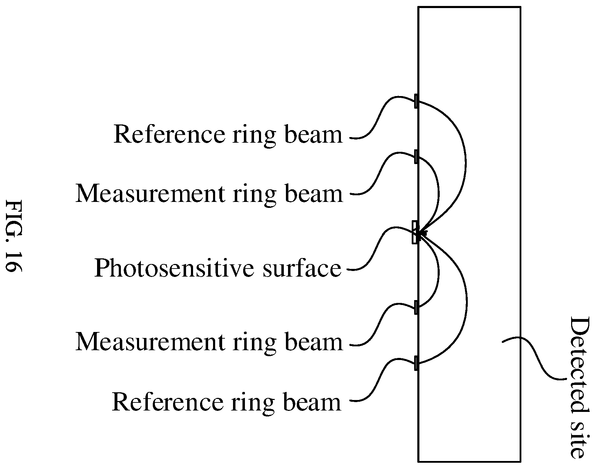

According to embodiments of the present disclosure, the form of the non-invasive detection for the tissue element may include a contact detection and a non-contact detection. The contact detection may include two manners. In a first manner, the photosensitive surface is in contact with the surface of the detected site, as shown in FIG. 16. FIG. 16 shows another schematic diagram of acquiring the second light intensity measurement value and the second light intensity reference value based on the photosensitive surface according to embodiments of the present disclosure. As shown in FIG. 16, the photosensitive surface is in contact with the surface of the detected site. In a second manner, the photosensitive surface is arranged at the first end of the light guide part, and the second end of the light guide part is in contact with the surface of the detected site, as shown in FIG. 7.

The non-contact detection may include two manners. In a first manner, the photosensitive surface is in non-contact with the surface of the detected site, and the photosensitive surface does not receive the first light intensity value through the light guide part, as shown in FIG. 15. In a second manner, the photosensitive surface is arranged at the first end of the light guide part, and the second end of the light guide part is in non-contact with the surface of the detected site, as shown in FIG. 8. It should be noted that for the description of the contact detection and the non-contact detection, reference may be made to the corresponding parts above, and details are not repeated here.

As shown in FIG. 7 and FIG. 8, according to embodiments of the present disclosure, the non-contact between the photosensitive surface and the surface of the detected site may be achieved by an arrangement that the photosensitive surface is arranged at the first end of the light guide part, the second end of the light guide part is in contact or non-contact with the surface of the detected site, and the first end of the light guide part and the second end of the light guide part are opposite end faces.

According to embodiments of the present disclosure, in order to achieve the non-contact between the photosensitive surface and the surface of the detected site, the photosensitive surface may be arranged at the first end of the light guide part. It should be noted that, for the description of arranging the photosensitive surface at the first end of the light guide part, reference may be made to the corresponding part above, and details are not repeated here.

According to embodiments of the present disclosure, the photosensitive surface is in non-contact with the surface of the detected site. Before acquiring the second light intensity measurement value that is emitted from the surface of the detected site after each measurement ring beam passes through the detected site based on the photosensitive surface corresponding to the geometric center and/or acquiring the second light intensity reference value that is emitted from the surface of the detected site after each reference ring beam passes through the detected site based on the photosensitive surface corresponding to the geometric center, the method may further include an operation of shielding an interference light.

According to embodiments of the present disclosure, after the measurement ring beam and/or the reference ring beam are/is transmitted to the detected site, a part of the measurement ring beam and a part of the reference ring beam may be directly reflected on the surface of the detected site to form a surface-reflected light, and a part of the measurement ring beam and a part of the reference ring beam passes through the detected site and a diffusely-scattered light (i.e., the second light intensity measurement value and the second light intensity reference value) is emitted from the surface of the detected site. The surface-reflected light does not interact with the tissue and therefore may not carry valid information. The valid information may be understood as a response caused by the change in the concentration of the tissue element to be detected in the detection process. Therefore, the surface-reflected light may be regarded as interference light. The diffusely-scattered light interacts with the skin tissue and carries valid information, and thus may be regarded as valid light.

If the photosensitive surface is in non-contact with the surface of the detected site, the surface-reflected light may be generated. Based on this, in order to further improve the detection accuracy, the interference light may be shielded before the second light intensity measurement value emitted from the surface of the detected site after each measurement ring beam passes through the detected site is acquired based on the photosensitive surface corresponding to the geometric center and/or the second light intensity reference value emitted from the surface of the detected site after each reference ring beam passes through the detected site is acquired based on the photosensitive surface corresponding to the geometric center, so that the second light intensity measurement value and/or the second light intensity reference value emitted from the surface of the detected site after each measurement ring beam and/or each reference ring beam pass/passes through the detected site are/is acquired based on the photosensitive surface. The interference light may be shielded in the following two manners.

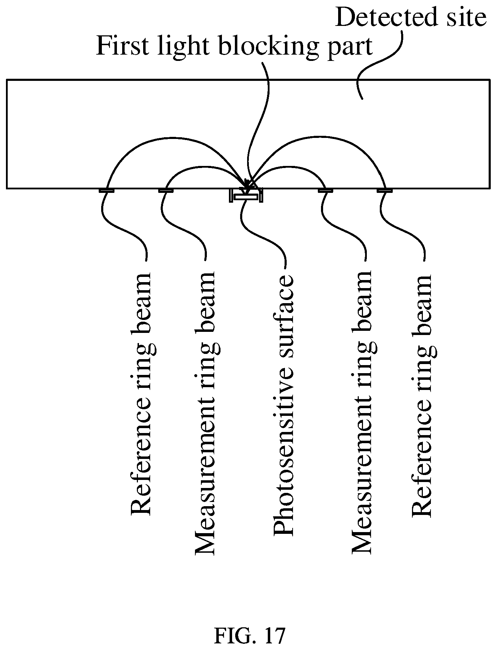

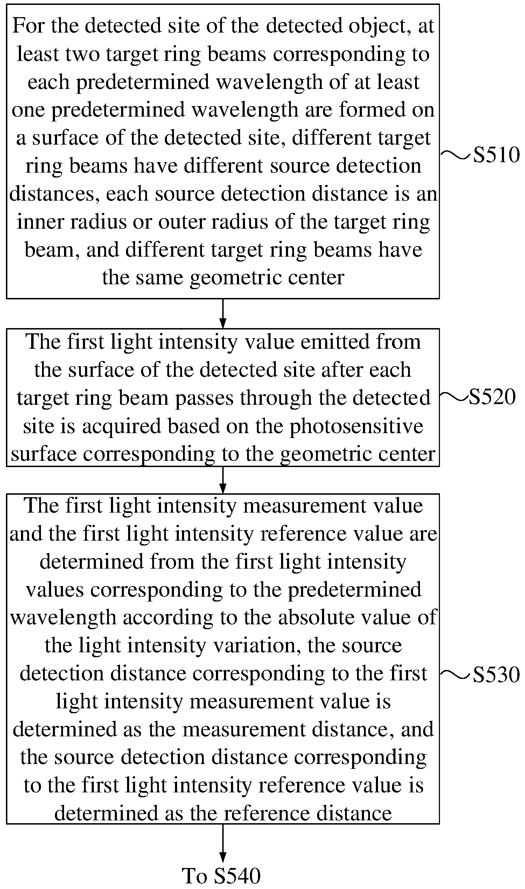

In a first manner, if the photosensitive surface is in non-contact with the surface of the detected site, and the photosensitive surface does not receive the second light intensity measurement value and the second light intensity reference value through the light guide part, a first light blocking part may be provided in a gap region between the photosensitive surface and the surface of the detected site, and the first light blocking part is in contact with the surface of the detected site. The photosensitive surface is arranged on an inner side of the first light blocking part. The first light blocking part is integral with the photosensitive surface or the first light blocking part is separate from the photosensitive surface, as shown in FIG. 17. FIG. 17 shows another schematic diagram of shielding the interference light according to embodiments of the present disclosure.

In a second manner, if the photosensitive surface is arranged at the first end of the light guide part, and the second end of the light guide part is in non-contact with the surface of the detected site, a second light blocking part may be provided in a gap region between the light guide part and the surface of the detected site, a first end of the second light blocking part is in contact with the second end of the light guide part, a second end of the second light blocking part is in contact with the surface of the detected site, and the second end of the second light blocking part and the first end of the second light blocking part are opposite end faces. A distance between the first end of the second light blocking part and the surface of the detected site is greater than a distance between the second end of the second light blocking part and the surface of the detected site, as shown in FIG. 10.

According to embodiments of the present disclosure, the interference light is shielded before acquiring the second light intensity measurement value and the second light intensity reference value emitted from the surface of the detected site after each measurement ring beam and/or each reference ring beam pass/passes through the detected site, so that only the diffusely-scattered light is acquired by the photosensitive surface. As the diffusely-scattered light carries the valid information, the detection accuracy may be further improved.

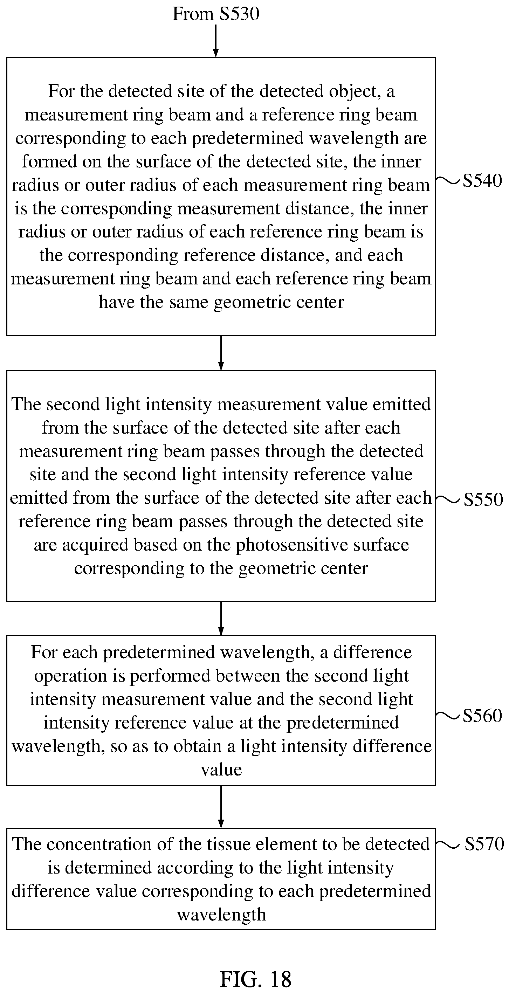

FIG. 18 shows a flowchart of another non-invasive detection method for a tissue element according to embodiments of the present disclosure. Such embodiments may be applied to improve the detection accuracy of the concentration of the tissue element to be detected.

As shown in FIG. 18, the method includes operations S510 to S570.

In operation S510, for the detected site of the detected object, at least two target ring beams corresponding to each predetermined wavelength are formed on a surface of the detected site.

According to embodiments of the present disclosure, different target ring beams have different source-detection distances, each source-detection distance is an inner radius or outer radius of the target ring beam, and different target ring beams have the same geometric center. The predetermined wavelength includes at least one predetermined wavelength. Each target ring beam is formed by the point-shaped spot scanning or the beam projection.

In operation S520, the first light intensity value emitted from the surface of the detected site after each target ring beam passes through the detected site is acquired based on the photosensitive surface corresponding to the geometric center.

In operation S530, the first light intensity measurement value and the first light intensity reference value are determined from the first light intensity values corresponding to the predetermined wavelength according to the absolute value of the light intensity variation caused by the change in the concentration of the tissue element to be detected, the source-detection distance corresponding to the first light intensity measurement value is determined as the measurement distance, and the source-detection distance corresponding to the first light intensity reference value is determined as the reference distance.

According to embodiments of the present disclosure, the first light intensity measurement value is the first light intensity value corresponding to the greatest absolute value of the light intensity variation caused by the change in the concentration of the tissue element to be detected, and the first light intensity reference value is the first light intensity value corresponding to the smallest absolute value of the light intensity variation caused by the change in the concentration of the tissue element to be detected. The light intensity variation caused by the change in the concentration of the tissue element to be detected is the variation between the first light intensity value and the corresponding predetermined light intensity value.

In operation S540, for the detected site of the detected object, a measurement ring beam and a reference ring beam corresponding to each predetermined wavelength are formed on the surface of the detected site.

According to embodiments of the present disclosure, the inner radius or outer radius of each measurement ring beam is the corresponding measurement distance, the inner radius or outer radius of each reference ring beam is the corresponding reference distance, and each measurement ring beam and each reference ring beam have the same geometric center. Each measurement ring beam is formed by the point-shaped light spot scanning or by the beam projection, and each reference ring beam is formed by the point-shaped light spot scanning or by the beam projection.

In operation S550, the second light intensity measurement value emitted from the surface of the detected site after each measurement ring beam passes through the detected site and the second light intensity reference value emitted from the surface of the detected site after each reference ring beam passes through the detected site are acquired based on the photosensitive surface corresponding to the geometric center.

In operation S560, for each predetermined wavelength, a difference operation is performed between the second light intensity measurement value and the second light intensity reference value at the predetermined wavelength, so as to obtain a light intensity difference value.

In operation S570, the concentration of the tissue element to be detected is determined according to the light intensity difference value corresponding to each predetermined wavelength.

According to embodiments of the present disclosure, the photosensitive surface is in contact or non-contact with the surface of the detected site. The non-contact between the photosensitive surface and the surface of the detected site may be achieved by an arrangement that the photosensitive surface is arranged at the first end of the light guide part, the second end of the light guide part is in contact or non-contact with the surface of the detected site, and the first end of the light guide part and the second end of the light guide part are opposite end faces. If the photosensitive surface is in non-contact with the surface of the detected site, the method may further include an operation of shielding interference light before operation S520, and an operation of shielding interference light before operation S550.

According to embodiments of the present disclosure, as the first light intensity value corresponding to each predetermined wavelength at each source-detection distance may be acquired by adjusting the size of the target ring beam, the first light intensity measurement value and/or the first light intensity reference value may be accurately determined, and the measurement distance and/or the reference distance may be accurately determined. On this basis, the second light intensity measurement value and/or the second light intensity reference value may be accurately determined according to the accurately determined measurement distance and/or reference distance, in combination with the method of forming the measurement ring beam and/or the reference ring beam. As the concentration of the tissue element to be detected is determined according to the accurately determined second light intensity measurement value and/or the second light intensity reference value, the detection accuracy may be improved. Through the difference operation, the common mode interference in the second light intensity reference value and the second light intensity measurement value may be eliminated, so that the detection accuracy may be further improved. In addition, the emission and reception manners of the target ring beam and the photosensitive surface may greatly reduce the requirements for the photoelectric detector, thereby reducing the manufacturing cost and being easy for implementation. Moreover, a continuous detection may be achieved.

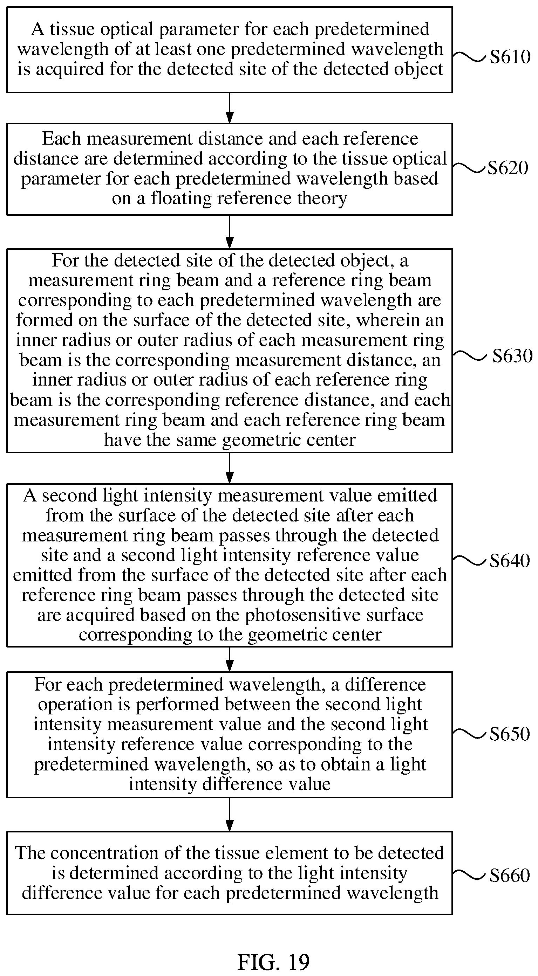

FIG. 19 shows a flowchart of still another non-invasive detection method for a tissue element according to embodiments of the present disclosure. Such embodiments may be applied to improve the detection accuracy of the concentration of the tissue element to be detected.

As shown in FIG. 19, the method includes operations S610 to S660.

In operation S610, a tissue optical parameter corresponding to each predetermined wavelength of at least one predetermined wavelength and a tissue optical parameter change relationship caused by the change in the concentration of the tissue element to be detected are acquired for the detected site of the detected object.

In operation S620, each measurement distance and each reference distance are determined according to the tissue optical parameter corresponding to each predetermined wavelength and the tissue optical parameter change relationship caused by the change in the concentration of the tissue element to be detected.

In operation S630, for the detected site of the detected object, a measurement ring beam and a reference ring beam corresponding to each predetermined wavelength are formed on the surface of the detected site.

According to embodiments of the present disclosure, an inner radius or outer radius of each measurement ring beam is the corresponding measurement distance, an inner radius or outer radius of each reference ring beam is the corresponding reference distance, and each measurement ring beam and each reference ring beam have the same geometric center. Each measurement ring beam is formed by the point-shaped light spot scanning or by the beam projection, and each reference ring beam is formed by the point-shaped light spot scanning or by the beam projection. The photosensitive surface is in contact or non-contact with the surface of the detected site. The non-contact between the photosensitive surface and the surface of the detected site may be achieved by an arrangement that the photosensitive surface is arranged at the first end of the light guide part, the second end of the light guide part is in contact or non-contact with the surface of the detected site, and the first end and the second end of the light guide part are opposite end faces.

In operation S640, the second light intensity measurement value emitted from the surface of the detected site after each measurement ring beam passes through the detected site and the second light intensity reference value emitted from the surface of the detected site after each reference ring beam passes through the detected site are acquired based on the photosensitive surface corresponding to the geometric center.

In operation S650, for each predetermined wavelength, a difference operation is performed between the second light intensity measurement value and the second light intensity reference value for the predetermined wavelength, so as to obtain a light intensity difference value.

In operation S660, the concentration of the tissue element to be detected is determined according to the light intensity difference value corresponding to each predetermined wavelength.

According to embodiments of the present disclosure, if the photosensitive surface is in non-contact with the surface of the detected site, the method may further include an operation of shielding interference light before operation S640.

The method of determining the distance in the non-invasive detection of the tissue element described in embodiments of the present disclosure may be performed by a device of determining a distance in a non-invasive detection of a tissue element, and the non-invasive detection method for the tissue element may be performed by a non-invasive detection device for a tissue element. The device of determining the distance in the non-invasive detection of the tissue element and the non-invasive detection device for the tissue element may be implemented in software and/or hardware, and the non-invasive detection device for the tissue element may be configured in a wearable apparatus, such as a smart watch.

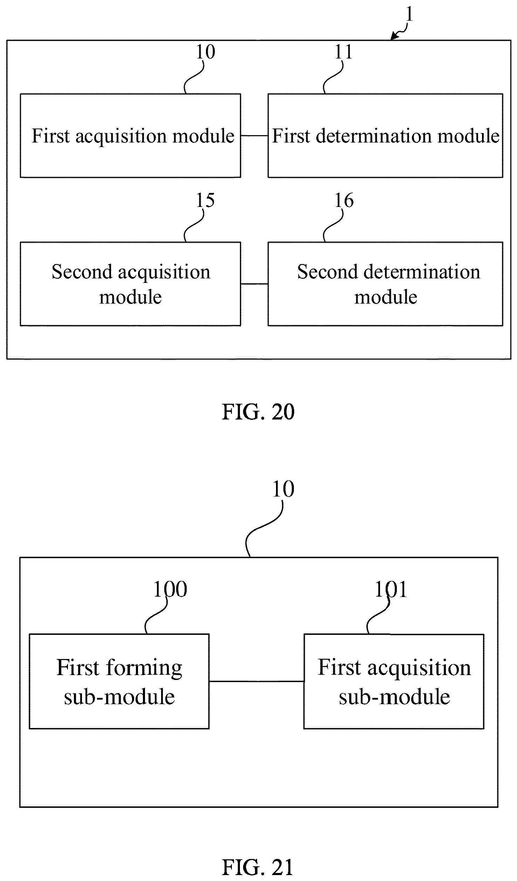

FIG. 20 shows a schematic structural diagram of a device of determining a distance in a non-invasive detection of a tissue element according to embodiments of the present disclosure. Such embodiments may be applied to improve the detection accuracy of the concentration of the tissue element to be detected.

As shown in FIG. 20, a device 1 of determining a distance in a non-invasive detection of a tissue element includes a first acquisition module 10 and a first determination module 11. A structure and an operating principle will be described below with reference to the accompanying drawings.

The first acquisition module 10 is used to acquire a first light intensity value corresponding to each predetermined wavelength of at least one predetermined wavelength at each source-detection distance of at least two source-detection distances for the detected site of the detected object.

The first determination module 11 is used to determine a first light intensity measurement value and/or a first light intensity reference value from the first light intensity values corresponding to the predetermined wavelength according to an absolute value of a light intensity variation caused by the change in the concentration of the tissue element to be detected, determine a source-detection distance corresponding to the first light intensity measurement value as a measurement distance, and determine a source-detection distance corresponding to the first light intensity reference value as a reference distance. The first light intensity measurement value is a first light intensity value corresponding to a greatest absolute value of the light intensity variation caused by the change in the concentration of the tissue element to be detected, the first light intensity reference value is a first light intensity value corresponding to a smallest absolute value of the light intensity variation caused by the change in the concentration of the tissue element to be detected, and the light intensity variation caused by the change in the concentration of the tissue element to be detected is a variation between the first light intensity value and the corresponding predetermined light intensity value.

According to embodiments of the present disclosure, for the specific processing procedures of the first acquisition module 10 and the first determination module 11, reference may be made to the above description in the corresponding part of the method of determining the distance in the non-invasive detection of the tissue element, and details are not repeated here.

As shown in FIG. 21, according to embodiments of the present disclosure, the first acquisition module 10 includes a first forming sub-module 100 and a first acquisition sub-module 101.

The first forming sub-module 100 is used to form, for the detected site of the detected object, at least two target ring beams corresponding to each predetermined wavelength on a surface of the detected site, where different target ring beams have different source-detection distances, each source-detection distance is an inner radius or outer radius of the target ring beam, and different target ring beams have the same geometric center.

The first acquisition sub-module 101 is used to acquire, based on the photosensitive surface corresponding to the geometric center, the first light intensity value emitted from the surface of the detected site after each target ring beam passes through the detected site.

According to embodiments of the present disclosure, for the specific processing procedures of the first forming sub-module 100 and the first acquisition sub-module 101, reference may be made to the above description in the corresponding part of the method of determining the distance in the non-invasive detection of the tissue element, and details are not repeated here.

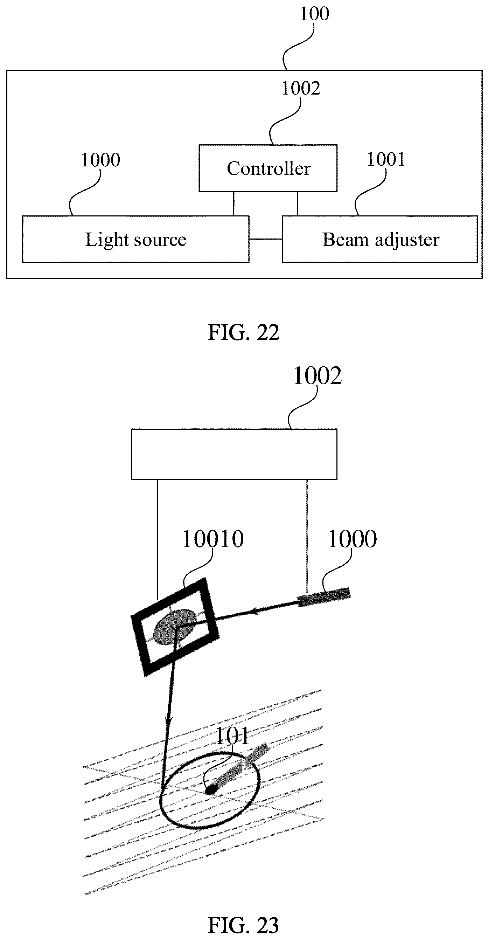

As shown in FIG. 22, according to embodiments of the present disclosure, the first forming sub-module 100 includes a light source 1000, a beam adjuster 1001 and a controller 1002. The controller 1002 may be communicatively connected with the light source 1000 and the beam adjuster 1001, respectively.

The controller 1002 is used to, for the detected site of the detected object, control the light source 1000 and the beam adjuster 1001 to cooperate to form at least two target ring beams corresponding to each predetermined wavelength on the surface of the detected site, according to a corresponding operating state instruction.

According to embodiments of the present disclosure, the operating state instruction may be an instruction for controlling operating states of the light source 1000 and the beam adjuster 1001. For the detected site of the detected object, the controller 1002 may control the light source 1000 and the beam adjuster 1001 to cooperate to form at least two target ring beams corresponding to each predetermined wavelength on the surface of the detected site, according to the corresponding operating state instruction. It may be understood that the controller 1002 may control the light source 1000 and the beam adjuster 1001 to cooperate to form each target ring beam corresponding to each predetermined wavelength, according to the corresponding operating state instruction.

As shown in FIG. 23, according to embodiments of the present disclosure, the beam adjuster 1001 includes a Micro-electromechanical System (MEMS) scanning mirror 10010.

The controller 1002 is used to, for the detected site of the detected object, control the light source 1000, according to a corresponding operating state instruction, to emit an incident beam corresponding to each predetermined wavelength and project each incident beam to the MEMS scanning mirror 10010, and control the MEMS scanning mirror 10010, according to the corresponding operating state instruction, to convert each incident beam into a corresponding target ring beam and project each target ring beam to the surface of the detected site.

According to embodiments of the present disclosure, the light source 1000 and the MEMS scanning mirror 10010 may cooperate to form each target ring beam under the control of the controller 1002. The MEMS scanning mirror 10010 may be a two-dimensional MEMS scanning mirror.

According to embodiments of the present disclosure, the controller 1002 may synchronously control the light source 1000 and the MEMS scanning mirror 10010 to achieve a two-dimensional scanning image composed of predetermined pixel points by scanning progressively. If a trajectory formed by the predetermined pixel points is a target ring, then the two-dimensional scanning image is a target ring image. The above-mentioned scanning method enables a determination of display time instants and spatial coordinates of the predetermined pixel points in the target ring image. The spatial coordinates of the predetermined pixel points in the target ring image are determined by a deflection angle of the MEMS scanning mirror 10010. The display time instants of the predetermined pixel points in the target ring image are determined by the light source 1000. That is, the light source 1000 and the MEMS scanning mirror 10010 may be synchronously controlled by the controller 1002, so as to achieve a correspondence between the display time instant and the spatial coordinate of the predetermined pixel point. Different predetermined pixel points may form target ring images having different sizes. The target ring images of different sizes may be projected to the detected site to form target ring beams of different sizes. It should be noted that the spatial coordinate and the display time instant of the predetermined pixel point may be embodied in the operating state instruction.

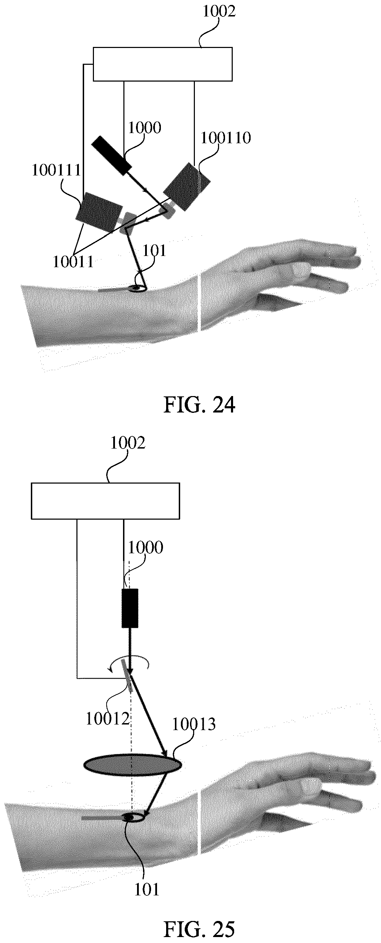

As shown in FIG. 24, according to embodiments of the present disclosure, the beam adjuster 1001 may include a galvo scanner assembly 10011.

The controller 1002 is used to, for the detected site of the detected object, control the light source 1000, according to a corresponding operating state instruction, to emit an incident beam corresponding to each predetermined wavelength and project each incident beam to the galvo scanner assembly 10011, and control the galvo scanner assembly 10011, according to the corresponding operating state instruction, to convert each incident beam into a corresponding target ring beam and project each target ring beam to the surface of the detected site.

According to embodiments of the present disclosure, the light source 1000 and the galvo scanner assembly 10011 may cooperate to form each target ring beam under the control of the controller 1002. That is, the incident beam corresponding to each predetermined wavelength that is emitted by the light source 1000 under the control of the controller 1002 according to the operating state instruction may be projected to the scanning position by the galvo scanner assembly 10011 under the control of the controller 1002 according to the operating state instruction. The incident beam may be a point-shaped light spot at the scanning position. Through 360° circular scanning of the point-shaped light spot and by forming a target ring beam with a variable size, target ring beams having different sizes may be formed.

As shown in FIG. 24, according to embodiments of the present disclosure, the galvo scanner assembly 10011 includes a first dual-axis galvo scanner 100110 and a second dual-axis galvo scanner 100111.

The controller 1002 is used to, for the detected site of the detected object, control the light source 1000, according to a corresponding operating state instruction, to emit an incident beam corresponding to each predetermined wavelength and project each incident beam to the first dual-axis galvo scanner 100110.

The controller 1002 is used to control the first dual-axis galvo scanner 100110, according to the corresponding operating state instruction, to deflect a first predetermined angle along an X-axis so that each incident beam is deflected by the first predetermined angle in the X-axis direction, and project the deflected incident beam to the second dual-axis galvo scanner 100111.

The controller 1002 is used to control the second dual-axis galvo scanner 100111, according to the operating state instruction, to deflect a second predetermined angle along a Y-axis to form each target ring beam, and project each target ring beam to the surface of the detected site.

According to embodiments of the present disclosure, the galvo scanner assembly 10011 includes a first dual-axis galvo scanner 100110 and a second dual-axis galvo scanner 100111, and the controller 1002 may control a change of the deflection direction of the first dual-axis galvo scanner 100110 and the second dual-axis galvo scanner 100111, so as to achieve a 360° rotation of the incident beam, that is, to achieve the circular scanning. In addition, the controller 1002 may control a change of the deflection angle of the first dual-axis galvo scanner 100110 and the second dual-axis galvo scanner 100111, so as to form a target ring beam with variable size by scanning, that is, to form target ring beams of different sizes. The controller 1002 may control the first dual-axis galvo scanner 100110 to deflect the first predetermined angle along the X-axis so that each incident beam is deflected by the first predetermined angle in the X-axis direction along with the first dual-axis galvo scanner 100110, and project the deflected incident beam to the second dual-axis galvo scanner 100111. The controller 1002 may control the second dual-axis galvo scanner 100111 to deflect the second predetermined angle along the Y-axis so that each deflected incident beam is deflected by the second predetermined angle in the Y-axis direction along with the second dual-axis galvo scanner 100111, so as to form each target ring beam. The above-mentioned first predetermined angle and second predetermined angle may be configured to determine that the incident beam is projected to the scanning position, and the incident beam is a point-shaped light spot at the scanning position. The controller 1002 may control the deflection angle and deflection direction of the first dual-axis galvo scanner 100110 and the second dual-axis galvo scanner 100111, so as to achieve the 360° rotation of the point-shaped light spot on the surface of the detected site, that is, to achieve the circular scanning. In addition, the target ring beam with variable size is formed by scanning, that is, target ring beams of different sizes are formed.

According to embodiments of the present disclosure, the controller 1002 may control, according to different operating state instructions, the first dual-axis galvo scanner 100110 and the second dual-axis galvo scanner 100111 to deflect different deflection angles and deflect in different deflection directions, so as to form target ring beams of different sizes.

According to embodiments of the present disclosure, a size of the first dual-axis galvo scanner 100110 may be smaller than that of the second dual-axis galvo scanner 100111. A galvanometer on which the incident beam is projected firstly may generally have a small size, which only needs to be larger than a size of the incident beam. The galvanometer on which the incident beam is projected firstly may be referred to as the first dual-axis galvo scanner. Accordingly, a galvanometer on which the incident beam is projected later may be referred to as the second dual-axis galvo scanner. As the X-axis scanning speed is fast and the galvanometer with small mass has a small inertia, the first dual-axis galvo scanner may be configured for X-axis scanning. As the second dual-axis galvo scanner needs to receive a full range scanned by the first dual-axis galvo scanner, a size of the second dual-axis galvo scanner needs to be larger than that of the first dual-axis galvo scanner, and the second dual-axis galvo scanner may be configured for Y-axis scanning. In embodiments of the present disclosure, the first dual-axis galvo scanner 100110 may be used as the first dual-axis galvo scanner, and the second dual-axis galvo scanner 100111 may be used as the second dual-axis galvo scanner. Based on the above, the first dual-axis galvo scanner 100110 may be configured for X-axis scanning, and the second dual-axis galvo scanner 100111 may be configured for Y-axis scanning.

As shown in FIG. 25, according to embodiments of the present disclosure, the beam adjuster 1001 includes a rotary mirror 10012 and a first voltage focusing lens 10013.

The controller 1002 is used to, for the detected site of the detected object, control the light source 1000, according to a corresponding operating state instruction, to emit the incident beam corresponding to each predetermined wavelength and project each incident beam to the rotary mirror 10012.

The controller 1002 is used to control, according to the corresponding operating state instruction, the rotary mirror 10012 to rotate at different angles, so as to convert each incident beam into a corresponding original ring beam, and project each original ring beam to the first voltage focusing lens 10013.

The controller 1002 is used to control, according to the corresponding operating state instruction, the first voltage focusing lens 10013 to adjust an inner radius or outer radius of each original ring beam to a corresponding source-detection distance so as to obtain each target ring beam, and project each target ring beam to the detected site.

According to embodiments of the present disclosure, the light source 1000, the rotary mirror 10012 and the first voltage focusing lens 10013 may cooperate to form each target ring beam under the control of the controller 1002. As shown in FIG. 25, the light source 1000 may be used to emit each incident beam corresponding to each predetermined wavelength, and each incident beam is converted into a corresponding original ring beam by the rotary mirror 10012 for continuous transmission, that is, the controller 1002 may control the rotary mirror 10012 to achieve a 360° rotational scanning of each incident beam, so as to form the original ring beam. Each original ring beam passes through the first voltage focusing lens 10013 to form a corresponding target ring beam. The size of the original ring beam is adjusted by controlling a focal length of the first voltage focusing lens 10013, so as to form target ring beams with different sizes.

According to embodiments of the present disclosure, each operating state instruction is generated by the controller 1002 according to a first state relationship table, in which a corresponding relationship between each target ring beam corresponding to each predetermined wavelength and an operating voltage of the first voltage focusing lens 10013 for the detected site of the detected object is stored.

According to embodiments of the present disclosure, as the target ring beams corresponding to different predetermined wavelengths have different dispersions, the same original ring beam corresponding to different predetermined wavelengths may form target ring beams with different sizes after passing through the first voltage focusing lens 10013 with the same operating voltage. In order to enable the same original ring beam corresponding to different predetermined wavelengths to form the target ring beams with the same size after passing through the first voltage focusing lens 10013, the operating voltage of the first voltage focusing lens 10013 needs to be adjusted according to the predetermined wavelengths, that is, the operating voltage of the first voltage focusing lens 10013 has a corresponding relationship with each target ring beam corresponding to each predetermined wavelength.

Based on the above, a first state relationship table may be pre-constructed, in which a corresponding relationship between each target ring beam corresponding to each predetermined wavelength and the operating voltage of the first voltage focusing lens 10013 for the detected site of the detected object is stored. The controller 1002 may generate the operating state instruction according to the first state relationship table, and then control the operating states of the rotary mirror 10012, the first voltage focusing lens 10013 and the light source 1000 according to the operating state instruction.

According to embodiments of the present disclosure, the above-mentioned beam adjuster 1001 may include the MEMS scanning mirror 10010. Alternatively, the beam adjuster 1001 may include the galvo scanner assembly 10011. Alternatively, the beam adjuster 1001 may include the rotary mirror 10012 and the first voltage focusing lens 10013. Under the control of the controller 1002, the above-mentioned components may respectively cooperate with the light source 1000 to form the target ring beam by means of point-shaped light spot scanning.

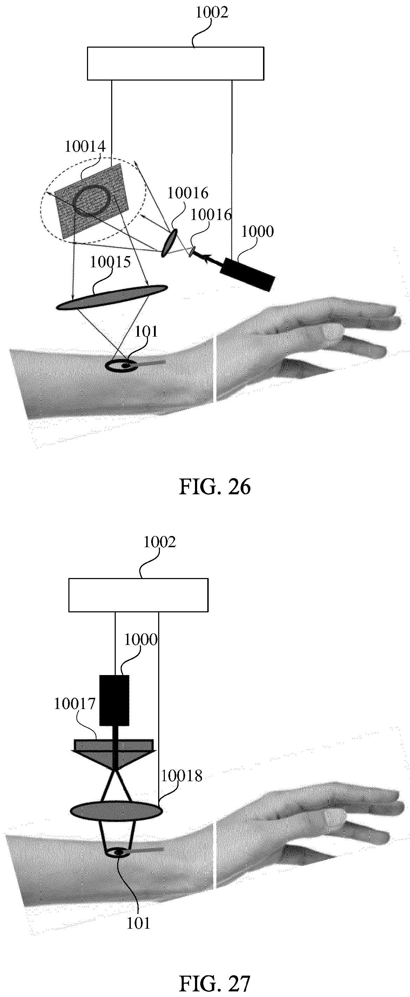

As shown in FIG. 26, according to embodiments of the present disclosure, the beam adjuster 1001 includes a micro-lens array 10014 and an imaging lens 10015.

The controller 1002 is used to, for the detected site of the detected object, control the light source 1000, according to a corresponding operating state instruction, to emit the incident beam corresponding to each predetermined wavelength and project each incident beam to the micro-lens array 10014, and control the micro-lens array 10014, according to the corresponding operating state instruction, to convert each incident beam into a corresponding target ring beam and project each target ring beam to the surface of the detected site through the imaging lens 10015.

According to embodiments of the present disclosure, the light source 1000 and the micro-lens array 10014 may cooperate to form each target ring beam under the control of the controller 1002. That is, the controller 1002 controls, according to the operating state instruction, a micro-lens corresponding to each target ring beam in the micro-lens array 10014 to be in an open state, and the incident beam corresponding to each predetermined wavelength that is emitted by the light source 1000 under the control of the controller 1002 may be reflected by the micro-lens in the open state to form each corresponding target ring beam. Each target ring beam is projected to the detected site through the imaging lens 10015. By controlling the micro-lens in the open state in the micro-lens array 10014, target ring beams of different sizes may be formed.

As shown in FIG. 26, according to embodiments of the present disclosure, the beam adjuster 1001 further includes a beam expanding lens group 10016.

The controller 1002 is used to, for the detected site of the detected object, control the light source 1000, according to a corresponding operating state instruction, to emit the incident beam corresponding to each predetermined wavelength and project each incident beam to the beam expanding lens group 10016.

The beam expanding lens group 10016 is used to expand each incident beam, and project each expanded incident beam to the micro-lens array 10014, so that the projection of each incident beam on the micro-lens array 10014 covers the micro-lens array 10014.

The controller 1002 is used to control, according to the corresponding operating state instruction, the micro-lens array 10014 to convert each incident beam into a corresponding target ring beam, and project each target ring beam to the surface of the detected site through the imaging lens 10015.

According to embodiments of the present disclosure, in order to achieve that the projection of the incident beam on the micro-lens array 10014 covers the micro-lens array 10014, the beam expanding lens group 10016 may be provided to expand the incident beam.

According to embodiments of the present disclosure, each operating state instruction is generated by the controller 1002 according to a second state relationship table, in which a corresponding relationship between each target ring beam corresponding to each predetermined wavelength and the micro-lens in the open state in the micro-lens array 10014 for the detected site of the detected object is stored.

According to embodiments of the present disclosure, as target ring beams corresponding to different predetermined wavelengths have different dispersions, sizes of the target ring beams corresponding to different predetermined wavelengths change from the same to different after these the target ring beams passes through the imaging lens 10015. That is, originally the same size of the target ring beams, due to the imaging lens 10015, the size becomes different on the surface of the detected site.. In order to achieve that different predetermined wavelengths may form the same target ring beam on the surface of the detected site, the micro-lens in the open state in the micro-lens array 10014 needs to be adjusted according to the predetermined wavelengths, that is, the micro-lens in the open state in the micro-lens array 10014 has a corresponding relationship with each target ring beam corresponding to each predetermined wavelength.

Based on the above, the second state relationship table may be pre-constructed, in which the corresponding relationship between each target ring beam corresponding to each predetermined wavelength and the micro-lens in the open state in the micro-lens array 10014 for the detected site of the detected object is stored. The controller 1002 may generate an operating state instruction according to the second state relationship table, and then control the operating states of the micro-lens array 10014 and the light source 1000 according to the operating state instruction.

As shown in FIG. 27, according to embodiments of the present disclosure, the beam adjuster 1001 includes a conical lens 10017 and a second voltage focusing lens 10018.

The controller 1002 is used to, for the detected site of the detected object, control the light source 1000, according to a corresponding operating state instruction, to emit the incident beam corresponding to each predetermined wavelength and project each incident beam to the conical lens 10017.

The conical lens 10017 is used to convert each incident beam into a conical beam, and project each conical beam to the second voltage focusing lens 10018 to be imaged as each original ring beam by the second voltage focusing lens 10018.

The controller 1002 is used to control the second voltage focusing lens 10018, according to the corresponding operating state instruction, to adjust an inner radius or outer radius of each original ring beam to a corresponding source-detection distance so as to obtain each target ring beam, and project each target ring beam to the detected site.

According to embodiments of the present disclosure, the light source 1000 and the second voltage focusing lens 10018 may cooperate to form each target ring beam under the control of the controller 1002. As shown in FIG. 27, the light source 1000 may be used to emit an incident beam for each predetermined wavelength. Each incident beam passes through the conical lens 10017 and is converted into a corresponding conical beam for continuous transmission. Each conical beam is projected on a receiving surface to form a corresponding original ring beam. Each original ring beam passes through the second voltage focusing lens 10018 to form a corresponding target ring beam. As described above, by controlling the focal length of the second voltage focusing lens 10018, target ring beams with different sizes may be formed.

According to embodiments of the present disclosure, a ring width of the target ring beam may be determined by a spot size of the incident beam.

According to embodiments of the present disclosure, each operating state instruction is generated by the controller 1002 according to a third state relationship table, in which a corresponding relationship between each target ring beam corresponding to each predetermined wavelength and the operating voltage of the second voltage focusing lens 10018 for the detected site of the detected object is stored.

According to embodiments of the present disclosure, as the target ring beams corresponding to different predetermined wavelengths have different dispersions, the same original ring beam corresponding to different predetermined wavelengths may form target ring beams with different sizes after passing through the second voltage focusing lens 10018 with the same operating voltage. In order to enable the same original ring beam corresponding to different predetermined wavelengths to form the target ring beams with the same size after passing through the second voltage focusing lens 10018, the operating voltage of the second voltage focusing lens 10018 needs to be adjusted according to the predetermined wavelengths, that is, the operating voltage of the second voltage focusing lens 10018 has a corresponding relationship with each target ring beam corresponding to each predetermined wavelength.

Based on the above, a third state relationship table may be pre-constructed, in which a corresponding relationship between each target ring beam corresponding to each predetermined wavelength and the operating voltage of the second voltage focusing lens 10018 for the detected site of the detected object is stored. The controller 1002 may generate the operating state instruction according to the third state relationship table, and then control the operating states of the second voltage focusing lens 10018 and the light source 1000 according to the operating state instruction.

According to embodiments of the present disclosure, the beam adjuster 1001 may include the micro-lens array 10014 and the imaging lens 10015. Alternatively, the beam adjuster 1001 may include the conical lens 10017 and the second voltage focusing lens 10018. Under the control of the controller 1002, the above-mentioned components cooperate with the light source 1000 respectively to form the target ring beam by beam projection.

As shown in FIG. 3 and FIG. 6 to FIG. 8, according to embodiments of the present disclosure, the photosensitive surface is in contact with the surface of the detected site, or the photosensitive surface is in non-contact with the surface of the detected site.

According to embodiments of the present disclosure, the contact detection may include the following manners. In a first manner, the photosensitive surface is in contact with the surface of the detected site, as shown in FIG. 6. In a second manner, the photosensitive surface is arranged at the first end of the light guide part, and the second end of the light guide part is in contact with the surface of the detected site, as shown in FIG. 7.

The non-contact detection may include two manners. In a first manner, the photosensitive surface is in non-contact with the surface of the detected site, and the photosensitive surface does not receive the first light intensity value through the light guide part, as shown in FIG. 3. In a second manner, the photosensitive surface is arranged at the first end of the light guide part, and the second end of the light guide part is in non-contact with the surface of the detected site, as shown in FIG. 8.

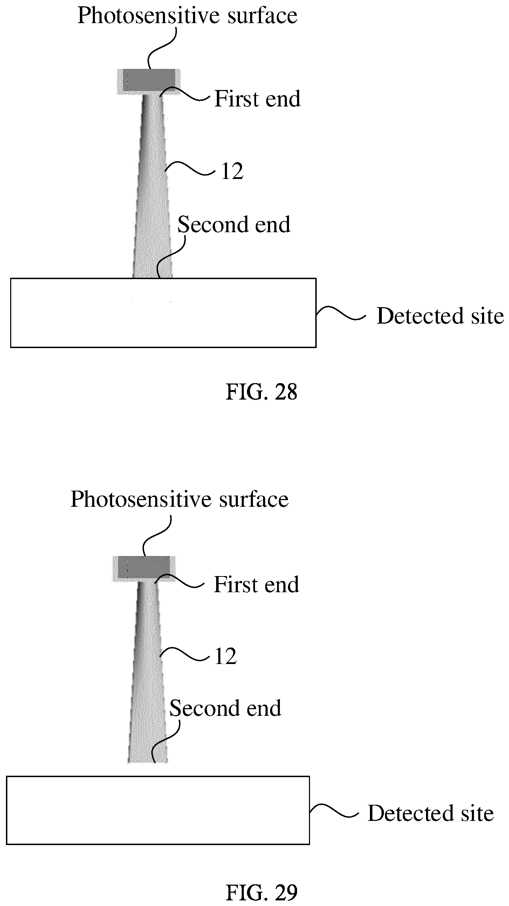

As shown in FIG. 28 and FIG. 29, according to embodiments of the present disclosure, the device 1 of determining the distance in the non-invasive detection of tissue element further includes a light guide part 12. The photosensitive surface is arranged at the first end of the light guide part 12, the second end of the light guide part 12 is in contact or non-contact with the surface of the detected site, and the first end of the light guide part 12 and the second end of the light guide part 12 are opposite end faces.

According to embodiments of the present disclosure, in order to achieve the non-contact between the photosensitive surface and the surface of the detected site, the photosensitive surface may be arranged at the first end of the light guide part 12. As shown in FIG. 28, FIG. 28 shows a schematic diagram of still another photosensitive surface in non-contact with the surface of the detected site according to embodiments of the present disclosure. If the photosensitive surface is arranged at the first end of the light guide part, and the second end of the light guide part is in non-contact with the surface of the detected site, it may be considered that the non-invasive detection of tissue element is the non-contact detection. As shown in FIG. 29, FIG. 29 shows a schematic diagram of still another photosensitive surface in non-contact with the surface of the detected site according to embodiments of the present disclosure. It should be noted that, for the description of arranging the photosensitive surface at the first end of the light guide part 12, reference may be made to the corresponding part above, and details are not repeated here.

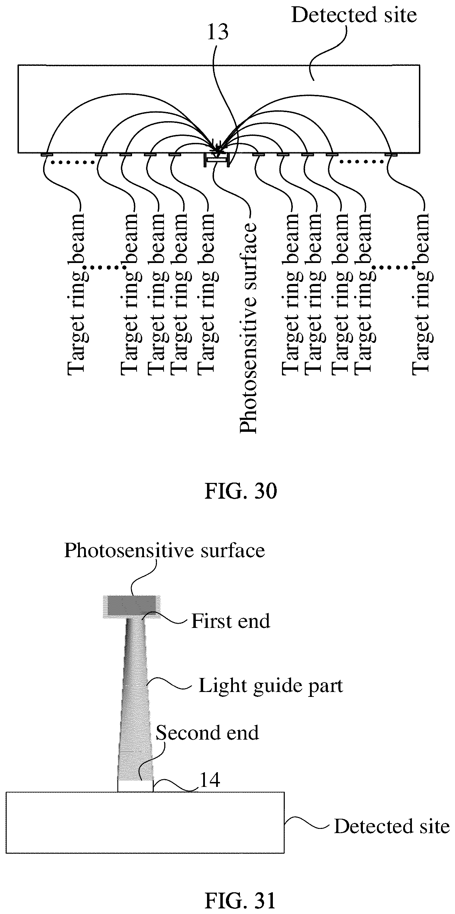

As shown in FIG. 30, according to embodiments of the present disclosure, the photosensitive surface is in non-contact with the surface of the detected site. The device 1 of determining the distance in the non-invasive detection of tissue element further includes a first light blocking part 13. The first light blocking part 13 is arranged in a gap region between the photosensitive surface and the surface of the detected site, and the first light blocking part 13 is in contact with the surface of the detected site. The photosensitive surface is arranged on an inner side of the first light blocking part 13. The first light blocking part 13 is integral with the photosensitive surface or the first light blocking part 13 is separate from the photosensitive surface.

According to embodiments of the present disclosure, if the photosensitive surface is in non-contact with the surface of the detected site, surface-reflected light may be generated. Based on this, in order to further improve the detection accuracy, the interference light needs to be shielded as follows. The device may be further provided with the first light blocking part 13. The first light blocking part 13 is specifically arranged in the gap region between the photosensitive surface and the surface of the detected site, and the first light blocking part 13 is arranged around the photosensitive surface, so that the photosensitive surface is located on an inner side of the first light blocking part 13, while ensuring that the first light blocking part 13 is in contact with the surface of the detected site, as shown in FIG. 30. FIG. 30 shows still another schematic diagram of shielding the interference light according to embodiments of the present disclosure.

According to embodiments of the present disclosure, the first light blocking part 13 may be integral with the photosensitive surface, that is, the first light blocking part 13 may serve as a periphery of the photosensitive surface, which is integral with the photosensitive surface. In addition, the first light blocking part 13 may also be separate from the photosensitive surface. The above may be set according to actual situations, which is not specifically limited here.

According to embodiments of the present disclosure, only the diffusely-scattered light is received by the photosensitive surface. As the diffusely-scattered light carries valid information, the detection accuracy may be further improved.

As shown in FIG. 31, according to embodiments of the present disclosure, the second end of the light guide part 12 is in non-contact with the surface of the detected site. The device 1 of determining the distance in the non-invasive detection of tissue element further includes a second light blocking part 14. The second light blocking part 14 is arranged in a gap region between the light guide part 12 and the surface of the detected site, the first end of the second light blocking part 14 is in contact with the second end of the light guide part 12, the second end of the second light blocking part 14 is in contact with the surface of the detected site, and the second end of the second light blocking part 14 and the first end of the second light blocking part 14 are opposite end faces.

According to embodiments of the present disclosure, if the photosensitive surface is arranged at the first end of the light guide part, and the second end of the light guide part is in non-contact with the surface of the detected site, it may be considered that the non-invasive detection of tissue element is the non-contact detection. Surface-reflected light may be generated in the non-contact detection. Therefore, in order to further improve the detection accuracy, the interference light needs to be shielded as follows. The device 1 of determining the distance in the non-invasive detection of tissue element may be further provided with the second light blocking part 14. The first end of the second light blocking part 14 is in contact with the second end of the light guide part 12, and the second end of the second light blocking part 14 is in contact with the surface of the detected site, so as to ensure that it is difficult for the interference light to enter the light guide part 12 and then be received by the photosensitive surface, as shown in FIG. 31. FIG. 31 shows still another schematic diagram of shielding the interference light according to embodiments of the present disclosure.

According to the embodiments of the present disclosure, only the diffusely-scattered light may be received by the photosensitive surface. As the diffusely-scattered light carries valid information, the detection accuracy may be further improved.

FIG. 20 shows a schematic structural diagram of a device of determining a distance in the non-invasive detection of tissue element according to embodiments of the present disclosure. Such embodiments may be applied to improve the detection accuracy of the concentration of the tissue element to be detected.

As shown in FIG. 20, a device 1 of determining a distance in the non-invasive detection of tissue element includes a second acquisition module 15 and a second determination module 16. A structure and an operating principle will be described below with reference to the accompanying drawings.

The second acquisition module 15 is used to acquire the tissue optical parameter at each predetermined wavelength of at least one predetermined wavelength and the tissue optical parameter change relationship caused by the change in the concentration of the tissue element to be detected for the detected site of the detected object.

The second determination module 16 is used to determine each measurement distance and/or each reference distance according to the tissue optical parameter at each predetermined wavelength and the tissue optical parameter change relationship caused by the change in the concentration of the tissue element to be detected.

According to embodiments of the present disclosure, for the specific processing procedures of the second acquisition module 15 and the second determination module 16, reference may be made to the description of the corresponding part of the method of determining the distance in the non-invasive detection of tissue element described above, and details are not repeated here.

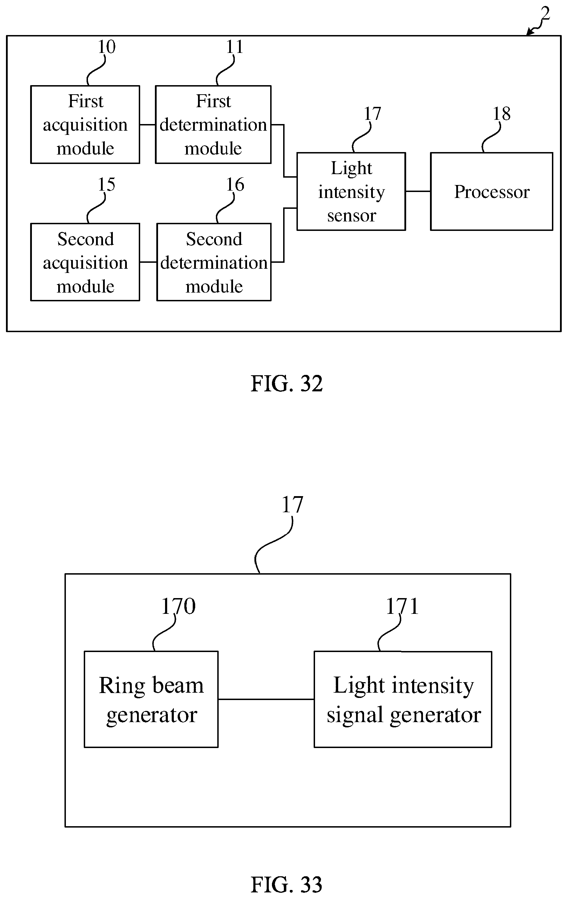

FIG. 32 shows a schematic structural diagram of a non-invasive detection device for tissue element according to embodiments of the present disclosure. Such embodiments may be applied to improve the detection accuracy of the concentration of the tissue element to be detected.

As shown in FIG. 32, a non-invasive detection device 2 for tissue element includes a light intensity sensor 17 and a processor 18. A structure and an operating principle will be described below with reference to the accompanying drawings.

The light intensity sensor 17 is used to acquire the second light intensity measurement value corresponding to each predetermined wavelength at the measurement distance, and/or the second light intensity reference value corresponding to each predetermined wavelength at the reference distance, for the detected site of the detected object. Each measurement distance and each reference distance are determined according to the device of determining the distance in the non-invasive detection of tissue element according to embodiments of the present disclosure, and the predetermined wavelength includes at least one predetermined wavelength.

The processor 18 is used to determine the concentration of the tissue element to be detected according to the second light intensity measurement value and/or the second light intensity reference value corresponding to each predetermined wavelength.

According to embodiments of the present disclosure, for the specific processing procedures of the light intensity sensor 17 and the processor 18, reference may be made to the description of the corresponding part of the non-invasive detection method for tissue element described above, and details are not repeated here.

As shown in FIG. 33, according to embodiments of the present disclosure, the light intensity sensor 17 includes a ring beam generator 170 and a light intensity signal generator 171.

The ring beam generator 170 is used to form a measurement ring beam and/or a reference ring beam corresponding to each predetermined wavelength on the surface of the detected site for the detected site of the detected object. The inner radius or outer radius of each measurement ring beam is the corresponding measurement distance, the inner radius or outer radius of each reference ring beam is the corresponding reference distance, and each measurement ring beam and each reference ring beam have the same geometric center.

The light intensity signal generator 171 is used to acquire, based on the photosensitive surface corresponding to the geometric center, the second light intensity measurement value emitted from the surface of the detected site after each measurement ring beam passes through the detected site, and/or the second light intensity reference value emitted from the surface of the detected site after each reference ring beam passes through the detected site.

According to embodiments of the present disclosure, for the specific processing procedures of the ring beam generator 170 and the light intensity signal generator 171, reference may be made to the description of the corresponding part of the non-invasive detection method for tissue element described above, and details are not repeated here.

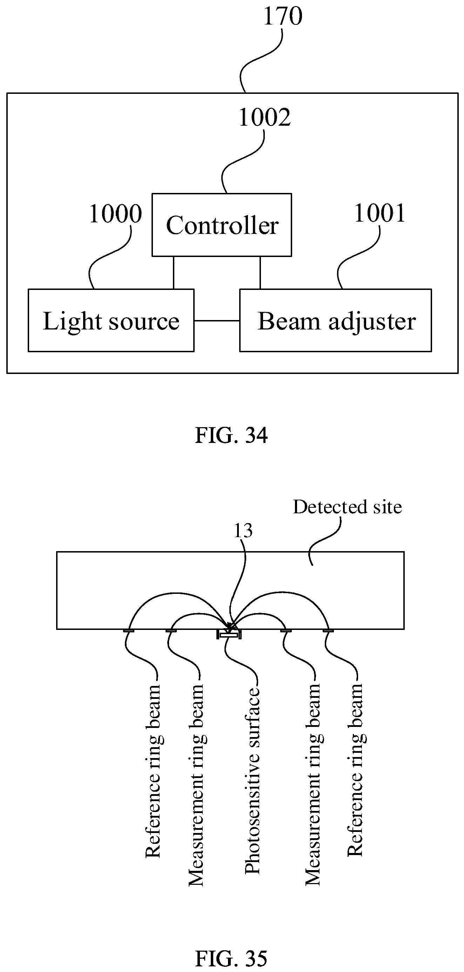

As shown in FIG. 34, according to embodiments of the present disclosure, the ring beam generator 170 includes a light source 1000, a beam adjuster 1001 and a controller 1002. The controller 1002 may be communicatively connected with the light source 1000 and the beam adjuster 1001, respectively.

The controller 1002 is used to, for the detected site of the detected object, control the light source 1000 and the beam adjuster 1001, according to a corresponding operating state instruction, to cooperate to form a measurement ring beam and/or a reference ring beam corresponding to each predetermined wavelength on the surface of the detected site.

According to embodiments of the present disclosure, the operating state instruction may be an instruction for controlling operating states of the light source 1000 and the beam adjuster 1001. The controller 1002 may control, for the detected site of the detected object, the light source 1000 and the beam adjuster 1001 to cooperate to form the measurement ring beam and/or the reference ring beam corresponding to each predetermined wavelength on the surface of the detected site according to the corresponding operating state instruction. It may be understood that the controller 1002 may control the light source 1000 and the beam adjuster 1001, according to the corresponding operating state instruction, to cooperate to form the measurement ring beam and/or the reference ring beam for each predetermined wavelength.

As shown in FIG. 23, according to embodiments of the present disclosure, the beam adjuster 1001 may include a MEMS scanning mirror 10010.

The controller 1002 is used to, for the detected site of the detected object, control the light source 1000, according to a corresponding operating state instruction, to emit an incident beam corresponding to each predetermined wavelength and project each incident beam to the MEMS scanning mirror 10010, and control the MEMS scanning mirror 10010, according to the corresponding operating state instruction, to convert each incident beam into a corresponding measurement ring beam and/or a corresponding reference ring beam and project each measurement ring beam and/or each reference ring beam to the surface of the detected site.

According to embodiments of the present disclosure, the light source 1000 and the MEMS scanning mirror 10010 may cooperate to form each measurement ring beam and/or each reference ring beam under the control of the controller 1002. The MEMS scanning mirror 10010 may be a two-dimensional MEMS scanning mirror. That is, the controller 1002 may synchronously control the light source 1000 and the MEMS scanning mirror 10010 to achieve a two-dimensional scanning image composed of predetermined pixel points by scanning progressively. If a trajectory formed by the predetermined pixel points is a measurement ring, then the two-dimensional scanning image is a measurement ring image. If the trajectory formed by the predetermined pixel points is a reference ring, then the two-dimensional scanning image is a reference ring image. The above-mentioned scanning method enables display time instants and spatial coordinates of the predetermined pixel points in the measurement ring image to be determined, and enables display time instants and spatial coordinates of the predetermined pixel points in the reference ring beam to be determined. The spatial coordinates of the predetermined pixel points in the measurement ring image and the spatial coordinates of the predetermined pixel points in the reference ring image are determined by a deflection angle of the MEMS scanning mirror 10010. The display time instants of the predetermined pixel points in the measurement ring image and the display time instants of the predetermined pixel points in the reference ring image are determined by the light source 1000. That is, the light source 1000 and the MEMS scanning mirror 10010 may be synchronously controlled by the controller 1002, so as to achieve a correspondence between the display time instant and the spatial coordinate of the predetermined pixel point. Different predetermined pixel points may form corresponding measurement ring image and reference ring image. Each measurement ring image and each reference ring image may be projected to the detected site to form each measurement ring beam and each reference ring beam. It should be noted that the spatial coordinate and the display time instant of the predetermined pixel point may be embodied in the operating state instruction.

As shown in FIG. 24, according to embodiments of the present disclosure, the beam adjuster 1001 includes a galvo scanner assembly 10011.

The controller 1002 is used to, for the detected site of the detected object, control the light source 1000, according to a corresponding operating state instruction, to emit an incident beam corresponding to each predetermined wavelength and project each incident beam to the galvo scanner assembly 10011, and control the galvo scanner assembly 10011, according to the corresponding operating state instruction, to convert each incident beam into a corresponding measurement ring beam and/or a corresponding reference ring beam, and project each measurement ring beam and/or each reference ring beam to the surface of the detected site.

According to embodiments of the present disclosure, the light source 1000 and the galvo scanner assembly 10011 may cooperate to form each measurement ring beam and/or each reference ring beam under the control of the controller 1002. That is, the incident beam corresponding to each predetermined wavelength that is emitted by the light source 1000 under the control of the controller 1002 according to the operating state instruction may be projected to the scanning position by the galvo scanner assembly 10011 under the control of the controller 1002 according to the operating state instruction. The incident beam is a point-shaped light spot at the scanning position. Through 360° circular scanning of the point-shaped light spot to form a ring beam with a variable size, the corresponding measurement ring beam and/or corresponding reference ring beam may be formed.

As shown in FIG. 24, according to embodiments of the present disclosure, the galvo scanner assembly 10011 includes a first dual-axis galvo scanner 100110 and a second dual-axis galvo scanner 100111.

The controller 1002 is used to, for the detected site of the detected object, control the light source 1000, according to a corresponding operating state instruction, to emit an incident beam corresponding to each predetermined wavelength and project each incident beam to the first dual-axis galvo scanner 100110.

The controller 1002 is used to control the first dual-axis galvo scanner 100110, according to the corresponding operating state instruction, to deflect a first predetermined angle along an X-axis so that each incident beam is deflected by the first predetermined angle in the X-axis direction, and project the deflected incident beam to the second dual-axis galvo scanner 100111.

The controller 1002 is used to control the second dual-axis galvo scanner 100111, according to the operating state instruction, to deflect a second predetermined angle along a Y-axis to form each measurement ring beam and/or each reference ring beam, and project each measurement ring beam and/or each reference ring beam to the surface of the detected site.

According to embodiments of the present disclosure, the galvo scanner assembly 10011 includes a first dual-axis galvo scanner 100110 and a second dual-axis galvo scanner 100111, and the controller 1002 may control the first dual-axis galvo scanner 100110 and the second dual-axis galvo scanner 100111 to achieve a 360° deflection of the incident beam. The controller 1002 may control the first dual-axis galvo scanner 100110 to deflect the first predetermined angle along the X-axis so that each incident beam is deflected by the first predetermined angle in the X-axis direction along with the first dual-axis galvo scanner 100110, and project the deflected incident beam to the second dual-axis galvo scanner 100111. The controller 1002 may control the second dual-axis galvo scanner 100111 to deflect the second predetermined angle along the Y-axis so that each deflected incident beam is deflected by the second predetermined angle in the Y-axis direction along with the second dual-axis galvo scanner 100111, so as to form each measurement ring beam and/or each reference ring beam. The above-mentioned first predetermined angle and second predetermined angle may be configured to determine that the incident beam is projected to the scanning position, and the incident beam is a point-shaped light spot at the scanning position. The controller 1002 may control the deflection angle and deflection direction of the first dual-axis galvo scanner 100110 and the second dual-axis galvo scanner 100111, so as to achieve the 360° rotation of the point-shaped light spot on the surface of the detected site, that is, to achieve the circular scanning. In addition, the ring beam with variable size is formed by scanning, that is, the corresponding measurement ring beam and/or corresponding reference ring beam may be formed.

According to embodiments of the present disclosure, the controller 1002 may control the first dual-axis galvo scanner 100110 and the second dual-axis galvo scanner 100111, according to different operating state instructions, to deflect different deflection angles and deflect in different deflection directions, so as to form each measurement ring beam and/or each reference ring beam.

According to embodiments of the present disclosure, a size of the first dual-axis galvo scanner 100110 may be smaller than that of the second dual-axis galvo scanner 100111. A galvanometer on which the incident beam is projected firstly may generally have a small size, which only needs to be larger than a size of the incident beam. The galvanometer on which the incident beam is projected firstly may be referred to as the first dual-axis galvo scanner. Accordingly, a galvanometer on which the incident beam is projected later may be referred to as the second dual-axis galvo scanner. As the X-axis scanning speed is fast and the galvanometer with small mass has a small inertia, the first dual-axis galvo scanner may be configured for X-axis scanning. As the second dual-axis galvo scanner needs to receive a full range scanned by the first dual-axis galvo scanner, a size of the second dual-axis galvo scanner needs to be larger than that of the first dual-axis galvo scanner, and the second dual-axis galvo scanner may be configured for Y-axis scanning. In embodiments of the present disclosure, the first dual-axis galvo scanner 100110 may be used as the first dual-axis galvo scanner, and the second dual-axis galvo scanner 100111 may be used as the second dual-axis galvo scanner. Based on the above, the first dual-axis galvo scanner 100110 may be configured for X-axis scanning, and the second dual-axis galvo scanner 100111 may be configured for Y-axis scanning.

As shown in FIG. 25, according to embodiments of the present disclosure, the beam adjuster 1001 includes a rotary mirror 10012 and a first voltage focusing lens 10013.

The controller 1002 is used to, for the detected site of the detected object, control the light source 1000, according to a corresponding operating state instruction, to emit an incident beam corresponding to each predetermined wavelength and project each incident beam to the rotary mirror 10012.

The controller 1002 is used to control, according to the corresponding operating state instruction, the rotary mirror 10012 to rotate at different angles, so as to convert each incident beam into a corresponding original ring beam, and project each original ring beam to the first voltage focusing lens 10013.

The controller 1002 is used to control, according to the corresponding operating state instruction, the first voltage focusing lens 10013 to adjust an inner radius or outer radius of each original ring beam to a corresponding measurement distance so as to obtain each measurement ring beam, and/or adjust an inner radius or outer radius of each original ring beam to a corresponding reference distance so as to obtain each reference ring beam, and project each measurement ring beam and/or each reference ring beam to the detected site.

According to embodiments of the present disclosure, the light source 1000, the rotary mirror 10012 and the first voltage focusing lens 10013 may cooperate to form each measurement ring beam and/or each reference ring beam under the control of the controller 1002. As shown in FIG. 25, the light source 1000 may be used to emit each incident beam corresponding to each predetermined wavelength, and each incident beam is converted into a corresponding original ring beam by the rotary mirror 10012 for continuous transmission, that is, the controller 1002 may control the rotary mirror 10012 to achieve a 360° rotational scanning of each incident beam, so as to form the original ring beam. Each original ring beam passes through the first voltage focusing lens 10013 to form the corresponding measurement ring beam and/or corresponding reference ring beam. The size of the original ring beam is adjusted by controlling a focal length of the first voltage focusing lens 10013, so as to form each measurement ring beam and/or each reference ring beam.

According to embodiments of the present disclosure, each operating state instruction is generated by the controller 1000 according to a first relationship table, in which a corresponding relationship between each measurement ring beam corresponding to each predetermined wavelength and the operating voltage of the first voltage focusing lens 10013 and/or a corresponding relationship between each reference ring beam corresponding to each predetermined wavelength and the operating voltage of the first voltage focusing lens 10013 for the detected site of the detected object are/is stored.

According to embodiments of the present disclosure, a first relationship table may be pre-constructed, in which a corresponding relationship between each measurement ring beam corresponding to each predetermined wavelength and the operating voltage of the first voltage focusing lens 10013 and/or a corresponding relationship between each reference ring beam corresponding to each predetermined wavelength and the operating voltage of the first voltage focusing lens 10013 for the detected site of the detected object are/is stored. The controller 1002 may generate the operating state instruction according to the first relationship table, and then control the operating states of the rotary mirror 10012, the first voltage focusing lens 10013 and the light source 1000 according to the operating state instruction.

According to embodiments of the present disclosure, the above-mentioned beam adjuster 1001 may include the MEMS scanning mirror 10010. Alternatively, the beam adjuster 1001 may include the galvo scanner assembly 10011. Alternatively, the beam adjuster 1001 may include the rotary mirror 10012 and the first voltage focusing lens 10013. Under the control of the controller 1002, the above-mentioned components may respectively cooperate with the light source 1000 to form the measurement ring beam and/or reference ring beam by means of point-shaped light spot scanning.

As shown in FIG. 26, according to embodiments of the present disclosure, the beam adjuster 1001 includes a micro-lens array 10014 and an imaging lens 10015.

The controller 1002 is used to, for the detected site of the detected object, control the light source 1000, according to a corresponding operating state instruction, to emit the incident beam corresponding to each predetermined wavelength and project each incident beam to the micro-lens array 10014, and control the micro-lens array 10014, according to the corresponding operating state instruction, to convert each incident beam into a corresponding measurement ring beam and/or a corresponding reference ring beam, and project each measurement ring beam and/or each reference ring beam to the surface of the detected site through the imaging lens 10015.

According to embodiments of the present disclosure, the light source 1000 and the micro-lens array 10014 may cooperate to form each measurement ring beam and/or each reference ring beam under the control of the controller 1002. That is, the controller 1002 controls, according to the operating state instruction, a micro-lens corresponding to each measurement ring beam in the micro-lens array 10014 and/or a micro-lens corresponding to each reference ring beam in the micro-lens array 10014 to be in an open state, and the incident beam corresponding to each predetermined wavelength that is emitted by the light source 1000 under the control of the controller 1002 may be reflected by the micro-lens in the open state to form each corresponding measurement ring beam and/or each corresponding reference ring beam. Each measurement ring beam and/or each reference ring beam may be projected to the detected site through the imaging lens 10015. By controlling the micro-lens in the open state in the micro-lens array 10014, each measurement ring beam and/or each reference ring beam may be formed.

As shown in FIG. 26, according to embodiments of the present disclosure, the beam adjuster 1001 further includes a beam expanding lens group 10016.

The controller 1002 is used to, for the detected site of the detected object, control the light source 1000, according to a corresponding operating state instruction, to emit an incident beam corresponding to each predetermined wavelength and project each incident beam to the beam expanding lens group 10016.

The beam expanding lens group 10016 is used to expand each incident beam, and project each expanded incident beam to the micro-lens array 10014, so that the projection of each incident beam on the micro-lens array 10014 covers the micro-lens array 10014.

The controller 1002 is used to control, according to the corresponding operating state instruction, the micro-lens array 10014 to convert each incident beam into a corresponding measurement ring beam and/or a corresponding reference ring beam, and project each measurement ring beam and/or each reference ring beam to the surface of the detected site through the imaging lens 10015.

According to embodiments of the present disclosure, in order to achieve that the projection of the incident beam on the micro-lens array 10014 covers the micro-lens array 10014, the beam expanding lens group 10016 may be provided to expand the incident beam.

According to embodiments of the present disclosure, each operating state instruction is generated by the controller 1002 according to a second relationship table, in which a corresponding relationship between each measurement ring beam corresponding to each predetermined wavelength and the micro-lens in the open state in the micro-lens array 10014 and/or a corresponding relationship between each reference ring beam corresponding to each predetermined wavelength and the micro-lens in the open state in the micro-lens array 10014 for the detected site of the detected object are/is stored.

According to embodiments of the present disclosure, the second relationship table may be pre-constructed, in which the corresponding relationship between each measurement ring beam corresponding to each predetermined wavelength and the micro-lens in the open state in the micro-lens array 10014 and/or the corresponding relationship between each reference ring beam corresponding to each predetermined wavelength and the micro-lens in the open state in the micro-lens array 10014 for the detected site of the detected object are/is stored. The controller 1002 may generate an operating state instruction according to the second relationship table, and then control the operating states of the micro-lens array 10014 and the light source 1000 according to the operating state instruction.

As shown in FIG. 27, according to embodiments of the present disclosure, the beam adjuster 1001 includes a conical lens 10017 and a second voltage focusing lens 10018.

The controller 1002 is used to, for the detected site of the detected object, control the light source 1000, according to a corresponding operating state instruction, to emit an incident beam corresponding to each predetermined wavelength and project each incident beam to the conical lens 10017.

The conical lens 10017 is used to convert each incident beam into a conical beam, and project each conical beam to the second voltage focusing lens 10018to be imaged as each original ring beam by the second voltage focusing lens 10018.

The controller 1002 is used to control the second voltage focusing lens 10018, according to a corresponding operating state instruction, to adjust an inner radius or outer radius of each original ring beam to a corresponding measurement distance so as to obtain each measurement ring beam, and/or adjust an inner radius or outer radius of each original ring beam to a corresponding reference distance so as to obtain each reference ring beam, and project each measurement ring beam and/or each reference ring beam to the detected site.

According to embodiments of the present disclosure, the light source 1000 and the second voltage focusing lens 10018 may cooperate to form each measurement ring beam and/or each reference ring beam under the control of the controller 1002. As shown in FIG. 27, the light source 1000 may be used to emit an incident beam for each predetermined wavelength. Each incident beam passes through the conical lens 10017 and is converted into a corresponding conical beam for continuous transmission. Each conical beam is projected on a receiving surface to form a corresponding original ring beam. Each original ring beam passes through the second voltage focusing lens 10018 to form a corresponding measurement ring beam and/or a corresponding reference ring beam. As described above, by controlling the focal length of the second voltage focusing lens 10018, each measurement ring beam and/or each reference ring beam may be formed.

According to embodiments of the present disclosure, each operating state instruction is generated by the controller 1002 according to a third relationship table, in which a corresponding relationship between each measurement ring beam corresponding to each predetermined wavelength and the operating voltage of the second voltage focusing lens 10018 and/or a corresponding relationship between each reference ring beam corresponding to each predetermined wavelength and the operating voltage of the second voltage focusing lens 10018 for the detected site of the detected object are/is stored.

According to embodiments of the present disclosure, a third relationship table may be pre-constructed, in which a corresponding relationship between each measurement ring beam corresponding to each predetermined wavelength and the operating voltage of the second voltage focusing lens 10018 and/or a corresponding relationship between each reference ring beam corresponding to each predetermined wavelength and the operating voltage of the second voltage focusing lens 10018 for the detected site of the detected object are/is stored. The controller 1002 may generate the operating state instruction according to the third relationship table, and then control the operating states of the second voltage focusing lens 10018 and the light source 1000 according to the operating state instruction.

According to embodiments of the present disclosure, the processor 18 is configured to perform, for each predetermined wavelength, a difference operation between the second light intensity measurement value and the second light intensity reference value at the predetermined wavelength, so as to obtain a light intensity difference value; and determine the concentration of the tissue element to be detected according to the light intensity difference value at each predetermined wavelength.

According to embodiments of the present disclosure, for the specific processing procedures of the difference sub-module and the determination sub-module, reference may be made to the description of the corresponding part of the non-invasive detection method for tissue element described above, which will not be described in detail here.

As shown in FIG. 7, FIG. 8, FIG. 15 and FIG. 16, according to embodiments of the present disclosure, the photosensitive surface is in contact with the surface of the detected site, or the photosensitive surface is in non-contact with the surface of the detected site.

According to embodiments of the present disclosure, the contact detection may include two manners. In a first manner, the photosensitive surface is in contact with the surface of the detected site, as shown in FIG. 16. In a second manner, the photosensitive surface is arranged at the first end of the light guide part, and the second end of the light guide part is in contact with the surface of the detected site, as shown in FIG. 7. The non-contact detection may include two manners. In a first manner, the photosensitive surface is in non-contact with the surface of the detected site, and the photosensitive surface does not receive the first light intensity value through the light guide part, as shown in FIG. 15. In a second manner, the photosensitive surface is arranged at the first end of the light guide part, and the second end of the light guide part is in non-contact with the surface of the detected site, as shown in FIG. 8.

As shown in FIG. 28 and FIG. 29, according to embodiments of the present disclosure, the non-invasive detection device 2 for tissue element further includes a light guide part 12. The photosensitive surface is arranged at the first end of the light guide part 12, the first end of the light guide part 12 is in non-contact with the surface of the detected site, the second end of the light guide part 12 is in contact or non-contact with the surface of the detected site, and the first end of the light guide part 12 and the second end of the light guide part 12 are opposite end faces.

According to embodiments of the present disclosure, it should be noted that, for the description of arranging the photosensitive surface at the first end of the light guide part 12, reference may be made to the corresponding part above, and details are not repeated here.

As shown in FIG. 35, according to embodiments of the present disclosure, the photosensitive surface is in non-contact with the surface of the detected site. The non-invasive detection device 2 for tissue element further includes a first light blocking part 13. The first light blocking part 13 is arranged in a gap region between the photosensitive surface and the surface of the detected site, and the first light blocking part 13 is in contact with the surface of the detected site. The photosensitive surface is arranged on an inner side of the first light blocking part 13. The first light blocking part 13 is integral with the photosensitive surface or the first light blocking part 13 is separate from the photosensitive surface.

According to embodiments of the present disclosure, for the specific description of the first light blocking part 13, reference may be made to the corresponding part above, and details are not repeated here.

As shown in FIG. 31, according to embodiments of the present disclosure, the second end of the light guide part 12 is in non-contact with the surface of the detected site. The non-invasive detection device 2 for tissue element further includes a second light blocking part 14. The second light blocking part 14 is arranged in the gap region between the light guide part 12 and the surface of the detected site, the first end of the second light blocking part 14 is in contact with the second end of the light guide part 12, the second end of the second light blocking part 14 is in contact with the surface of the detected site, and the second end of the second light blocking part 14 and the first end of the second light blocking part 14 are opposite end faces.

According to embodiments of the present disclosure, for the description of the second light blocking part 14, reference may be made to the corresponding part above, and details are not repeated here.

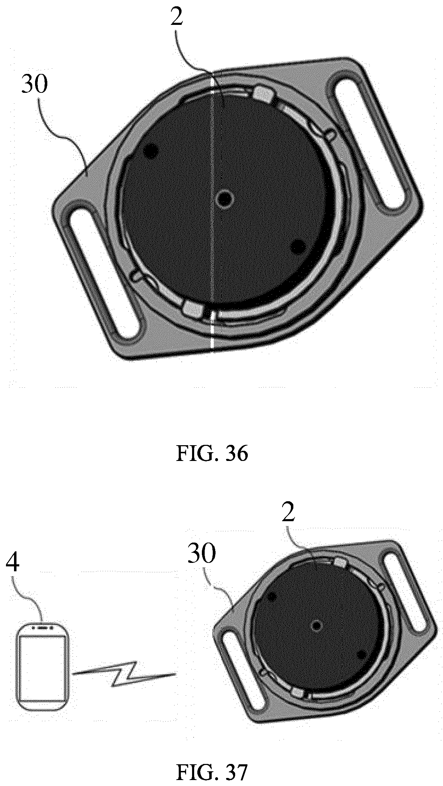

FIG. 36 shows a schematic structural diagram of a wearable apparatus according to embodiments of the present disclosure. Such embodiments may be applied to improve the detection accuracy of the concentration of the tissue element to be detected.

As shown in FIG. 36, the wearable apparatus 3 includes a body 30 and the non-invasive detection device 2 for tissue element according to embodiments of the present disclosure. The non-invasive detection device 2 for tissue element is arranged on the body 30, and the non-invasive detection device 2 for tissue element includes a light intensity sensor 17 and a processor 18. A structure and an operating principle will be described below with reference to the accompanying drawings.

The wearable apparatus 3 is worn on the detected site.

The light intensity sensor 17 is used to, for the detected site of the detected object, acquire the second light intensity measurement value corresponding to each predetermined wavelength of at least one predetermined wavelength at the measurement distance, and/or the second light intensity reference value corresponding to each predetermined wavelength at the reference distance. Each measurement distance and each reference distance are determined according to the devices described in embodiments of the present disclosure.

The processor 18 is used to determine the concentration of the tissue element to be detected according to the second light intensity measurement value and/or the second light intensity reference value corresponding to each predetermined wavelength.

According to embodiments of the present disclosure, the non-invasive detection device 2 for tissue element may be arranged on the body 30. When the non-invasive detection device 2 for tissue element needs to be used for detection of tissue element, the wearable apparatus 3 may be worn on the detected site. In addition, the detection of the non-invasive detection device 2 for tissue element is easily affected by a detection condition and thus the detection accuracy may be affected. In order to ensure a stability of the detection condition and further improve the detection accuracy, the non-invasive detection device 2 for tissue element may be fixed, so that a positional relationship between the detected site and the non-invasive detection device 2 for tissue element is a predetermined relationship. As described above, the position of the non-invasive detection device 1 for tissue element may be fixed on the body 30, and the stability of the detection condition may be ensured, so that the detection accuracy may be improved. In addition, for the structure and operating principle of the non-invasive detection device 2 for tissue element, reference may be made to the above description for the non-invasive detection device 2, and details are not repeated here.

According to embodiments of the present disclosure, the wearable apparatus 3 may further include a display module communicatively connected with the processor 18. The processor 18 may transmit the concentration of the tissue element to be detected to the display module, and the display module may display the concentration of the tissue element to be detected, so that the concentration of the tissue element to be detected may be obtained by the detected object through the display module. In addition, the wearable apparatus 3 may further include a voice module communicatively connected with the processor 18. The processor 18 may transmit the concentration of the tissue element to be detected to the voice module, and the voice module may generate a voice command according to the concentration of the tissue element to be detected and play the voice command, so that the concentration of the tissue element to be detected may be obtained by the detected object.

In the technical solution of this embodiment, due to a substantial reduction in the size of the detection device, the detection device may be installed on a wearable apparatus, and then easily worn and fixed on the detected site, so that the stability of the detection condition may be ensured, the stability of the detection condition may be improved, and a portable detection may be achieved. On this basis, since the measurement distance and/or the reference distance corresponding to each predetermined wavelength may be accurately determined for the detected site of the detected object, the second light intensity measurement value and/or the second light intensity reference value may be accurately determined according to the accurately determined measurement distance and/or reference distance. The concentration of the tissue element to be detected may be determined according to the accurately determined second light intensity measurement value and/or second light intensity reference value, so that the detection accuracy may be improved.

FIG. 37 shows a schematic structural diagram of a non-invasive detection system for tissue element according to embodiments of the present disclosure. Such embodiments may be applied to improve the detection accuracy of the concentration of the tissue element to be detected.

As shown in FIG. 37, the non-invasive detection system for tissue element includes the wearable apparatus 3 described in embodiments of the present disclosure and a terminal 4. The wearable apparatus 3 includes the body 30 and the non-invasive detection device 2 for tissue element, and the non-invasive detection device 2 for tissue element is arranged on the body 30. The non-invasive detection device 2 for tissue element includes a light intensity sensor 17 and a processor 18. The processor 18 may be communicatively connected with the light intensity sensor 17 and the terminal 4, respectively. A structure and operating principle thereof will be described below with reference to the accompanying drawings.

The wearable apparatus 3 is worn on the detected site.

The light intensity sensor 17 is used to, for the detected site of the detected object, acquire the second light intensity measurement value corresponding to each predetermined wavelength of at least one predetermined wavelength at the measurement distance, and/or the second light intensity reference value corresponding to each predetermined wavelength at the reference distance. Each measurement distance and each reference distance are determined according to the device described in embodiments of the present disclosure.

The processor 18 is used to process each second light intensity measurement value and/or each second light intensity reference value for each predetermined wavelength, so as to obtain each processed second light intensity measurement value and/or each processed second light intensity reference value for each predetermined wavelength, and transmit each processed second light intensity measurement value and/or each processed second light intensity reference value for each predetermined wavelength to the terminal 4.

The terminal 4 is used to determine the concentration of the tissue element to be detected according to each processed second light intensity measurement value and/or each processed second light intensity reference value for each predetermined wavelength.

According to embodiments of the present disclosure, different from preceding embodiments, the wearable apparatus 3 and the terminal 4 may cooperate to determine the concentration of the tissue element to be detected in order to reduce the cost of the non-invasive detection device 2 for tissue element. That is, the processor 18 may process each second light intensity measurement value and/or each second light intensity reference value for each predetermined wavelength to obtain each processed second light intensity measurement value and/or each processed second light intensity reference value for each predetermined wavelength, and transmit each processed second light intensity measurement value and/or each processed second light intensity reference value for each predetermined wavelength to the terminal 4. The terminal 4 may determine the concentration of the tissue element to be detected according to each processed second light intensity measurement value and/or each processed second light intensity reference value for each predetermined wavelength. The processing operation of each second light intensity measurement value and/or each second light intensity reference value by the processor 18 may include current-to-voltage conversion, amplification, and analog-to-digital conversion, etc. The terminal 4 may determine the concentration of the tissue element to be detected according to each processed second light intensity measurement value and/or each processed second light intensity reference value by using the same methods as the non-invasive detection methods for tissue element described in embodiments of the present disclosure, which will not be described in detail here. In addition, a structure and an operating principle of the wearable apparatus 3 may be referred to the description of the wearable apparatus 3 above, and details are not repeated here.

According to embodiments of the present disclosure, the terminal 4 may further display the concentration of the tissue element to be detected, so that the concentration of the tissue element to be detected may be obtained by the detected object. The terminal 4 may further generate a voice command containing the concentration of the tissue element to be detected, and play the voice command, so that the concentration of the tissue element to be detected may be obtained by the detected object.

According to embodiments of the present disclosure, in addition to determining the concentration of the tissue element to be detected by the cooperation of the terminal 4 and the wearable apparatus 3, the concentration of the tissue element to be detected may also be determined by a cooperation of a cloud server and the wearable apparatus 3.

The invention is as defined in the appended claims.

## Claims

1. A non-invasive detection method for a tissue element, comprising:
acquiring (S410), for a detected site of a detected object, a second light intensity measurement value for each predetermined wavelength of at least one predetermined wavelength at a measurement distance, and/or a second light intensity reference value for each predetermined wavelength of at least one predetermined wavelength at a reference distance, wherein the measurement distance is a source-detection distance corresponding to a first light intensity measurement value, and the reference distance is a source-detection distance corresponding to a first light intensity reference value; and
determining (S420) a concentration of a tissue element to be detected according to the second light intensity measurement value for each predetermined wavelength and/or the second light intensity reference value for each predetermined wavelength,
wherein the acquiring, for a detected site of a detected object, a second light intensity measurement value for each predetermined wavelength at a measurement distance, and/or a second light intensity reference value for each predetermined wavelength at a reference distance comprises:
forming (S540), for the detected site of the detected object, a measurement ring beam and/or a reference ring beam corresponding to each predetermined wavelength on a surface of the detected site, wherein an inner radius or outer radius of each measurement ring beam is a corresponding measurement distance, an inner radius or outer radius of each reference ring beam is a corresponding reference distance, and each measurement ring beam and each reference ring beam have a same geometric center; and
acquiring (S550), based on a photosensitive surface corresponding to the geometric center, the second light intensity measurement value emitted from the surface of the detected site after each measurement ring beam passes through the detected site, and/or the second light intensity reference value emitted from the surface of the detected site after each reference ring beam passes through the detected site.

2. The method according to claim 1, wherein the first light intensity measurement value is a first light intensity value corresponding to a greatest absolute value of a light intensity variation caused by a change in the concentration of the tissue element to be detected, the first light intensity reference value is a first light intensity value corresponding to a smallest absolute value of a light intensity variation caused by a change in the concentration of the tissue element to be detected, and the light intensity variation caused by the change in the concentration of the tissue element to be detected is a variation between the first light intensity value and a corresponding predetermined light intensity value.

3. The method according to claim 1 or 2, wherein each measurement ring beam is formed by a point-shaped light spot scanning or formed by a beam projection, and each reference ring beam is formed by the point-shaped light spot scanning or formed by the beam projection.

4. The method according to any one of claims 1 to 3, wherein the determining a concentration of a tissue element to be detected according to the second light intensity measurement value for each predetermined wavelength and/or the second light intensity reference value for each predetermined wavelength comprises:
performing (S560), for each predetermined wavelength, a difference operation between the second light intensity measurement value for the predetermined wavelength and the second light intensity reference value for the predetermined wavelength, so as to obtain a light intensity difference value; and
determining (S570) the concentration of the tissue element to be detected according to the light intensity difference value for each predetermined wavelength.

5. The method according to claim 1 or 2, wherein a non-contact between the photosensitive surface and the surface of the detected site is formed by:
providing the photosensitive surface at a first end of a light guide part (12), a second end of the light guide part (12) being in contact or non-contact with the surface of the detected site, wherein the second end of the light guide part (12) and the first end of the light guide part (12) are opposite end faces.

6. A non-invasive detection device (2) for a tissue element, comprising:
a light intensity sensor (17) configured to acquire, for a detected site of a detected object, a second light intensity measurement value for each predetermined wavelength of at least one predetermined wavelength at a measurement distance, and/or a second light intensity reference value for each predetermined wavelength of at least one predetermined wavelength at a reference distance, wherein the measurement distance is a source-detection distance corresponding to a first light intensity measurement value, and the reference distance is a source-detection distance corresponding to a first light intensity reference value; and
a processor (18) configured to determine a concentration of a tissue element to be detected according to the second light intensity measurement value for each predetermined wavelength and/or the second light intensity reference value for each predetermined wavelength,
wherein the light intensity sensor (17) comprises:
a ring beam generator (170) configured to form, for the detected site of the detected object, a measurement ring beam and/or a reference ring beam corresponding to each predetermined wavelength on a surface of the detected site, wherein an inner radius or outer radius of each measurement ring beam is a corresponding measurement distance, an inner radius or outer radius of each reference ring beam is a corresponding reference distance, and each measurement ring beam and each reference ring beam have a same geometric center; and
a light intensity signal generator (171) configured to acquire, based on a photosensitive surface corresponding to the geometric center, the second light intensity measurement value emitted from the surface of the detected site after each measurement ring beam passes through the detected site, and/or the second light intensity reference value emitted from the surface of the detected site after each reference ring beam passes through the detected site.

7. The device according to claim 6, wherein the first light intensity measurement value is a first light intensity value corresponding to a greatest absolute value of a light intensity variation caused by a change in the concentration of the tissue element to be detected, the first light intensity reference value is a first light intensity value corresponding to a smallest absolute value of a light intensity variation caused by a change in the concentration of the tissue element to be detected, and the light intensity variation caused by the change in the concentration of the tissue element to be detected is a variation between the first light intensity value and a corresponding predetermined light intensity value.

8. The device according to claim 6 or 7, wherein the ring beam generator (170) comprises a light source (1000), a beam adjuster (1001) and a controller (1002), and
the controller (1002) is communicatively connected with the light source (1000) and the beam adjuster (1001) respectively, and
the controller (1002) is configured to, for the detected site of the detected object, control the light source (1000) and the beam adjuster (1001) to cooperate to form a measurement ring beam and/or a reference ring beam corresponding to each predetermined wavelength on the surface of the detected site, according to a corresponding operating state instruction.

9. The device according to any one of claims 6 to 8, wherein the beam adjuster (1001) comprises a MEMS scanning mirror (10010), and
wherein the controller (1002) is configured to, for the detected site of the detected object, control the light source (1000), according to a corresponding operating state instruction, to emit an incident beam corresponding to each predetermined wavelength and project each incident beam to the MEMS scanning mirror (10010), and control the MEMS scanning mirror (10010), according to the corresponding operating state instruction, to convert each incident beam into a corresponding measurement ring beam and/or a corresponding reference ring beam and project each measurement ring beam and/or each reference ring beam to the surface of the detected site.

10. The device according to any one of claims 6 to 8, wherein the beam adjuster (1001) comprises a galvo scanner assembly (10011), and
wherein the controller (1002) is configured to, for the detected site of the detected object, control the light source (1000), according to a corresponding operating state instruction, to emit an incident beam corresponding to each predetermined wavelength and project each incident beam to the galvo scanner assembly (10011), and control the galvo scanner assembly (10011), according to the corresponding operating state instruction, to convert each incident beam into a corresponding measurement ring beam and/or a corresponding reference ring beam and project each measurement ring beam and/or each reference ring beam to the surface of the detected site.

11. The device according to claim 10, wherein the galvo scanner assembly (10011) comprises a first dual-axis galvo scanner (100110) and a second dual-axis galvo scanner (100111), and
wherein the controller (1002) is configured to, for the detected site of the detected object, control the light source (1000), according to a corresponding operating state instruction, to emit an incident beam corresponding to each predetermined wavelength and project each incident beam to the first dual-axis galvo scanner (100110);
the controller (1002) is configured to control the first dual-axis galvo scanner (100110), according to the corresponding operating state instruction, to deflect a first predetermined angle along an X-axis, so that each incident beam is deflected by the first predetermined angle in an X-axis direction, and project each deflected incident beam to the second dual-axis galvo scanner (100111); and
the controller (1002) is configured to control the second dual-axis galvo scanner (100111), according to the operating state instruction, to deflect a second predetermined angle in a Y-axis direction, so that each deflected incident beam is deflected by the second predetermined angle in an Y-axis direction, so as to form each measurement ring beam and/or each reference ring beam, and project each measurement ring beam and/or each reference ring beam to the surface of the detected site.

12. The device according to any one of claims 6 to 8, wherein the beam adjuster (1001) comprises a rotary mirror (10012) and a first voltage focusing lens (10013), and
wherein the controller (1002) is configured to, for the detected site of the detected object, control the light source (1000), according to a corresponding operating state instruction, to emit an incident beam corresponding to each predetermined wavelength and project each incident beam to the rotary mirror (10012);
the controller (1002) is configured to control the rotary mirror (10012), according to the corresponding operating state instruction, to rotate at different angles to convert each incident beam into a corresponding original ring beam, and project each original ring beam to the first voltage focusing lens (10013); and
the controller (1002) is configured to control the first voltage focusing lens (10013), according to the corresponding operating state instruction, to adjust an inner radius or outer radius of each original ring beam to a corresponding measurement distance to obtain each measurement ring beam, and/or adjust an inner radius or outer radius of each original ring beam to a corresponding reference distance to obtain each reference ring beam, and project each measurement ring beam and/or each reference ring beam to the detected site.

13. The device according to claim 12, wherein each operating state instruction is generated by the controller (1002) according to a first relationship table in which a corresponding relationship between each measurement ring beam corresponding to each predetermined wavelength and an operating voltage of the first voltage focusing lens (10013) and/or a corresponding relationship between each reference ring beam corresponding to each predetermined wavelength and an operating voltage of the first voltage focusing lens (10013) for the detected site of the detected object are/is stored.

14. The device according to any one of claims 6 to 8, wherein the beam adjuster (1001) comprises a micro-lens array (10014) and an imaging lens (10015), and
wherein the controller (1002) is configured to, for the detected site of the detected object, control the light source (1000), according to a corresponding operating state instruction, to emit an incident beam corresponding to each predetermined wavelength and project each incident beam to the micro-lens array (10014), and control the micro-lens array (10014), according to the corresponding operating state instruction, to convert each incident beam into a corresponding measurement ring beam and/or a corresponding reference ring beam, and project each measurement ring beam and/or each reference ring beam to the surface of the detected site through the imaging lens (10015).

15. The device according to claim 14, wherein the beam adjuster (1001) further comprises a beam expanding lens group (10016), and
wherein the controller (1002) is configured to, for the detected site of the detected object, control the light source (1000), according to a corresponding operating state instruction, to emit the incident beam corresponding to each predetermined wavelength and project each incident beam to the beam expanding lens group (10016);
the beam expanding lens group (10016) is configured to expand each incident beam, and project each expanded incident beam to the micro-lens array (10014), so that a projection of each incident beam on the micro-lens array (10014) covers the micro-lens array (10014); and
the controller (1002) is configured to control the micro-lens array (10014), according to the corresponding operating state instruction, to convert each incident beam into a corresponding measurement ring beam and/or a corresponding reference ring beam, and project each measurement ring beam and/or each reference ring beam to the surface of the detected site through the imaging lens (10015).

16. The device according to claim 15, wherein each operating state instruction is generated by the controller (1002) according to a second relationship table in which a corresponding relationship between each measurement ring beam corresponding to each predetermined wavelength and a micro-lens in an open state in the micro-lens array (10014) and/or a corresponding relationship between each reference ring beam corresponding to each predetermined wavelength and a micro-lens in an open state in the micro-lens array (10014) for the detected site of the detected object are/is stored.

17. The device according to any one of claims 6 to 8, wherein the beam adjuster (1001) comprises a conical lens (10017) and a second voltage focusing lens (10018), and
wherein the controller (1002) is configured to, for the detected site of the detected object, control the light source (1000), according to a corresponding operating state instruction, to emit an incident beam corresponding to each predetermined wavelength and project each incident beam to the conical lens (10017);
the conical lens (10017) is configured to convert each incident beam into a conical beam, and project each conical beam to the second voltage focusing lens (10018) to be imaged as each original ring beam by the second voltage focusing lens (10018); and
the controller (1002) is configured to control the second voltage focusing lens (10018), according to the corresponding operating state instruction, to adjust an inner radius or outer radius of each original ring beam to a corresponding measurement distance to obtain each measurement ring beam, and/or adjust an inner radius or outer radius of each original ring beam to a corresponding reference distance to obtain each reference ring beam, and project each measurement ring beam and/or each reference ring beam to the detected site.

18. The device according to claim 17, wherein each operating state instruction is generated by the controller (1002) according to a third relationship table in which a corresponding relationship between each measurement ring beam corresponding to each predetermined wavelength and an operating voltage of the second voltage focusing lens (10018) and/or a corresponding relationship between each reference ring beam corresponding to each predetermined wavelength and an operating voltage of the second voltage focusing lens (10018) for the detected site of the detected object are/is stored.

19. The device according to any one of claims 6 to 18, wherein the processor (18) is configured to:
perform, for each predetermined wavelength, a difference operation on the second light intensity measurement value and the second light intensity reference value for the predetermined wavelength, so as to obtain a light intensity difference value; and
determine the concentration of the tissue element to be detected according to the light intensity difference value for each predetermined wavelength.

20. A wearable apparatus (3), comprising a body (30) and the non-invasive detection device (2) for the tissue element according to any one of claims 6 to 19,
wherein the non-invasive detection device (2) for the tissue element is arranged on the body (30), and the wearable apparatus (3) is worn on the detected site.

## Patentansprüche

1. Verfahren zur nicht-invasiven Detektion für ein Gewebeelement, umfassend:
Erfassen (S410), für eine detektierte Stelle eines detektierten Objekts, eines zweiten Lichtintensitätsmesswertes für jede zuvor festgelegte Wellenlänge von mindestens einer zuvor festgelegten Wellenlänge in einer Messdistanz und/oder eines zweiten Lichtintensitätsreferenzwertes für jede zuvor festgelegte Wellenlänge von mindestens einer zuvor festgelegten Wellenlänge in einer Referenzdistanz, wobei die Messdistanz eine Quellendetektionsdistanz ist, die einem ersten Lichtintensitätsmesswert entspricht, und die Referenzdistanz eine Quellendetektionsdistanz ist, die einem ersten Lichtintensitätsreferenzwert entspricht; und
Bestimmen (S420) einer Konzentration eines zu detektierenden Gewebeelements gemäß dem zweiten Lichtintensitätsmesswert für jede zuvor festgelegte Wellenlänge und/oder dem zweiten Lichtintensitätsreferenzwert für jede zuvor festgelegte Wellenlänge,
wobei das Erfassen, für eine detektierte Stelle eines detektierten Objekts, eines zweiten Lichtintensitätsmesswertes für jede zuvor festgelegte Wellenlänge in einer Messdistanz und/oder eines zweiten Lichtintensitätsreferenzwertes für jede zuvor festgelegte Wellenlänge in einer Referenzdistanz umfasst:
Bilden (S540), für die detektierte Stelle des detektierten Objekts, eines Messringstrahls und/oder eines Referenzringstrahls, der jeder zuvor festgelegten Wellenlänge entspricht, auf einer Oberfläche der detektierten Stelle, wobei ein Innenradius oder ein Außenradius jedes Messringstrahls eine entsprechende Messdistanz ist, ein Innenradius oder ein Außenradius jedes Referenzringstrahls eine entsprechende Referenzdistanz ist und jeder Messringstrahl und jeder Referenzringstrahl einen selben geometrischen Mittelpunkt aufweisen; und
Erfassen (S550), auf der Grundlage einer lichtempfindlichen Oberfläche, die dem geometrischen Mittelpunkt entspricht, des zweiten Lichtintensitätsmesswertes, der von der Oberfläche der detektierten Stelle emittiert wird, nachdem jeder Messringstrahl die detektierte Stelle durchquert hat, und/oder des zweiten Lichtintensitätsreferenzwertes, der von der Oberfläche der detektierten Stelle emittiert wird, nachdem jeder Referenzringstrahl die detektierte Stelle durchquert hat.

2. Verfahren nach Anspruch 1, wobei der erste Lichtintensitätsmesswert ein erster Lichtintensitätswert ist, der einem größten absoluten Wert einer Lichtintensitätsvariation entspricht, die durch eine Änderung der Konzentration des zu detektierenden Gewebeelements verursacht wird, der erste Lichtintensitätsreferenzwert ein erster Lichtintensitätswert ist, der einem kleinsten absoluten Wert einer Lichtintensitätsvariation entspricht, die durch eine Änderung der Konzentration des zu detektierenden Gewebeelements verursacht wird, und die Lichtintensitätsvariation, die durch die Änderung der Konzentration des zu detektierenden Gewebeelements verursacht wird, eine Variation zwischen dem ersten Lichtintensitätswert und einem entsprechenden zuvor festgelegten Lichtintensitätswert ist.

3. Verfahren nach Anspruch 1 oder 2, wobei jeder Messringstrahl von einer punktförmigen Lichtfleckabtastung gebildet wird oder durch eine Strahlprojektion gebildet wird und jeder Referenzringstrahl von der punktförmigen Lichtfleckabtastung gebildet wird oder von der Strahlprojektion gebildet wird.

4. Verfahren nach einem der Ansprüche 1 bis 3, wobei das Bestimmen einer Konzentration eines zu detektierenden Gewebeelements gemäß dem zweiten Lichtintensitätsmesswert für jede zuvor festgelegte Wellenlänge und/oder dem zweiten Lichtintensitätsreferenzwert für jede zuvor festgelegte Wellenlänge umfasst:
Durchführen (S560), für jede zuvor festgelegte Wellenlänge, einer Differenzoperation zwischen dem zweiten Lichtintensitätsmesswert für die zuvor festgelegte Wellenlänge und dem zweiten Lichtintensitätsreferenzwert für die zuvor festgelegte Wellenlänge, um einen Lichtintensitätsdifferenzwert zu erhalten; und
Bestimmen (S570) der Konzentration des zu detektierenden Gewebeelements gemäß dem Lichtintensitätsdifferenzwert für jede zuvor festgelegte Wellenlänge.

5. Verfahren nach Anspruch 1 oder 2, wobei eine Nichtkontakt zwischen der lichtempfindlichen Oberfläche und der Oberfläche der detektierten Stelle gebildet wird durch:
Bereitstellen der lichtempfindlichen Oberfläche an einem ersten Ende eines Lichtleiterteils (12), wobei ein zweites Ende des Lichtleiterteils (12) in Kontakt oder Nichtkontakt mit der Oberfläche der detektierten Stelle steht, wobei das zweite Ende des Lichtleiterteils (12) und das erste Ende des Lichtleiterteils (12) entgegengesetzte Endflächen sind.

6. Vorrichtung (2) zur nicht-invasiven Detektion für ein Gewebeelement, umfassend:
einen Lichtintensitätssensor (17), der eingerichtet ist zum Erfassen, für eine detektierte Stelle eines detektierten Objekts, eines zweiten Lichtintensitätsmesswertes für jede zuvor festgelegte Wellenlänge von mindestens einer zuvor festgelegten Wellenlänge in einer Messdistanz und/oder eines zweiten Lichtintensitätsreferenzwertes für jede zuvor festgelegte Wellenlänge von mindestens einer zuvor festgelegten Wellenlänge in einer Referenzdistanz, wobei die Messdistanz eine Quellendetektionsdistanz ist, die einem ersten Lichtintensitätsmesswert entspricht, und die Referenzdistanz eine Quellendetektionsdistanz ist, die einem ersten Lichtintensitätsreferenzwert entspricht; und
einen Prozessor (18), der eingerichtet ist zum Bestimmen einer Konzentration eines zu detektierenden Gewebeelements gemäß dem zweiten Lichtintensitätsmesswert für jede zuvor festgelegte Wellenlänge und/oder dem zweiten Lichtintensitätsreferenzwert für jede zuvor festgelegte Wellenlänge,
wobei der Lichtintensitätssensor (17) umfasst:
einen Ringstrahlgenerator (170), der eingerichtet ist zum Bilden, für die detektierte Stelle des detektierten Objekts, eines Messringstrahls und/oder eines Referenzringstrahls, der jeder zuvor festgelegten Wellenlänge entspricht, auf einer Oberfläche der detektierten Stelle, wobei ein Innenradius oder ein Außenradius jedes Messringstrahls eine entsprechende Messdistanz ist, ein Innenradius oder ein Außenradius jedes Referenzringstrahls eine entsprechende Referenzdistanz ist und jeder Messringstrahl und jeder Referenzringstrahl einen selben geometrischen Mittelpunkt aufweisen; und
einen Lichtintensitätssignalgenerator (171), der eingerichtet ist zum Erfassen, auf der Grundlage einer lichtempfindlichen Oberfläche, die dem geometrischen Mittelpunkt entspricht, des zweiten Lichtintensitätsmesswertes, der von der Oberfläche der detektierten Stelle emittiert wird, nachdem jeder Messringstrahl die detektierte Stelle durchquert hat, und/oder des zweiten Lichtintensitätsreferenzwertes, der von der Oberfläche der detektierten Stelle emittiert wird, nachdem jeder Referenzringstrahl die detektierte Stelle durchquert hat.

7. Vorrichtung nach Anspruch 6, wobei der erste Lichtintensitätsmesswert ein erster Lichtintensitätswert ist, der einem größten absoluten Wert einer Lichtintensitätsvariation entspricht, die durch eine Änderung der Konzentration des zu detektierenden Gewebeelements verursacht wird, der erste Lichtintensitätsreferenzwert ein erster Lichtintensitätswert ist, der einem kleinsten absoluten Wert einer Lichtintensitätsvariation entspricht, die durch eine Änderung der Konzentration des zu detektierenden Gewebeelements verursacht wird, und die Lichtintensitätsvariation, die durch die Änderung der Konzentration des zu detektierenden Gewebeelements verursacht wird, eine Variation zwischen dem ersten Lichtintensitätswert und einem entsprechenden zuvor festgelegten Lichtintensitätswert ist.

8. Vorrichtung nach Anspruch 6 oder 7, wobei der Ringstrahlgenerator (170) eine Lichtquelle (1000), eine Strahleinstellvorrichtung (1001) und eine Steuervorrichtung (1002) umfasst und
die Steuervorrichtung (1002) kommunikativ mit der Lichtquelle (1000) bzw. der Strahleinstellvorrichtung (1001) verbunden ist und
die Steuervorrichtung (1002) dazu eingerichtet ist, für die detektierte Stelle des detektierten Objekts die Lichtquelle (1000) und die Strahleinstellvorrichtung (1001) so zu steuern, dass sie zusammenwirken, um einen Messringstrahl und/oder einen Referenzringstrahl entsprechend jeder zuvor festgelegten Wellenlänge auf der Oberfläche der detektierten Stelle gemäß einer entsprechenden Betriebszustandsinstruktion zu bilden.

9. Vorrichtung nach einem der Ansprüche 6 bis 8, wobei die Strahleinstellvorrichtung (1001) einen MEMS-Abtastspiegel (10010) umfasst, und
wobei die Steuervorrichtung (1002) dazu eingerichtet ist, für die detektierte Stelle des detektierten Objekts die Lichtquelle (1000) gemäß einer entsprechenden Betriebszustandsinstruktion so zu steuern, dass sie einen einfallenden Strahl entsprechend jeder zuvor festgelegten Wellenlänge emittiert und jeden einfallenden Strahl auf den MEMS-Abtastspiegel (10010) projiziert, und den MEMS-Abtastspiegel (10010) gemäß der entsprechenden Betriebszustandsinstruktion so zu steuern, dass er jeden einfallenden Strahl in einen entsprechenden Messringstrahl und/oder einen entsprechenden Referenzringstrahl umwandelt und jeden Messringstrahl und/oder jeden Referenzringstrahl auf die Oberfläche der detektierten Stelle projiziert.

10. Vorrichtung nach einem der Ansprüche 6 bis 8, wobei die Strahleinstellvorrichtung (1001) eine Galvoscanner-Anordnung (10011) umfasst, und
wobei die Steuervorrichtung (1002) dazu eingerichtet ist, für die detektierte Stelle des detektierten Objekts die Lichtquelle (1000) gemäß einer entsprechenden Betriebszustandsinstruktion so zu steuern, dass sie einen einfallenden Strahl entsprechend jeder zuvor festgelegten Wellenlänge emittiert und jeden einfallenden Strahl auf die Galvoscanner-Anordnung (10011) projiziert, und die Galvoscanner-Anordnung (10011) gemäß der entsprechenden Betriebszustandsinstruktion so zu steuern, dass sie jeden einfallenden Strahl in einen entsprechenden Messringstrahl und/oder einen entsprechenden Referenzringstrahl umwandelt und jeden Messringstrahl und/oder jeden Referenzringstrahl auf die Oberfläche der detektierten Stelle projiziert.

11. Vorrichtung nach Anspruch 10, wobei die Galvoscanner-Anordnung (10011) einen ersten zweiachsigen Galvoscanner (100110) und einen zweiten zweiachsigen Galvoscanner (100111) umfasst, und
wobei die Steuervorrichtung (1002) dazu eingerichtet ist, für die detektierte Stelle des detektierten Objekts die Lichtquelle (1000) gemäß einer entsprechenden Betriebszustandsinstruktion so zu steuern, dass sie einen einfallenden Strahl entsprechend jeder zuvor festgelegten Wellenlänge emittiert und jeden einfallenden Strahl auf den ersten zweiachsigen Galvoscanner (100110) projiziert;
die Steuervorrichtung (1002) dazu eingerichtet ist, den ersten zweiachsigen Galvoscanner (100110) gemäß der entsprechenden Betriebszustandsinstruktion so zu steuern, dass er einen ersten zuvor festgelegten Winkel entlang einer X-Achse so ablenkt, dass jeder einfallende Strahl um den ersten zuvor festgelegten Winkel in einer X-Achsenrichtung abgelenkt wird, und jeden abgelenkten einfallenden Strahl auf den zweiten zweiachsigen Galvoscanner (100111) projiziert; und
die Steuervorrichtung (1002) dazu eingerichtet ist, den zweiten zweiachsigen Galvoscanner (100111) gemäß der Betriebszustandsinstruktion so zu steuern, dass er einen zweiten zuvor festgelegten Winkel in einer Y-Achsenrichtung so ablenkt, dass jeder abgelenkte einfallende Strahl um den zweiten zuvor festgelegten Winkel in einer Y-Achsenrichtung so abgelenkt wird, dass jeder Messringstrahl und/oder jeder Referenzringstrahl gebildet wird, und jeden Messringstrahl und/oder jeden Referenzringstrahl auf die Oberfläche der detektierten Stelle projiziert.

12. Vorrichtung nach einem der Ansprüche 6 bis 8, wobei die Strahleinstellvorrichtung (1001) einen Drehspiegel (10012) und eine erste Spannungsfokussierlinse (10013) umfasst, und
wobei die Steuervorrichtung (1002) dazu eingerichtet ist, für die detektierte Stelle des detektierten Objekts die Lichtquelle (1000) gemäß einer entsprechenden Betriebszustandsinstruktion so zu steuern, dass sie einen einfallenden Strahl entsprechend jeder zuvor festgelegten Wellenlänge emittiert und jeden einfallenden Strahl auf den Drehspiegel (10012) projiziert;
die Steuervorrichtung (1002) dazu eingerichtet ist, den Drehspiegel (10012) gemäß der entsprechenden Betriebszustandsinstruktion so zu steuern, dass er sich in verschiedenen Winkeln dreht, um jeden einfallenden Strahl in einen entsprechenden ursprünglichen Ringstrahl umzuwandeln, und jeden ursprünglichen Ringstrahl auf die erste Spannungsfokussierlinse (10013) projiziert; und
die Steuervorrichtung (1002) dazu eingerichtet ist, die erste Spannungsfokussierlinse (10013) gemäß der entsprechenden Betriebszustandsinstruktion so zu steuern, dass sie einen Innenradius oder einen Außenradius jedes ursprünglichen Ringstrahls auf eine entsprechende Messdistanz einstellt, um jeden Messringstrahl zu erhalten, und/oder einen Innenradius oder einen Außenradius jedes ursprünglichen Ringstrahls auf eine entsprechende Referenzdistanz einstellt, um jeden Referenzringstrahl zu erhalten, und jeden Messringstrahl und/oder jeden Referenzringstrahl auf die detektierte Stelle projiziert.

13. Vorrichtung nach Anspruch 12, wobei jede Betriebszustandsinstruktion durch die Steuervorrichtung (1002) gemäß einer ersten Beziehungstabelle generiert wird, in der eine entsprechende Beziehung zwischen jedem Messringstrahl entsprechend jeder zuvor festgelegten Wellenlänge und einer Betriebsspannung der ersten Spannungsfokussierlinse (10013) und/oder eine entsprechende Beziehung zwischen jedem Referenzringstrahl entsprechend jeder zuvor festgelegten Wellenlänge und einer Betriebsspannung der ersten Spannungsfokussierlinse (10013) für die detektierte Stelle des detektierten Objekts gespeichert sind.

14. Vorrichtung nach einem der Ansprüche 6 bis 8, wobei die Strahleinstellvorrichtung (1001) eine Mikrolinsenanordnung (10014) und eine Bildgebungslinse (10015) umfasst, und
wobei die Steuervorrichtung (1002) dazu eingerichtet ist, für die detektierte Stelle des detektierten Objekts die Lichtquelle (1000) gemäß einer entsprechenden Betriebszustandsinstruktion so zu steuern, dass sie einen einfallenden Strahl entsprechend jeder zuvor festgelegten Wellenlänge emittiert und jeden einfallenden Strahl auf die Mikrolinsenanordnung (10014) projiziert, und die Mikrolinsenanordnung (10014) gemäß der entsprechenden Betriebszustandsinstruktion so zu steuern, dass sie jeden einfallenden Strahl in einen entsprechenden Messringstrahl und/oder einen entsprechenden Referenzringstrahl umwandelt und jeden Messringstrahl und/oder jeden Referenzringstrahl durch die Bildgebungslinse (10015) auf die Oberfläche der detektierten Stelle projiziert.

15. Vorrichtung nach Anspruch 14, wobei die Strahleinstellvorrichtung (1001) des Weiteren eine Strahlaufweitungslinsengruppe (10016) umfasst, und
wobei die Steuervorrichtung (1002) dazu eingerichtet ist, für die detektierte Stelle des detektierten Objekts die Lichtquelle (1000) gemäß einer entsprechenden Betriebszustandsinstruktion so zu steuern, dass sie den einfallenden Strahl entsprechend jeder zuvor festgelegten Wellenlänge emittiert und jeden einfallenden Strahl auf die Strahlaufweitungslinsengruppe (10016) projiziert;
die Strahlaufweitungslinsengruppe (10016) dazu eingerichtet ist, jeden einfallenden Strahl aufzuweiten und jeden aufgeweiteten einfallenden Strahl auf die Mikrolinsenanordnung (10014) zu projizieren, dergestalt, dass eine Projektion jedes auf die Mikrolinsenanordnung (10014) einfallenden Strahls die Mikrolinsenanordnung (10014) bedeckt; und
die Steuervorrichtung (1002) dazu eingerichtet ist, die Mikrolinsenanordnung (10014) gemäß der entsprechenden Betriebszustandsinstruktion so zu steuern, dass sie jeden einfallenden Strahl in einen entsprechenden Messringstrahl und/oder einen entsprechenden Referenzringstrahl umwandelt und jeden Messringstrahl und/oder jeden Referenzringstrahl durch die Bildgebungslinse (10015) auf die Oberfläche der detektierten Stelle projiziert.

16. Vorrichtung nach Anspruch 15, wobei jede Betriebszustandsinstruktion durch die Steuervorrichtung (1002) gemäß einer zweiten Beziehungstabelle generiert wird, in der eine entsprechende Beziehung zwischen jedem Messringstrahl entsprechend jeder zuvor festgelegten Wellenlänge und einer Mikrolinse in einem offenen Zustand in der Mikrolinsenanordnung (10014) und/oder eine entsprechende Beziehung zwischen jedem Referenzringstrahl entsprechend jeder zuvor festgelegten Wellenlänge und einer Mikrolinse in einem offenen Zustand in der Mikrolinsenanordnung (10014) für die detektierte Stelle des detektierten Objekts gespeichert sind.

17. Vorrichtung nach einem der Ansprüche 6 bis 8, wobei die Strahleinstellvorrichtung (1001) eine konische Linse (10017) und eine zweite Spannungsfokussierlinse (10018) umfasst, und
wobei der die Steuervorrichtung (1002) dazu eingerichtet ist, für die detektierte Stelle des detektierten Objekts die Lichtquelle (1000) gemäß einer entsprechenden Betriebszustandsinstruktion so zu steuern, dass sie einen einfallenden Strahl entsprechend jeder zuvor festgelegten Wellenlänge emittiert und jeden einfallenden Strahl auf die konische Linse (10017) projiziert;
die konische Linse (10017) dazu eingerichtet ist, jeden einfallenden Strahl in einen konischen Strahl umzuwandeln und jeden konischen Strahl auf die zweite Spannungsfokussierlinse (10018) zu projizieren, um durch die zweite Spannungsfokussierlinse (10018) als jeder ursprüngliche Ringstrahl abgebildet zu werden; und
die Steuervorrichtung (1002) dazu eingerichtet ist, die zweite Spannungsfokussierlinse (10018) gemäß der entsprechenden Betriebszustandsinstruktion so zu steuern, dass sie einen Innenradius oder einen Außenradius jedes ursprünglichen Ringstrahls auf eine entsprechende Messdistanz einstellt, um jeden Messringstrahl zu erhalten, und/oder einen Innenradius oder einen Außenradius jedes ursprünglichen Ringstrahls auf eine entsprechende Referenzdistanz einstellt, um jeden Referenzringstrahl zu erhalten, und jeden Messringstrahl und/oder jeden Referenzringstrahl auf die detektierte Stelle projiziert.

18. Vorrichtung nach Anspruch 17, wobei jede Betriebszustandsinstruktion durch den die Steuervorrichtung (1002) gemäß einer dritten Beziehungstabelle generiert wird, in der eine entsprechende Beziehung zwischen jedem Messringstrahl entsprechend jeder zuvor festgelegten Wellenlänge und einer Betriebsspannung der zweiten Spannungsfokussierlinse (10018) und/oder eine entsprechende Beziehung zwischen jedem Referenzringstrahl entsprechend jeder zuvor festgelegten Wellenlänge und einer Betriebsspannung der zweiten Spannungsfokussierlinse (10018) für die detektierte Stelle des detektierten Objekts gespeichert sind.

19. Vorrichtung nach einem der Ansprüche 6 bis 18, wobei der Prozessor (18) eingerichtet ist zum
Durchführen, für jede zuvor festgelegte Wellenlänge, einer Differenzoperation an dem zweiten Lichtintensitätsmesswert und dem zweiten Lichtintensitätsreferenzwert für die zuvor festgelegte Wellenlänge, um einen Lichtintensitätsdifferenzwert zu erhalten; und
Bestimmen der Konzentration des zu detektierenden Gewebeelements gemäß dem Lichtintensitätsdifferenzwert für jede zuvor festgelegte Wellenlänge.

20. Am Körper tragbare Vorrichtung (3), umfassend einen Körper (30) und die Vorrichtung (2) zur nicht-invasiven Detektion für ein Gewebeelement nach einem der Ansprüche 6 bis 19,
wobei die Vorrichtung (2) zur nicht-invasiven Detektion für das Gewebeelement an dem Körper (30) angeordnet ist, und wobei die am Körper tragbare Vorrichtung (3) an der detektierten Stelle getragen wird.

## Revendications

1. Procédé de détection non invasive d'un élément tissulaire, comprenant de :
acquérir (S410), pour un site détecté d'un objet détecté, une deuxième valeur de mesure d'intensité lumineuse pour chaque longueur d'onde prédéterminée d'au moins une longueur d'onde prédéterminée à une distance de mesure et/ou une deuxième valeur de référence d'intensité lumineuse pour chaque longueur d'onde prédéterminée d'au moins une longueur d'onde prédéterminée à une distance de référence, dans lequel la distance de mesure est une distance source-détection correspondant à une première valeur de mesure d'intensité lumineuse et la distance de référence est une distance source-détection correspondant à une première valeur de référence d'intensité lumineuse ; et
déterminer (S420) une concentration d'un élément tissulaire à détecter selon la deuxième valeur de mesure d'intensité lumineuse pour chaque longueur d'onde prédéterminée et/ou la deuxième valeur de référence d'intensité lumineuse pour chaque longueur d'onde prédéterminée,
dans lequel le fait d'acquérir, pour un site détecté d'un objet détecté, une deuxième valeur de mesure d'intensité lumineuse pour chaque longueur d'onde prédéterminée à une distance de mesure et/ou une deuxième valeur de référence d'intensité lumineuse pour chaque longueur d'onde prédéterminée à une distance de référence comprend de :
former (S540), pour le site détecté de l'objet détecté, un faisceau annulaire de mesure et/ou un faisceau annulaire de référence correspondant à chaque longueur d'onde prédéterminée sur une surface du site détecté, dans lequel un rayon intérieur ou un rayon extérieur de chaque faisceau annulaire de mesure est une distance de mesure correspondante, un rayon intérieur ou un rayon extérieur de chaque faisceau annulaire de référence est une distance de référence correspondante, et chaque faisceau annulaire de mesure et chaque faisceau annulaire de référence ont un même centre géométrique ; et
acquérir (S550), sur la base d'une surface photosensible correspondant au centre géométrique, la deuxième valeur de mesure d'intensité lumineuse émise depuis la surface du site détecté après que chaque faisceau annulaire de mesure a traversé le site détecté et/ou la deuxième valeur de référence d'intensité lumineuse émise depuis la surface du site détecté après que chaque faisceau annulaire de référence a traversé le site détecté.

2. Procédé selon la revendication 1, dans lequel la première valeur de mesure d'intensité lumineuse est une première valeur d'intensité lumineuse correspondant à une valeur absolue maximale d'une variation d'intensité lumineuse provoquée par un changement dans la concentration de l'élément tissulaire à détecter, la première valeur de référence d'intensité lumineuse est une première valeur d'intensité lumineuse correspondant à une valeur absolue minimale d'une variation d'intensité lumineuse provoquée par un changement dans la concentration de l'élément tissulaire à détecter, et la variation d'intensité lumineuse provoquée par le changement dans la concentration de l'élément tissulaire à détecter est une variation entre la première valeur d'intensité lumineuse et une valeur d'intensité lumineuse prédéterminée correspondante.

3. Procédé selon la revendication 1 ou 2, dans lequel chaque faisceau annulaire de mesure est formé par un balayage de tache lumineuse ponctuelle ou formé par une projection de faisceau, et chaque faisceau annulaire de référence est formé par le balayage de tache lumineuse ponctuelle ou formé par la projection de faisceau.

4. Procédé selon l'une quelconque des revendications 1 à 3, dans lequel le fait de déterminer une concentration d'un élément tissulaire à détecter selon la deuxième valeur de mesure d'intensité lumineuse pour chaque longueur d'onde prédéterminée et/ou la deuxième valeur de référence d'intensité lumineuse pour chaque longueur d'onde prédéterminée comprend de :
effectuer (S560), pour chaque longueur d'onde prédéterminée, une opération de différence entre la deuxième valeur de mesure d'intensité lumineuse pour la longueur d'onde prédéterminée et la deuxième valeur de référence d'intensité lumineuse pour la longueur d'onde prédéterminée, de manière à obtenir une valeur de différence d'intensité lumineuse ; et
déterminer (S570) la concentration de l'élément tissulaire à détecter selon la valeur de différence d'intensité lumineuse pour chaque longueur d'onde prédéterminée.

5. Procédé selon la revendication 1 ou 2, dans lequel un non-contact entre la surface photosensible et la surface du site détecté est formé en :
prévoyant la surface photosensible à une première extrémité d'une partie de guidage de lumière (12), une seconde extrémité de la partie de guidage de lumière (12) étant en contact ou sans contact avec la surface du site détecté, dans lequel la seconde extrémité de la partie de guidage de lumière (12) et la première extrémité de la partie de guidage de lumière (12) sont des faces d'extrémité opposées.

6. Dispositif de détection non invasive (2) pour un élément tissulaire, comprenant :
un capteur d'intensité lumineuse (17) configuré pour acquérir, pour un site détecté d'un objet détecté, une deuxième valeur de mesure d'intensité lumineuse pour chaque longueur d'onde prédéterminée d'au moins une longueur d'onde prédéterminée à une distance de mesure et/ou une deuxième valeur de référence d'intensité lumineuse pour chaque longueur d'onde prédéterminée d'au moins une longueur d'onde prédéterminée à une distance de référence, dans lequel la distance de mesure est une distance source-détection correspondant à une première valeur de mesure d'intensité lumineuse et la distance de référence est une distance source-détection correspondant à une première valeur de référence d'intensité lumineuse ; et
un processeur (18) configuré pour déterminer une concentration d'un élément tissulaire à détecter selon la deuxième valeur de mesure d'intensité lumineuse pour chaque longueur d'onde prédéterminée et/ou la deuxième valeur de référence d'intensité lumineuse pour chaque longueur d'onde prédéterminée,
dans lequel le capteur d'intensité lumineuse (17) comprend :
un générateur de faisceau annulaire (170) configuré pour former, pour le site détecté de l'objet détecté, un faisceau annulaire de mesure et/ou un faisceau annulaire de référence correspondant à chaque longueur d'onde prédéterminée sur une surface du site détecté, dans lequel un rayon intérieur ou un rayon extérieur de chaque faisceau annulaire de mesure est une distance de mesure correspondante, un rayon intérieur ou un rayon extérieur de chaque faisceau annulaire de référence est une distance de référence correspondante, et chaque faisceau annulaire de mesure et chaque faisceau annulaire de référence ont un même centre géométrique ; et
un générateur de signal d'intensité lumineuse (171) configuré pour acquérir, sur la base d'une surface photosensible correspondant au centre géométrique, la deuxième valeur de mesure d'intensité lumineuse émise depuis la surface du site détecté après que chaque faisceau annulaire de mesure a traversé le site détecté et/ou la deuxième valeur de référence d'intensité lumineuse émise depuis la surface du site détecté après que chaque faisceau annulaire de référence a traversé le site détecté.

7. Dispositif selon la revendication 6, dans lequel la première valeur de mesure d'intensité lumineuse est une première valeur d'intensité lumineuse correspondant à une valeur absolue maximale d'une variation d'intensité lumineuse provoquée par un changement dans la concentration de l'élément tissulaire à détecter, la première valeur de référence d'intensité lumineuse est une première valeur d'intensité lumineuse correspondant à une valeur absolue minimale d'une variation d'intensité lumineuse provoquée par un changement dans la concentration de l'élément tissulaire à détecter, et la variation d'intensité lumineuse provoquée par le changement dans la concentration de l'élément tissulaire à détecter est une variation entre la première valeur d'intensité lumineuse et une valeur d'intensité lumineuse prédéterminée correspondante.

8. Dispositif selon la revendication 6 ou 7, dans lequel le générateur de faisceau annulaire (170) comprend une source lumineuse (1000), un dispositif de réglage de faisceau (1001) et un dispositif de commande (1002), et
le dispositif de commande (1002) est connecté de manière communicative à la source lumineuse (1000) et au dispositif de réglage de faisceau (1001), respectivement, et
le dispositif de commande (1002) est configuré pour, pour le site détecté de l'objet détecté, commander la source lumineuse (1000) et le dispositif de réglage de faisceau (1001) afin qu'ils coopèrent pour former un faisceau annulaire de mesure et/ou un faisceau annulaire de référence correspondant à chaque longueur d'onde prédéterminée sur la surface du site détecté, selon une instruction d'état de fonctionnement correspondante.

9. Dispositif selon l'une quelconque des revendications 6 à 8, dans lequel le dispositif de réglage de faisceau (1001) comprend un miroir de balayage MEMS (10010), et
dans lequel le dispositif de commande (1002) est configuré pour, pour le site détecté de l'objet détecté, commander la source lumineuse (1000), selon une instruction d'état de fonctionnement correspondante, afin d'émettre un faisceau incident correspondant à chaque longueur d'onde prédéterminée et de projeter chaque faisceau incident vers le miroir de balayage MEMS (10010), et pour commander le miroir de balayage MEMS (10010), selon l'instruction d'état de fonctionnement correspondante, afin de convertir chaque faisceau incident en un faisceau annulaire de mesure correspondant et/ou un faisceau annulaire de référence correspondant et de projeter chaque faisceau annulaire de mesure et/ou chaque faisceau annulaire de référence sur la surface du site détecté.

10. Dispositif selon l'une quelconque des revendications 6 à 8, dans lequel le dispositif de réglage de faisceau (1001) comprend un ensemble de scanners galvanométriques (10011), et
dans lequel le dispositif de commande (1002) est configuré pour, pour le site détecté de l'objet détecté, commander la source lumineuse (1000), selon une instruction d'état de fonctionnement correspondante, afin d'émettre un faisceau incident correspondant à chaque longueur d'onde prédéterminée et de projeter chaque faisceau incident vers l'ensemble de scanners galvanométriques (10011), et pour commander l'ensemble de scanners galvanométriques (10011), selon l'instruction d'état de fonctionnement correspondante, afin de convertir chaque faisceau incident en un faisceau annulaire de mesure correspondant et/ou un faisceau annulaire de référence correspondant et de projeter chaque faisceau annulaire de mesure et/ou chaque faisceau annulaire de référence sur la surface du site détecté.

11. Dispositif selon la revendication 10, dans lequel l'ensemble de scanners galvanométriques (10011) comprend un premier scanner galvanométrique à deux axes (100110) et un deuxième scanner galvanométrique à deux axes (100111), et
dans lequel le dispositif de commande (1002) est configuré pour, pour le site détecté de l'objet détecté, commander la source lumineuse (1000), selon une instruction d'état de fonctionnement correspondante, afin d'émettre un faisceau incident correspondant à chaque longueur d'onde prédéterminée et de projeter chaque faisceau incident vers le premier scanner galvanométrique à deux axes (100110) ;
le dispositif de commande (1002) est configuré pour commander le premier scanner galvanométrique à deux axes (100110), selon l'instruction d'état de fonctionnement correspondante, afin de dévier d'un premier angle prédéterminé le long d'un axe X, de sorte que chaque faisceau incident soit dévié du premier angle prédéterminé dans une direction d'axe X, et de projeter chaque faisceau incident dévié vers le deuxième scanner galvanométrique à deux axes (100111) ; et
le dispositif de commande (1002) est configuré pour commander le deuxième scanner galvanométrique à deux axes (100111), selon l'instruction d'état de fonctionnement, afin de dévier d'un deuxième angle prédéterminé dans une direction d'axe Y, de sorte que chaque faisceau incident dévié soit dévié du deuxième angle prédéterminé dans une direction d'axe Y, de manière à former chaque faisceau annulaire de mesure et/ou chaque faisceau annulaire de référence, et de projeter chaque faisceau annulaire de mesure et/ou chaque faisceau annulaire de référence sur la surface du site détecté.

12. Dispositif selon l'une quelconque des revendications 6 à 8, dans lequel le dispositif de réglage de faisceau (1001) comprend un miroir rotatif (10012) et une première lentille à focalisation par tension (10013), et
dans lequel le dispositif de commande (1002) est configuré pour, pour le site détecté de l'objet détecté, commander la source lumineuse (1000), selon une instruction d'état de fonctionnement correspondante, afin d'émettre un faisceau incident correspondant à chaque longueur d'onde prédéterminée et de projeter chaque faisceau incident vers le miroir rotatif (10012) ;
le dispositif de commande (1002) est configuré pour commander le miroir rotatif (10012), selon l'instruction d'état de fonctionnement correspondante, afin de le faire tourner à différents angles pour convertir chaque faisceau incident en un faisceau annulaire d'origine correspondant, et de projeter chaque faisceau annulaire d'origine vers la première lentille à focalisation par tension (10013) ; et
le dispositif de commande (1002) est configuré pour commander la première lentille à focalisation par tension (10013), selon l'instruction d'état de fonctionnement correspondante, afin d'ajuster un rayon intérieur ou un rayon extérieur de chaque faisceau annulaire d'origine à une distance de mesure correspondante pour obtenir chaque faisceau annulaire de mesure, et/ou d'ajuster un rayon intérieur ou un rayon extérieur de chaque faisceau annulaire d'origine à une distance de référence correspondante pour obtenir chaque faisceau annulaire de référence, et de projeter chaque faisceau annulaire de mesure et/ou chaque faisceau annulaire de référence vers le site détecté.

13. Dispositif selon la revendication 12, dans lequel chaque instruction d'état de fonctionnement est générée par le dispositif de commande (1002) selon un premier tableau de relations dans lequel sont stockées une relation correspondante entre chaque faisceau annulaire de mesure correspondant à chaque longueur d'onde prédéterminée et une tension de fonctionnement de la première lentille à focalisation par tension (10013) et/ou une relation correspondante entre chaque faisceau annulaire de référence correspondant à chaque longueur d'onde prédéterminée et une tension de fonctionnement de la première lentille à focalisation par tension (10013) pour le site détecté de l'objet détecté.

14. Dispositif selon l'une quelconque des revendications 6 à 8, dans lequel le dispositif de réglage de faisceau (1001) comprend un réseau de microlentilles (10014) et une lentille d'imagerie (10015), et
dans lequel le dispositif de commande (1002) est configuré pour, pour le site détecté de l'objet détecté, commander la source lumineuse (1000), selon une instruction d'état de fonctionnement correspondante, afin d'émettre un faisceau incident correspondant à chaque longueur d'onde prédéterminée et de projeter chaque faisceau incident vers le réseau de microlentilles (10014), et pour commander le réseau de microlentilles (10014), selon l'instruction d'état de fonctionnement correspondante, afin de convertir chaque faisceau incident en un faisceau annulaire de mesure correspondant et/ou un faisceau annulaire de référence correspondant, et de projeter chaque faisceau annulaire de mesure et/ou chaque faisceau annulaire de référence sur la surface du site détecté à travers la lentille d'imagerie (10015).

15. Dispositif selon la revendication 14, dans lequel le dispositif de réglage de faisceau (1001) comprend en outre un groupe de lentilles d'expansion de faisceau (10016), et
dans lequel le dispositif de commande (1002) est configuré pour, pour le site détecté de l'objet détecté, commander la source lumineuse (1000), selon une instruction d'état de fonctionnement correspondante, afin d'émettre le faisceau incident correspondant à chaque longueur d'onde prédéterminée et de projeter chaque faisceau incident vers le groupe de lentilles d'expansion de faisceau (10016) ;
le groupe de lentilles d'expansion de faisceau (10016) est configuré pour élargir chaque faisceau incident et projeter chaque faisceau incident élargi vers le réseau de microlentilles (10014), de sorte qu'une projection de chaque faisceau incident sur le réseau de microlentilles (10014) couvre le réseau de microlentilles (10014) ; et
le dispositif de commande (1002) est configuré pour commander le réseau de microlentilles (10014), selon l'instruction d'état de fonctionnement correspondante, afin de convertir chaque faisceau incident en un faisceau annulaire de mesure correspondant et/ou un faisceau annulaire de référence correspondant, et de projeter chaque faisceau annulaire de mesure et/ou chaque faisceau annulaire de référence sur la surface du site détecté à travers la lentille d'imagerie (10015).

16. Dispositif selon la revendication 15, dans lequel chaque instruction d'état de fonctionnement est générée par le dispositif de commande (1002) selon un deuxième tableau de relations dans lequel sont stockées une relation correspondante entre chaque faisceau annulaire de mesure correspondant à chaque longueur d'onde prédéterminée et une microlentille à l'état ouvert dans le réseau de microlentilles (10014) et/ou une relation correspondante entre chaque faisceau annulaire de référence correspondant à chaque longueur d'onde prédéterminée et une microlentille à l'état ouvert dans le réseau de microlentilles (10014) pour le site détecté de l'objet détecté.

17. Dispositif selon l'une quelconque des revendications 6 à 8, dans lequel le dispositif de réglage de faisceau (1001) comprend une lentille conique (10017) et une deuxième lentille à focalisation par tension (10018), et
dans lequel le dispositif de commande (1002) est configuré pour, pour le site détecté de l'objet détecté, commander la source lumineuse (1000), selon une instruction d'état de fonctionnement correspondante, afin d'émettre un faisceau incident correspondant à chaque longueur d'onde prédéterminée et de projeter chaque faisceau incident vers la lentille conique (10017) ;
la lentille conique (10017) est configurée pour convertir chaque faisceau incident en un faisceau conique et projeter chaque faisceau conique vers la deuxième lentille à focalisation par tension (10018) afin qu'il soit imagé comme chaque faisceau annulaire d'origine par la deuxième lentille à focalisation par tension (10018) ; et
le dispositif de commande (1002) est configuré pour commander la deuxième lentille à focalisation par tension (10018), selon l'instruction d'état de fonctionnement correspondante, afin d'ajuster un rayon intérieur ou un rayon extérieur de chaque faisceau annulaire d'origine à une distance de mesure correspondante pour obtenir chaque faisceau annulaire de mesure, et/ou d'ajuster un rayon intérieur ou un rayon extérieur de chaque faisceau annulaire d'origine à une distance de référence correspondante pour obtenir chaque faisceau annulaire de référence, et de projeter chaque faisceau annulaire de mesure et/ou chaque faisceau annulaire de référence vers le site détecté.

18. Dispositif selon la revendication 17, dans lequel chaque instruction d'état de fonctionnement est générée par le dispositif de commande (1002) selon un troisième tableau de relations dans lequel sont stockées une relation correspondante entre chaque faisceau annulaire de mesure correspondant à chaque longueur d'onde prédéterminée et une tension de fonctionnement de la deuxième lentille à focalisation par tension (10018) et/ou une relation correspondante entre chaque faisceau annulaire de référence correspondant à chaque longueur d'onde prédéterminée et une tension de fonctionnement de la deuxième lentille à focalisation par tension (10018) pour le site détecté de l'objet détecté.

19. Dispositif selon l'une quelconque des revendications 6 à 18, dans lequel le processeur (18) est configuré pour :
effectuer, pour chaque longueur d'onde prédéterminée, une opération de différence sur la deuxième valeur de mesure d'intensité lumineuse et la deuxième valeur de référence d'intensité lumineuse pour la longueur d'onde prédéterminée, de manière à obtenir une valeur de différence d'intensité lumineuse ; et
déterminer la concentration de l'élément tissulaire à détecter selon la valeur de différence d'intensité lumineuse pour chaque longueur d'onde prédéterminée.

20. Appareil portable (3), comprenant un corps (30) et le dispositif de détection non invasive (2) pour l'élément tissulaire selon l'une quelconque des revendications 6 à 19,
dans lequel le dispositif de détection non invasive (2) pour l'élément tissulaire est disposé sur le corps (30), et l'appareil portable (3) est porté sur le site détecté.
